# EUROPEAN PATENT SPECIFICATION

(11) **EP 3 849 663 B1**
(45) Date of publication and mention of the grant of the patent: **02.08.2023**
(21) Application number: 19773705.9
(22) Date of filing: 11.09.2019
(51) Int. Cl.: A61P 35/00, C07D 401/04, A61K 31/4184, C07D 401/14

(54) **PYRIMIDINYL-HETEROARYLOXY-NAPHTHYL COMPOUNDS AND METHODS OF USE**
PYRIMIDINYL-HETEROARYLOXY-NAPHTHYL-VERBINDUNGEN UND ANWENDUNGSVERFAHREN
COMPOSÉS PYRIMIDINYL-HÉTÉROARYLOXY-NAPHTYLE ET PROCÉDÉS D'UTILISATION

(30) Priority: 12.09.2018 WO PCT/CN2018/105184
(43) Date of publication of application: 21.07.2021
(73) Proprietor: Genentech, Inc., South San Francisco, CA 94080-4990 (US)
(72) Inventor: BRAUN, Marie-Gabrielle, South San Francisco, California 94080-4990 (US); GIBBONS, Paul, South San Francisco, California 94080-4990 (US); LEE, Wendy, South San Francisco, California 94080-4990 (US); LY, Cuong Q., South San Francisco, California 94080-4990 (US); RUDOLPH, Joachim, South San Francisco, California 94080-4990 (US); SCHWARZ, Jacob Bradley, South San Francisco, California 94080-4990 (US); ASHKENAZI, Avi, South San Francisco, California 94080-4990 (US); BEVERIDGE, Ramsay, Montreal, Québec H4S 2E1 (CA); ZHAO, Liang, Montreal, Québec H4S 2E1 (CA); LEMIRE, Alexandre, Montreal, Québec H4S 2E1 (CA); FU, Leo, Shanghai 200131 (CN); LAI, Kwong Wah, Shanghai 200131 (CN); WANG, Fei, Shanghai 200131 (CN)
(74) Representative: J A Kemp LLP
(86) International application number: PCT/US2019/050698
(87) International publication number: WO 2020/056061

(56) References cited:
- PAUL E. HARRINGTON ET AL: "Unfolded Protein Response in Cancer: IRE1[alpha] Inhibition by Selective Kinase Ligands Does Not Impair Tumor Cell Viability", ACS MEDICINAL CHEMISTRY LETTERS, vol. 6, no. 1, 29 September 2014 (2014-09-29), pages 68-72, XP055478639, US ISSN: 1948-5875, DOI: 10.1021/ml500315b cited in the application
- LIKUN WANG ET AL: "Divergent allosteric control of the IRE1 alpha endoribonuclease using kinase inhibitors", NATURE CHEMICAL BIOLOGY, NATURE PUBLISHING GROUP, BASINGSTOKE, vol. 8, no. 12, 21 October 2012 (2012-10-21), pages 982-989, XP002761451, ISSN: 1552-4450, DOI: 10.1038/NCHEMBIO.1094 [retrieved on 2012-10-21] cited in the application

## Description

### BACKGROUND OF THE INVENTION

The kinase/endoribonuclease inositol requiring enzyme 1 (IRE1α), one of the key sensors of misfolded protein accumulation in the endoplasmic reticulum that triggers the unfolded protein response (UPR), is a potential therapeutic target for diverse diseases including cancer for inhibitors that bind to the ATP-binding site on the kinase moiety of IRE1α and block its endoribonuclease activity. IRE1α is a transmembrane, bifunctional protein with a luminal domain that binds to misfolded proteins, a transmembrane segment, and a cytoplasmic portion consisting of a kinase moiety and a tandem endoribonuclease domain. Structure-activity relationship (SAR) studies led to compounds selective in recombinant IRE1α kinase screens and potent against endoribonuclease activity of recombinant IRE1α as well as cellular IRE1α. IRE1α activity mediates certain cytoprotective and pro-survival functions of the UPR, increases viability and growth in certain tumor cell lines, and can be an effective therapeutic target for specific small molecule inhibitors that block malignant tumor growth, contrary to an earlier report (Harrington, P.E. et al (2015) ACS Med. Chem. Lett. 6:68-72). In addition, inhibitors of IRE1α can be therapeutically useful for other types of diseases besides cancer including certain autoimmune, neurodegenerative, fibrotic and metabolic disorders (Wang M. and Kaufman, R.J. (2016) Nature 529:326-335).

Homeostatic regulation of protein folding in the endoplasmic reticulum (ER) is under the control of three key intracellular signaling pathways: IRE1α, PERK, and ATF6, which together orchestrate the unfolded protein response (UPR) (Schroder, et al (2005) Mutat Res-Fund Mol Mech Metagenesis 569:29 - 63). An increase in demand for protein folding in the ER or certain types of cellular injury or stress lead to the accumulation of unfolded proteins in the ER - a condition called ER stress. Cells respond to ER stress by activating the UPR to help adjust or maintain their high-fidelity protein synthetic capacity (Walter, P. and Ron, D. (2011) Science, 334:1081-1086). IRE1α is the most evolutionarily conserved of the three branches of the UPR. Importantly, the UPR makes life/ death decisions for the cell, depending on the severity and duration of ER stress, and the final outcome is either cell survival and recovery or programmed cell death (apoptosis) (Sovolyova et al, (2014) Biol Chem 395: 1-13). All three pathways of the UPR form a coordinated reaction to the accumulation of unfolded proteins; and several studies have demonstrated that there is cross talk between the different pathways (Yamamoto et al, J. Biochem. (2004) 136:343-350); Arai et al, FEBS Letts. (2006) 580:184-190; Adachi et al, Cell Struct. Func. (2008) 33:75-89). ER stress and activation of the UPR can be caused by mechanical injury, inflammation, genetic mutations, infections, oxidative stress, metabolic stress, and other types of cellular stress associated with malignancy. ER stress has also been implicated in diseases that result in fibrotic remodeling of internal organs, such as chronic liver diseases (Galligan et al, J. Toxicol. (2012) Vol. 2012, Article ID 207594, 12 pgs.; Shin et al, Cell Reports (2013) 5:654-665; Ji, Int. J. Hepatol. (2014) Vol. 2014, Article ID 513787, 11 pages), pulmonary fibrosis (Baek et al, Am. J. Resp. Cell Mol. Bio. (2012) 46:731-739); Tanjore et al, Biochim Biophys Acta (2012, online), (2013) 1832:940-947), kidney fibrosis (Chiang et al, Mol. Med. (2011) 17:1295-1305), cardiovascular disease (Spitler & Webb, Hypertension (2014) 63:e40-e45), and inflammatory bowel disease (Bogaert et al, PLoS One (2011) 6(10) e25589; Cao et al, Gastroent (2013) 144:989-1000).

IRE1α is a transmembrane, bifunctional protein with cytoplasmic kinase and endoribonuclease activity. The N-terminal domain of IRE1α is proposed to sense the presence of unfolded proteins in the ER lumen, triggering activation of the cytoplasmic kinase domain, which, in turn, activates the C-terminal endoribonuclease. IRE1α transmits information across the ER lipid bilayer (Tirasophon et al, Genes & Develop. (2000) 14:2725-2736). Increased ER protein load and presence of unfolded proteins leads to the dissociation of the ER chaperone GRP78/BiP from IRE1α molecules, which bind to misfolded proteins and then undergo dimerization and trans-autophosphorylation in the cytoplasmic kinase domain. This leads to activation of the IRE1α endoribonuclease moiety in the cytosol. The IRE1α endoribonuclease has the ability to cleave the mRNA that encodes unspliced X box protein 1 (XBP1u); this excises a 26-nucleotide intron and leads to formation of spliced XBP1 (XBP1s) mRNA, which encodes a potent transcription factor. After transport into the nucleus, the XBP1s protein binds to UPR promoter elements to initiate transcription of genes that enhance the ability of the ER to cope with unfolded proteins, for example, through enhanced ER-associated degradation of misfolded proteins, and through elevated levels of chaperones and disulfide isomerases that support protein folding in the ER. IRE1α activation is also associated with enlargement of the ER volume, which has been interpreted as an adaptive mechanism to increase protein folding capacity (Sriburi et al, J. Cell. Bio. (2004) 167:35-41); (Chen, Y. (2013) Trends Cell Biol., 23,547-555). In addition, the IRE1α endoribonuclease cleaves various mRNAs in a process called regulated IRE1α -dependent decay of mRNA (RIDD), which reduces both protein translation and import of proteins into the ER to help reestablish homeostasis (Hollien & Weissman, Science (2006) 313:104-107). In cancer cells, IRE1α suppresses ER-stress-induced apoptosis by reducing the mRNA levels of death receptor 5 (DR5) through RIDD (Lu et al., Science (2014) 345:98-101).

Besides degrading mRNA (Binet et al, Cell Metabol. (2013) 17:353-371), it was recently shown that IRE1α also has the ability to degrade microRNAs (miRs) (Upton et al, Science (2012) 338:818-822). miRs are short noncoding RNA oligonucleotides consisting of 17-25 nucleotides that generally act to inhibit gene expression by binding to complementary sequences in the 30-untranslated region of target mRNAs, either to repress mRNA translation or to induce mRNA cleavage. A number of cellular functions such as proliferation, differentiation, and apoptosis are regulated by miRs, and aberrant miR expression is observed in a variety of human diseases including fibrosis (Bowen et al, J. Pathol (2013) 229:274-285). Inhibitors that specifically target individual components of the UPR have recently been described. The inhibitor 4µ8C that stably binds to lysine 907 in the IRE1a endoribonuclease domain has been shown to inhibit both RIDD activity and XBP-1 splicing (Cross et al, Proc Natl. Acad. Sci. (2012) 109:E869-E878). High levels of 4µ8C cause no measurable toxicity in cells and concentrations ranging from 80 to 128 1M of 4µ8C completely block XBP1 splicing without affecting IRE1α (alpha) kinase activity (Cross et al, 2012). The inhibitor 4µ8C thus represents an important tool to delineate the functions of IRE1α in vivo as IRE1α-knockout mice die during embryonic development. Inhibition of IRE1α prevents activation of myofibroblasts and reduces fibrosis in animal models of liver and skin fibrosis. Pharmacological inhibition of IRE1α could revert the profibrotic phenotype of activated myofibroblasts isolated from patients with scleroderma and indicates that ER stress inhibitors should be taken into consideration when developing new strategies for the treatment of fibrotic diseases (Heindryckx, F. et al (2016) EMBO Molecular Medicine Vol 8(7):729-744).

Activation of the UPR has been shown to be an important survival pathway for tumors of secretory cell origin like multiple myeloma that have a very high protein synthesis burden. Therefore, efforts to disrupt the UPR by blocking the IRE1α endoribonuclease cleavage and activation of XBP1 have been an active area of cancer research. As a specific IRE1α RNase product, XBP1s is a direct indicator of functional IRE1 inhibition. A potent and selective IRE1α inhibitor would serve as an important tool to test the hypothesis that, without full UPR activation, tumor cells would be driven to apoptosis. IRE1α inhibitors and activating compounds have been reported (Harrington, P.E. et al (2015) ACS Med. Chem. Lett. 6:68-72; Volkmann, K., et al (2011) J. Biol. Chem., 286:12743-12755; Cross, B.C.S., et al (2012) Proc. Natl. Acad. Sci. U.S.A., 109:E869-E878; Wang, L., et al (2012) Nat. Chem. Biol., 8:982-989; Ghosh, R., et al (2014) Cell, 158:534-548; Ranatunga, S., et al (2014) J. Med. Chem., 57, 4289-4301; US 9382230; US 8815885).

There remains a need for potent and selective inhibitors having suitable pharmacological properties for the treatment of cancers provided herein in patients.

### BRIEF SUMMARY OF THE INVENTION

The present invention is set out in the appended set of claims. For avoidance of doubt, all references in the specification to a particular therapeutic method are, in the context of the present invention, to be interpreted as being directed to the relevant compound, pharmaceutical salt thereof, or pharmaceutical composition for use in the specified therapeutic method.

Disclosed are pyrimidinyl-heteroaryloxy-naphthyl compounds that target IRE1α, compositions containing these compounds, and methods for the treatment of cancers provided herein.

In one aspect, provided is a compound of Formula (I) or Formula (II), or a pharmaceutically acceptable salt thereof, as described herein.

In another aspect provided herein is a compound as set forth in Table 1 herein.

In another aspect provided herein is a pharmaceutical composition comprising a compound or a pharmaceutically acceptable salt thereof as described herein, and one or more pharmaceutically acceptable excipients.

In still another aspect provided herein is a method for treating cancer where the method comprises administering to a patient having cancer an effective amount of a compound or a pharmaceutically acceptable salt thereof as described herein.

In still another aspect provided herein is a method of inhibiting or killing a cancer cell expressing Ire1, where the method comprises contacting the cancer cell expressing Ire1 with a compound or pharmaceutically acceptable salt thereof described herein.

In yet another aspect provided herein is a method of modulating Ire1 activity, where the method comprises contacting Ire1 with a compound or pharmaceutically acceptable salt thereof described herein.

Further provided herein are uses of a compound or a pharmaceutically acceptable salt thereof described herein in the manufacture of a medicament for the treatment of cancer.

The compounds or pharmaceutically acceptable salts thereof described herein are useful in methods for treating cancer as provided herein.

Also provided is a kit for treating cancer, where the kit comprises a pharmaceutical composition comprising a the compound described herein, or a pharmaceutically acceptable salt thereof; and instructions for use.

### DETAILED DESCRIPTION OF THE INVENTION

Disclosed herein, are pyrimidinyl-heteroaryloxy-naphthyl compounds of Formula (I) and (II), and pharmaceutical compositions thereof that are inhibitors or modulators of IRE1α. As such, the compounds and compositions are useful in treating diseases and disorders mediated by IRE1α.

While the disclosure herein provides enumerated embodiments, it will be understood that they are not intended to limit the invention to those embodiments. On the contrary, the scope of the present invention is as defined by the claims. In the event that one or more of the incorporated literature, patents, and similar materials differs from or contradicts this application, including but not limited to defined terms, term usage, described techniques, or the like, this application controls. Unless otherwise defined, all technical and scientific terms used herein have the same meaning as commonly understood by one of ordinary skill in the art to which this invention belongs. Although methods and materials similar or equivalent to those described herein can be used in the practice or testing of the methods and materials are described below. The nomenclature used in this Application is based on IUPAC systematic nomenclature, unless indicated otherwise.

### Definitions

"Alkyl" as used herein refers to a saturated linear (i.e. unbranched) or branched univalent hydrocarbon chain or combination thereof, having the number of carbon atoms designated (*i*.*e*., C₁-₁₀ means one to ten carbon atoms). Particular alkyl groups are those having 1 to 20 carbon atoms (a "C₁-₂₀ alkyl"), having a 1 to 8 carbon atoms (a "C₁-₈ alkyl"), having 1 to 6 carbon atoms (a "C₁-₆ alkyl"), having 2 to 6 carbon atoms (a "C₂-₆ alkyl"), or having 1 to 4 carbon atoms (a "C₁-₄ alkyl"). Examples of alkyl group include, but are not limited to, groups such as methyl, ethyl, n-propyl, isopropyl, n-butyl, t-butyl, isobutyl, sec-butyl, homologs and isomers of, for example, n-pentyl, n-hexyl, n-heptyl, n-octyl, and the like.

"Alkenyl" as used herein refers to an unsaturated linear (i.e., unbranched) or branched univalent hydrocarbon chain or combination thereof, having at least one site of olefinic unsaturation (*i.e.*, having at least one moiety of the formula C=C) and having the number of carbon atoms designated (i.e., C₂₋₁₀ means two to ten carbon atoms). The alkenyl group can be in "cis" or "trans" configurations, or alternatively in "E" or "Z" configurations. Particular alkenyl groups are those having 2 to 20 carbon atoms (a "C₂₋₂₀ alkenyl"), having a 2 to 8 carbon atoms (a "C₂₋₈ alkenyl"), having 2 to 6 carbon atoms (a "C₂₋₆ alkenyl"), or having 2 to 4 carbon atoms (a "C₂₋₄ alkenyl"). Example of alkenyl group include, but are not limited to, groups such as ethenyl (or vinyl), prop-1-enyl, prop-2-enyl (or allyl), 2-methylprop-1-enyl, but-1-enyl, but-2-enyl, but-3-enyl, buta-1,3-dienyl, 2-methylbuta-1,3-dienyl, homologs and isomers thereof, and the like.

"Alkynyl" as used herein refers to an unsaturated linear (i.e. unbranched) or branched univalent hydrocarbon chain or combination thereof, having at least one site of acetylenic unsaturation (i.e., having at least one moiety of the formula C=C) having the number of carbon atoms designated (i.e., C₂₋₁₀ means two to ten carbon atoms). Particular alkynyl groups are those having 2 to 20 carbon atoms (a "C₂₋₂₀ alkynyl"), having a 2 to 8 carbon atoms (a "C₂₋₈ alkynyl"), having 2 to 6 carbon atoms (a "C₂₋₆ alkynyl"), having 2 to 4 carbon atoms (a "C₂₋₄ alkynyl"). Examples of alkynyl group include, but are not limited to, groups such as ethynyl (or acetylenyl), prop-1-ynyl, prop-2-ynyl (or propargyl), but-1-ynyl, but-2-ynyl, but-3-ynyl, homologs and isomers thereof, and the like.

"Alkylene" as used herein refers to the same residues as alkyl, but having bivalency. Particular alkylene groups are those having 1 to 6 carbon atoms (a "C₁₋₆ alkylene"), 1 to 5 carbon atoms (a "C₁₋₅ alkylene"), having 1 to 4 carbon atoms (a "C₁₋₄ alkylene"), or 1 to 3 carbon atoms (a "C₁₋₃ alkylene"). Examples of alkylene include, but are not limited to, groups such as methylene (-CH₂-) , ethylene (-CH₂-CH₂-), propylene (-CH₂-CH₂-CH₂-), butylene (-CH₂-CH₂-CH₂-CH₂-), and the like.

"Cycloalkyl" as used herein refers to non-aromatic, saturated or unsaturated cyclic univalent hydrocarbon structures having the number of carbon atoms designated (*i*.*e*., (C₃-₁₀ means three to ten carbon atoms). Cycloalkyl can consist of one ring, such as cyclohexyl, or multiple rings, such as adamantly, but excludes aryl groups. A cycloalkyl comprising more than one ring can be fused, spiro, or bridged, or combinations thereof. Particular cycloalkyl groups are those having from 3 to 12 annular carbon atoms. A preferred cycloalkyl is a cyclic hydrocarbon having from 3 to 7 annular carbon atoms (a "C₃₋₈ cycloalkyl"), or having 3 to 6 carbon atoms (a "C₃₋₆ cycloalkyl "). Examples of cycloalkyl include, but are not limited to, cyclopropyl, cyclobutyl, cyclopentyl, cyclohyxyl, 1-cyclohexenyl, 3-cyclohexenyl, cycloheptyl, norbornyl, and the like.

"Heterocycloalkyl" as used herein refers to a cycloalkyl as defined herein where one or more of the ring carbon atoms have been replaced by a heteroatom such as, for example, nitrogen, oxygen, or sulfur. Representative examples of a heterocycloalkyl group include, but are not limited to, aziridinyl, azetidinyl, azepanyl, oxetanyl, pyrrolidyl, imidazolidinyl (e.g., imidazolidin-4-onyl or imidazolidin-2,4-dionyl), pyrazolidinyl, thiazolidinyl, tetrahydrothiophenyl, tetrahydrofuranyl, dioxolyl, pyrrolinyl, imidazolinyl, pyrazolinyl, thiazolinyl, piperidyl, piperidinyl, piperazinyl, piperazin-2-onyl, morpholinyl, thiomorpholinyl, tetrahydropyranyl (e.g., tetrahydro-2H-pyranyl), tetrahydrothiopyranyl, oxathianyl, dioxyl, dithianyl, pyranyl, dihydrodithiinyl, 1,4-dioxaspiro[4.5]decanyl, homopiperazinyl, quinuclidyl, and tetrahydropyrimidin-2(1H)-one groups.

"Aryl" as used herein refers to an unsaturated aromatic carbocyclic group having a single ring (e.g., phenyl) or multiple condensed rings (e.g., naphthyl or anthryl) which condensed rings can or can not be aromatic. Particular aryl groups are those having from 6 to 14 annular (i.e., ring) carbon atoms (a "C₆₋₁₄ aryl"). Preferred aryl groups include those having 5 to 6 ring carbons. An aryl group having more than one ring where at least one ring is non-aromatic can be connected to the parent structure at either an aromatic ring position or at a non-aromatic ring position. In one variation, an aryl group having more than one ring where at least one ring is non-aromatic is connected to the parent structure at an aromatic ring position.

"Heteroaryl" as used herein refers to an unsaturated aromatic cyclic group having from 1 to 14 annular (i.e., ring) carbon atoms and at least one annular heteroatom, including but not limited to heteroatoms such as nitrogen, phosphorus, oxygen and sulfur. A heteroaryl group can have a single ring (e.g., pyridyl, furyl) or multiple condensed rings (e.g., indolizinyl, benzothienyl) which condensed rings can or can not be aromatic. Particular heteroaryl groups are 5- to 14-membered rings having 1 to 12 annular (i.e., ring) carbon atoms and 1 to 6 annular (i.e., ring) heteroatoms independently selected from nitrogen, phosphorus, oxygen and sulfur; 5- to 10-membered rings having 1 to 8 annular carbon atoms and 1 to 4 annular heteroatoms independently selected from nitrogen, phosphorus, oxygen and sulfur; and 5-, 6- or 7-membered rings having 1 to 5 annular carbon atoms and 1 to 4 annular heteroatoms independently selected from nitrogen, oxygen and sulfur In one variation, heteroaryl include monocyclic aromatic 5-, 6- or 7-membered rings having from 1 to 6 annular carbon atoms and 1 to 4 annular heteroatoms independently selected from nitrogen, oxygen and sulfur. In another variation, heteroaryl includes polycyclic aromatic rings having from 1 to 12 annular carbon atoms and 1 to 6 annular heteroatoms independently selected from nitrogen, phosphorus, oxygen and sulfur. Still further, a heteroaryl as described herein can include rings have 5 or 6 members. A heteroaryl group having more than one ring where at least one ring is non-aromatic can be connected to the parent structure at either an aromatic ring position or at a non-aromatic ring position. In one variation, a heteroaryl group having more than one ring where at least one ring is non-aromatic is connected to the parent structure at an aromatic ring position.

"Halo" or Halogen" refers to fluoro, chloro, bromo and/or iodo. Where a residue is substituted with more than one halogen, it can be referred to by using a prefix corresponding to the number of halogen moieties attached, e.g., dihaloaryl, dihaloalkyl, trihaloaryl etc. refer to aryl and alkyl substituted with two ("di") or three ("tri") halo groups, which can be but are not necessarily the same halo; thus 4-chloro-3-fluorophenyl is within the scope of dihaloaryl. An alkyl group in which one or more hydrogen is replaced with a halo group is referred to as a "haloalkyl", for example, "C₁₋₆ haloalkyl." An alkyl group in which each hydrogen is replaced with a halo group is referred to as a "perhaloalkyl." A preferred perhaloalkyl group is trifluoroalkyl (-CF₃). Similarly, "perhaloalkoxy" refers to an alkoxy group in which a halogen takes the place of each H in the hydrocarbon making up the alkyl moiety of the alkoxy group. An example of a perhaloalkoxy group is trifluoromethoxy (-OCF₃).

The terms "treat" and "treatment" refer to therapeutic treatment, wherein the object is to slow down (lessen) an undesired physiological change or disorder, such as the development or spread of cancer. Beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those with the condition or disorder.

The phrase "effective amount" means an amount of a compound described herein that (i) treats the particular disease, condition, or disorder, (ii) attenuates, ameliorates, or eliminates one or more symptoms of the particular disease, condition, or disorder, (iii) prevents or delays the onset of one or more symptoms of the particular cancer described herein or (iv) favorably alters the clinical response of a patient to the treatment, where the inhibition and favorability is relative to a control (e.g. non-treatment or prior treatment with an anti-cancer agent such as that described herein). The therapeutically effective amount of the drug can reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug can prevent growth and/or kill existing cancer cells, it can be cytostatic and/or cytotoxic. For cancer therapy, efficacy can be measured, for example, by assessing the time to disease progression (TTP) and/or determining the response rate (RR).

The term "clinical response" refers to inhibition of disease progression, inhibition of tumor growth, reduction of primary tumor, relief of tumor-related symptoms, inhibition of tumor secreted factors (including tumor secreted hormones, such as those that contribute to carcinoid syndrome), delayed appearance of primary or secondary tumors, slowed development of primary or secondary tumors, decreased occurrence of primary or secondary tumors, slowed or decreased severity of secondary effects of disease, arrested tumor growth and regression of tumors, increased Time To Progression (TTP), increased Progression Free Survival (PFS), increased Overall Survival (OS), among others. OS as used herein means the time from treatment onset until death from any cause. In general, clinical response refers to primary or secondary measures of efficacy known and understood in the art. Treatment and clinical response as described herein can be assessed using international standards for a given condition.

The term "Time To Progression" or "TTP" as used herein refers to the time from treatment onset until tumor progression.

The term "Progression Free Survival" or "PFS" refers to the time from treatment onset until tumor progression or death. In one embodiment, PFS rates can be computed using the Kaplan-Meier estimates.

The clinical response of a patient described herein can be characterized as a complete or partial response. "Complete response" (CR) refers to an absence of clinically detectable cancer with normalization of any previously abnormal radiographic studies, bone marrow, and cerebrospinal fluid (CSF) or abnormal monoclonal protein measurements. "Partial response" (PR) refers to at least about a 10%, 20%, 30%, 40%, 50%, 60%, 70%, 80%, or 90% decrease in all measurable cancer burden (i.e., the number of malignant cells present in the subject, or the measured bulk of tumor masses or the quantity of abnormal monoclonal protein). The term "treatment" includes both a complete and a partial response.

The terms "patient" and "subject" are used interchangeably herein and refer to an animal, including, but not limited to, an animal such a cow, monkey, horse, sheep, pig, chicken, turkey, quail, cat, dog, mouse, rat, rabbit or guinea pig, in one embodiment a mammal, in another embodiment a human. In one embodiment, a subject is a human having or at risk for having cancer, in particular, a cancer described herein. In one embodiment, a patient is a human having histologically or cytologically-confirmed cancer, including subjects who have progressed on (or not been able to tolerate) standard anticancer therapy or for whom no standard anticancer therapy exists.

The terms "cancer" refers to or describe the physiological condition in mammals that is typically characterized by unregulated cell growth. A "tumor" comprises one or more cancerous cells. Examples of cancer include, but are not limited to, carcinoma, lymphoma, blastoma, sarcoma, and leukemia or lymphoid malignancies. More particular examples of such cancers include squamous cell cancer (e.g., epithelial squamous cell cancer), lung cancer including small- cell lung cancer, non-small cell lung cancer ("NSCLC"), small-cell lung cancer, non-small cell lung cancer (NSCLC), lung adenocarcinoma, squamous cell lung cancer, peritoneum cancer, hepatocellular cancer, stomach cancer, gastrointestinal cancer, esophageal cancer, pancreatic cancer, glioblastoma, cervical cancer, ovarian cancer, liver cancer, bladder cancer, breast cancer, colon cancer, rectal cancer, colorectal cancer, endometrial cancer, uterine cancer, salivary gland carcinoma, renal cancer, prostate cancer, vulval cancer, thyroid cancer, hepatocellular carcinoma (HCC), anal carcinoma, penile carcinoma, or head and neck cancer.

"Hematological malignancies" (British spelling "Haematological" malignancies) are the types of cancer that affect blood, bone marrow, and lymph nodes. As the three are intimately connected through the immune system, a disease affecting one of the three will often affect the others as well: although lymphoma is a disease of the lymph nodes, it often spreads to the bone marrow, affecting the blood. Hematological malignancies are malignant neoplasms (i.e. cancer), and they are generally treated by specialists in hematology and/or oncology. Hematological malignancies can derive from either of the two major blood cell lineages: myeloid and lymphoid cell lines. Lymphomas, lymphocytic leukemias, and myeloma are from the lymphoid line, while acute and chronic myelogenous leukemia, myelodysplastic syndromes and myeloproliferative diseases are myeloid in origin. Exemplary leukemias include acute lymphoblastic leukemia (ALL), acute myelogenous leukemia (AML), chronic lymphocytic leukemia (CLL), chronic myelogenous leukemia (CML), acute monocytic leukemia (AMOL) and small lymphocytic lymphoma (SLL). Exemplary lymphomas include Hodgkin's lymphomas (all four subtypes) and Non-Hodgkin's lymphomas (NHL, all subtypes).

A "anti-cancer agent" is a chemical compound useful in the treatment of cancer, regardless of mechanism of action. Classes of anti-cancer agents include, but are not limited to: alkylating agents, antimetabolites, anti-hormone therapies, endocrine therapies, immunomodulatory agents, spindle poison plant alkaloids, cytotoxic/antitumor antibiotics, topoisomerase inhibitors, antibodies, photosensitizers, and kinase inhibitors. Anti-cancer agents include compounds used in targeted therapy and conventional chemotherapy.

Examplary anti-cancer agents include proteasome inhibitors such as bortezomib (VELCADE), carfilzomib (KYPROLIS) and ixazomib (NINLARO). Other examples include immunomodulatory agents such as lenalidomide (REVLIMID) and pomalidomide (POMALYST).

Other exemplary anti-cancer agents include inhibitors of B-cell receptor targets such as BTK, Bcl-2 and JAK inhibitors and include, for example, venetoclax (VENCLEXTA) and ibrutinib (IMBRUVICA).

Additional anti-cancer agents include, for example, Abemaciclib (VERZENIO); abiraterone (ZYTIGA, YONSA); aclarubicin; acivicin; acodazole; acronine; actinomycin; acylfulvene; adecypenol; adozelesin; adriamycin; aldesleukin; altretamine; ambamustine; ambomycin; ametantrone; amidox; amifostine; aminoglutethimide; aminolevulinic acid; amrubicin; amsacrine; anagrelide; anastrozole; andrographolide; antarelix; anthramycin; aphidicolin glycinate; apurinic acid; ARRY-300; arabinoside; asperlin; asulacrine; atamestane; atrimustine; azasetron; azatoxin; azatyrosine; azacitidine; AZD6244; AZD8330; azetepa; azotomycin; balanol; batimastat; bendamustine; benzochlorins; benzodopa; benzoylstaurosporine; beta-alethine; betaclamycin B; betulinic acid; bicalutamide; binimetinib; bisantrene; bisaziridinylspermine; bisnafide; bistratene; bleomycin; busulfan; bizelesin; breflate; bortezomib; brequinar; bropirimine; budotitane; buthionine; bryostatin; cactinomycin; calusterone; calcipotriol; calphostin C; camptothecin; capecitabine (XELODA); caracemide; carbetimer; carboplatin; carboquone; carmustine; carubicin; carzelesin; castanospermine; celecoxib; cetrorelix; cetuximab (ERBITUX); chloroquinoxaline; cicaprost; chlorambucil; chlorofusin; cisplatin; cladribine; clomifene; clotrimazole; crisnatol; crisnatol; cypemycin; cyclophosphamide; cytarabine; cytostatin; dacarbazine; dactinomycin; daratumamab; daunorubicin; decarbazine; dacliximab; dasatinib; decitabine; deslorelin; dexamethasone; dexifosfamide; dexrazoxane; dexverapamil; dexormaplatin; dezaguanine; diaziquone; dihydrotaxol; docosanol; dolasetron; docetaxel; doxorubicin; doxifluridine; droloxifene; dromostanolone; dronabinol; duazomycin; ebselen; ecomustine; edelfosine; edrecolomab; edatrexate; eflornithine; elemene; emitefur; elsamitrucin; enloplatin; enpromate; epipropidine; epirubicin; epristeride; erbulozole; erlotinib (TARCEVA); esorubicin; estramustine; etanidazole; etoposide; etoprine; exemestane; fadrozole; fazarabine; fenretinide; filgrastim; finasteride; flavopiridol; flezelastine; fluasterone; floxuridine; fludarabine; fludarabine; fluorodaunorubicin; forfenimex; formestane; fluorouracil; floxouridine; flurocitabine; fosquidone; fostriecin; fotemustine; fulvestrant (FASLODEX); gadolinium; gallium; galocitabine; ganirelix; gemcitabine; geldanamycin; gefitinib; gossyphol; hydroxyurea; hepsulfam; heregulin; ibandronate; ibrutinib; idarubicin; idelalisib (ZYDELIG), ifosfamide; canfosfamide; ilmofosine; iproplatin; idoxifene; idramantone; ilmofosine; ilomastat; imatinib mesylate (GLEEVEC); imiquimod; iobenguane; iododoxorubicin; ipomeanol; irinotecan; itasetron; iimofosine; lanreotide; lapatinib (TYKERB); leinamycin; lenograstim; lentinan; leptolstatin; letrozole; leuprorelin; levamisole; liarozole; lobaplatin; lombricine; lometrexol; lonidamine; lonafarnib (SARASAR); losoxantrone; lovastatin; loxoribine; lurtotecan; lapatinib; leucovorin; lometrexol; lomustine; maitansine; marimastat; masoprocol; maspin; menogaril; merbarone; meterelin; methioninase; metoclopramide; mifepristone; miltefosine; mirimostim; mitoguazone; mitolactol; mitonafide; mitoxantrone; mofarotene; molgramostim; mopidamol; maytansine; megestrol acetate; melengestrol acetate; melphalan; mercaptopurine; methotrexate; methotrexate sodium; metoprine; meturedepa; mitinmitomycin; mitosper; mitotane; mitoxantrone; mycophenolic acid; nafarelin; nagrestip; napavin; nedaplatin; nemorubicin; neridronic acid; nilutamide; nisamycin; oblimersen (GENASENSE); octreotide; okicenone; onapristone; ondansetron; ormaplatin; oxisuran; oxaloplatin; osaterone; oxaliplatin; oxaunomycin; palauamine; palbociclib (IBRANCE); panitumumab (VECTIBIX); panomifene; pegaspargase; picibanil; pirarubicin; piritrexim; prednisone; prednisolone, paclitaxel; nab-paclitaxel (ABRAXANE); prednimustine; procarbazine; puromycin; raltitrexed; ramosetron; rapamycin (RAPAMUNE); rhizoxin; ribociclib (KISQALI), rituximab; rogletimide; rohitukine; romurtide; roquinimex; romidepsin; safingol; saintopin; sargramostim; semustine; sizofiran; sobuzoxane; sorafenib (NEXAVAR); sunitinib; spiromustine; squalamine; suradista; suramin; swainsonine; spiroplatin; streptonigrin; streptozocin; sulofenur; tallimustine; tamoxifen; tauromustine; tazarotene; tellurapyrylium; temoporfin; temozolomide; teniposide; tetrachlorodecaoxide; tetrazomine; thrombopoietin; thymalfasin; thymotrinan; tirapazamine; toremifene; tretinoin; trimetrexate; triptorelin; tropisetron; talisomycin; taxotere; teroxirone; testolactone; thiamiprine; thiotepa; tirapazamine; toremifene; trastuzumab; trastuzumab emtansine; trestolone acetate; triciribine phosphate; trimetrexate; uracil mustard; vandetanib (CAPRELSA); variolin B; velaresol; veramine; verteporfin; vemurafenib; vinorelbine; vinxaltine; vitaxin; vinblastine; vincristine; vindesine; vinepidine; vinglycinate; vinleurosine; vinorelbine; vinrosidine; vinzolidine; vorozole; wortmannin; zanoterone; zeniplatin; zilascorb; zinostatin stimalamer; zinostatin; and zorubicin.

In some embodiments, an anti-cancer agent includes, for example, idelalisib (ZYDELIG), docetaxel, fluorouracil, gemcitabine (GEMZAR), cisplatin, cis-diamine, carboplatin, paclitaxel, nab-paclitaxel, trastuzumab (HERCEPTIN), temozolomide, tamoxifen, 4-hydroxytamoxifen, and doxorubicin.

Also included in the definition of anti-cancer agent are: (i) anti-estrogens and selective estrogen receptor modulators (SERMs), including, for example, tamoxifen, raloxifene, droloxifene, 4-hydroxytamoxifen, trioxifene, ketoxifene, LY117018, onapristone, and toremifine citrate; (ii) selective estrogen receptor modulators (SERDs) such as brilanestrant, GDC-0927, GDC-9545, AZ9496, AZ9833, GNE-274, and fulvestrant (FASLODEX); (iii) aromatase inhibitors such as, for example, 4(5)-imidazoles, aminoglutethimide, megestrol acetate, exemestane, formestanie, fadrozole, vorozole, letrozole, and anastrozole; (iv) anti-androgens such as apalutamide, abiraterone, enzalutamide, flutamide, nilutamide, bicalutamide, leuprolide, and goserelin.

Further included in the definition of anti-cancer agents are: (iv) MEK inhibitors such as cobimetinib; (v) lipid kinase inhibitors, such as taselisib; (vi) antisense oligonucleotides such as oblimersen; (vii) ribozymes such as VEGF expression inhibitors such as angiozyme; (viii) vaccines such as gene therapy vaccines, for example, ALLOVECTIN, LEUVECTIN, and VAXID; (ix) topoisomerase 1 inhibitors such as LURTOTECAN; ABARELIX rmRH; and (x) anti-angiogenic agents such as bevacizumab.

In some embodiments herein, the anti-cancer agents is a therapeutic antibody such as atezolizumab, nivolumab, daratumumab, pembrolizumab, alemtuzumab, bevacizumab; cetuximab; panitumumab, rituximab, pertuzumab, trastuzumab, trastuzumab emtansine, or tositumomab.

A "metabolite" is a product produced through metabolism in the body of a specified compound or salt thereof. Metabolites of a compound can be identified using routine techniques and their activities determined using tests such as those described herein. Such products can result for example from the oxidation, reduction, hydrolysis, amidation, deamidation, esterification, deesterification, enzymatic cleavage, and the like, of the administered compound. Accordingly, further provided herein are metabolites of compounds or pharmaceutically acceptable salts thereof described herein, including compounds produced by a process comprising contacting a compound of pharmaceutically acceptable salt thereof described herein with a mammal for a period of time sufficient to yield a metabolic product thereof.

The term "package insert" is used to refer to instructions customarily included in commercial packages of therapeutic products, that contain information about the indications, usage, dosage, administration, contraindications and/or warnings concerning the use of such therapeutic products.

The term "chiral" refers to molecules which have the property of nonsuperimposability of the mirror image partner, while the term "achiral" refers to molecules which are superimposable on their mirror image partner.

The term "stereoisomers" refers to compounds which have identical chemical constitution, but differ with regard to the arrangement of the atoms or groups in space.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers can separate under high resolution analytical procedures such as electrophoresis and chromatography.

"Enantiomers" refer to two stereoisomers of a compound which are nonsuperimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The compounds or pharmaceutically acceptable salts thereof as described herein can contain asymmetric or chiral centers, and therefore exist in different stereoisomeric forms. It is intended that all stereoisomeric forms of the compounds or pharmaceutically acceptable salts thereof as described herein, including but not limited to, diastereomers, enantiomers and atropisomers, as well as mixtures thereof such as racemic mixtures, are included herein. Many organic compounds exist in optically active forms, i.e., they have the ability to rotate the plane of plane-polarized light. In describing an optically active compound, the prefixes D and L, or *R* and *S*, are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer can also be referred to as an enantiomer, and a mixture of such isomers is often called an enantiomeric mixture. A 50:50 mixture of enantiomers is referred to as a racemic mixture or a racemate, which can occur where there has been no stereoselection or stereospecificity in a chemical reaction or process. The terms "racemic mixture" and "racemate" refer to an equimolar mixture of two enantiomeric species, devoid of optical activity. Enantiomers can be separated from a racemic mixture by a chiral separation method, such as supercritical fluid chromatography (SFC). Assignment of configuration at chiral centers in separated stereoisomers can be tentative, and depicted in Table 1 structures for illustrative purposes, before stereochemistry is definitively established, such as from x-ray crystallographic data.

The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

The term "pharmaceutically acceptable salts" denotes salts which are not biologically or otherwise undesirable. Pharmaceutically acceptable salts include both acid and base addition salts. The phrase "pharmaceutically acceptable" indicates that the substance or composition must be compatible chemically and/or toxicologically, with the other ingredients comprising a formulation, and/or the mammal being treated therewith.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, aryl-aliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, malonic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid "mesylate", ethanesulfonic acid, p-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, and polyamine resins.

A "solvate" refers to an association or complex of one or more solvent molecules and a compound or pharmaceutically acceptable salt thereof as described herein. Examples of solvents that form solvates include, but are not limited to, water (i.e., "hydrate"), isopropanol, ethanol, methanol, DMSO, ethylacetate (EtOAc), acetic acid (AcOH), and ethanolamine.

The term "EC₅₀" is the half maximal effective concentration" and denotes the plasma concentration of a particular compound required for obtaining 50% of the maximum of a particular effect in vivo.

The term "Ki" is the inhibition constant and denotes the absolute binding affinity of a particular inhibitor to a receptor. It is measured using competition binding assays and is equal to the concentration where the particular inhibitor would occupy 50% of the receptors if no competing ligand (e.g. a radioligand) was present. Ki values can be converted logarithmically to pKi values (-log Ki), in which higher values indicate exponentially greater potency.

The term "IC₅₀" is the half maximal inhibitory concentration and denotes the concentration of a particular compound required for obtaining 50% inhibition of a biological process in vitro. IC₅₀ values can be converted logarithmically to pIC₅₀ values (-log IC₅₀), in which higher values indicate exponentially greater potency. The IC₅₀ value is not an absolute value but depends on experimental conditions e.g. concentrations employed, and can be converted to an absolute inhibition constant (Ki) using the Cheng-Prusoff equation (Biochem. Pharmacol. (1973) 22:3099). Other percent inhibition parameters, such as IC₇₀, IC₉₀, etc., can be calculated.

Any formula or structure given herein, including Formula I compounds, is intended to represent unlabeled forms as well as isotopically labeled forms of the compounds. Isotopically labeled compounds have structures depicted by the formulas given herein except that one or more atoms are replaced by an atom having a selected atomic mass or mass number. Examples of isotopes that can be incorporated into compounds or pharmaceutically acceptable salts thereof as described herein include isotopes of hydrogen, carbon, nitrogen, oxygen, phosphorous, fluorine, and chlorine, such as, but not limited to ²H (deuterium, D), ³H (tritium), ¹¹C, ¹³C, ¹⁴C, ¹⁵N, ¹⁸F, ³¹P, ³²P, ³⁵S, ³⁶Cl, and ¹²⁵I. Various isotopically labeled compounds or pharmaceutically acceptable salts thereof as described herein, for example those into which radioactive isotopes such as ³H and ¹⁴C are incorporated. Such isotopically labeled compounds can be useful in metabolic studies, reaction kinetic studies, detection or imaging techniques, such as positron emission tomography (PET) or single-photon emission computed tomography (SPECT) including drug or substrate tissue distribution assays, or in radioactive treatment of patients. Deuterium labeled or substituted therapeutic compounds or pharmaceutically acceptable salts thereof as described herein can have improved DMPK (drug metabolism and pharmacokinetics) properties, relating to distribution, metabolism, and excretion (ADME). Substitution with heavier isotopes such as deuterium can afford certain therapeutic advantages resulting from greater metabolic stability, for example increased in vivo half-life or reduced dosage requirements. An 18F labeled compound can be useful for PET or SPECT studies. Isotopically labeled compounds or pharmaceutically acceptable salts thereof as described herein can generally be prepared by carrying out the procedures disclosed in the schemes or in the examples and preparations described below by substituting a readily available isotopically labeled reagent for a non-isotopically labeled reagent. Further, substitution with heavier isotopes, particularly deuterium (i.e., ²H or D) can afford certain therapeutic advantages resulting from greater metabolic stability, for example increased in vivo half-life or reduced dosage requirements or an improvement in therapeutic index. It is understood that deuterium in this context is regarded as a substituent in the compound of the formula (I). The concentration of such a heavier isotope, specifically deuterium, can be defined by an isotopic enrichment factor. In the compounds or pharmaceutically acceptable salts thereof as described herein any atom not specifically designated as a particular isotope is meant to represent any stable isotope of that atom. Unless otherwise stated, when a position is designated specifically as "H" or "hydrogen", the position is understood to have hydrogen at its natural abundance isotopic composition. Accordingly, in the compounds or pharmaceutically acceptable salts thereof as described herein any atom specifically designated as a deuterium (D) is meant to represent deuterium.

### Pyrimidinyl-heteroaryloxy-naphthyl

Provided herein are compounds having formula (I) or formula (II); 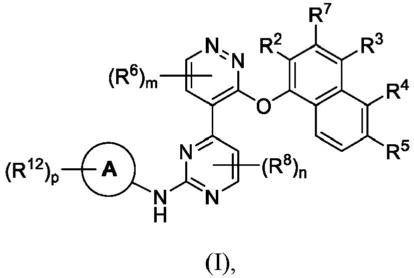 or 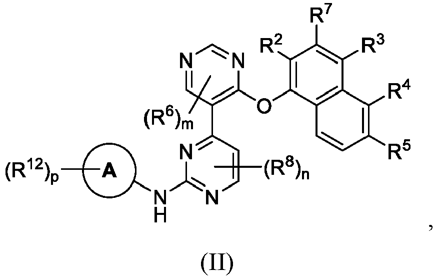
or a pharmaceutically acceptable salt thereof,
wherein;
   R¹ is hydrogen, halogen, -CN, -NO₂, -NRaR^{b}, -NR^{a}C(O)R^{b}, -NR^{a}C(O)R^{b},-NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a},-OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b},-P(O)R^{a}R^{b}, R¹⁰-substituted or unsubstituted C₁₋₆ alkyl, R¹⁰-substituted or unsubstituted 2 to 6 membered heteroalkyl, R¹⁰-substituted or unsubstituted C₁₋₆ haloalkyl, R¹⁰-substituted or unsubstituted C₃₋C₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heterocycloalkyl;
   R² is hydrogen, halogen, -CN, R¹⁰-substituted or unsubstituted C₁₋₆ alkoxy, or R¹⁰-substituted or unsubstituted C₁₋₆ alkyl;
   R³ is -L¹-R¹, hydrogen, halogen, -CN, -NO₂, -NRaR^{b}, -NR^{a}C(O)R^{b}, -NR^{a}C(O)R^{b}, -NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a},-OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b},-P(O)R^{a}R^{b}, R¹⁰-substituted or unsubstituted C₁₋₆ alkoxy, R¹⁰-substituted or unsubstituted C₁₋₆ haloalkyl, R¹⁰-substituted or unsubstituted C₁₋₆ alkyl, R¹⁰-substituted or unsubstituted C₃₋₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heteroaryl;
   R⁴ is -L²-R⁹-, halogen, -CN, -NO₂, -NR^{a}R^{b}, -NR^{a}C(O)R^{b}, -NR^{a}C(O)R^{b},-NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a},-OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b},-P(O)R^{a}R^{b}, R¹⁰-substituted or unsubstituted C₁₋₆ alkoxy, R¹⁰-substituted or unsubstituted C₁₋₆ haloalkyl, R¹⁰-substituted or unsubstituted C₁₋₆ alkyl, R¹⁰-substituted or unsubstituted 2 to 6 membered heteroalkyl, R¹⁰-substituted or unsubstituted C₃₋₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heteroaryl;
   R⁵ is hydrogen, halogen, or R¹⁰-substituted or unsubstituted C₁₋₃ alkyl;
   each R⁶ is independently hydrogen, halogen, -CN, -NO₂, -OR^{a}, -NR^{a}R^{b}, -C(O)R^{a},-C(O)OR^{a}, -SR^{a}, -S(O)₂R^{a}, -P(O)R^{a}R^{b}, R¹⁰-substituted or unsubstituted C₁₋₆ alkoxy, R¹⁰-substituted or unsubstituted C₁₋₆ haloalkyl, R¹⁰-substituted or unsubstituted C₁₋₆ alkyl, R¹⁰-substituted or unsubstituted C₃₋₆ cycloalkyl, -SO₂(C₁₋₆ alkyl), or -SO₂(C₁₋₆ haloalkyl);
   R⁷ is hydrogen, halogen, -CN, R¹⁰-substituted or unsubstituted C₁₋₆ alkyl, R¹⁰-substituted or unsubstituted C₁₋₆ alkoxy, R¹⁰-substituted or unsubstituted C₁₋₆ haloalkyl, or R¹⁰-substituted or unsubstituted C₃₋₆ cycloalkyl;
   each R⁸ is independently hydrogen, halogen, or R¹⁰-substituted or unsubstituted C₁₋₃ alkyl;
   R⁹ is hydrogen, halogen, R¹⁰-substituted or unsubstituted C₁₋₆ alkyl, R¹⁰-substituted or unsubstituted 2 to 6 membered heteroalkyl, R¹⁰-substituted or unsubstituted C₁₋₆ haloalkyl, R¹⁰-substituted or unsubstituted C₃₋C₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heterocycloalkyl;
   each R¹⁰ is independently halogen, -N₃, -CF₃, -CN, -NO₂, -NR^{a}R^{b}, -NR^{a}C(O)R^{b}, -NR^{a}C(O)R^{b}, -NR^{a}C(O)OR^{b}, -NR^{a}C(O)NR^{b}, -S(O)₂NR^{a}, -C(O)R^{a}, -C(O)OR^{a}, -C(O)NR^{a}R^{b},-OR^{a}, -OC(O)R^{a}, -OC(O)NR^{a}R^{b}, -SR^{a}, -S(O)R^{a}, -S(O)₂R^{a}, -S(O)₃R^{a}, -S(O)(=NH)R^{a},-S(O)₂NR^{a}R^{b}, -P(O)R^{a}R^{b}, R¹¹-substituted or unsubstituted C₁₋₆ alkyl, R¹¹-substituted or unsubstituted 2 to 6 membered heteroalkyl, R¹¹-substituted or unsubstituted C₃₋₇ cycloalkyl, R¹¹-substituted or unsubstituted 3 to 7 membered heterocycloalkyl, R¹¹-substituted or unsubstituted C₅₋₆ aryl, or R¹¹-substituted or unsubstituted 5 to 6 membered heteroaryl;
   each R¹¹ is independently halogen, -N₃, -CF₃, -CCl₃, -CBr₃, -CI₃, -CN, -CHO, -OH, -NH₂, -COOH, -CONH₂, -NO₂, -SH, -SO₂Cl, -SO₃H, -SO₄H, -SO₂NH₂, unsubstituted C₁₋₆ alkyl, unsubstituted 2 to 6 membered heteroalkyl, unsubstituted C₃₋₇ cycloalkyl, unsubstituted 3 to 7 membered heterocycloalkyl, unsubstituted C₅₋₆ aryl, or unsubstituted 5 to 6 membered heteroaryl;
   each R¹² is independently hydrogen, halogen, a NPG, or R¹⁰-substituted or unsubstituted C₁₋₃ alkyl;
   L¹ is -NHSO₂-, -NHC(O)-, NHCO(O)-, -NHC(O)NH-, -NH-, -O-, -S-, or -SO₂-;
   L² is -NHSO₂-, -NHC(O)-, NHCO(O)-, -NHC(O)NH-, -NH-, -O-, -S-, or -SO₂-;
   each R^{a} and R^{b} is independently hydrogen, unsubstituted C₁₋₆ alkyl, unsubstituted C₁₋₆ haloalkyl, unsubstituted C₁₋₆ alkoxy, unsubstituted C₂₋₆ alkenyl, unsubstituted C₂₋₆ alkynyl, unsubstituted C₃₋₇ cycloalkyl, unsubstituted 3 to 7 membered heterocycloalkyl, unsubstituted C₅₋₆ aryl, or unsubstituted 5 or 6 membered heteroaryl;
   m is 1 or 2;
   n is 1 or 2;
   p is 1, 2, 3, 4, 5 or 6; and
   Ring A is C₃₋₆ cycloalkyl, 3 to 6 membered heterocycloalkyl, C₅₋₆ aryl, or 5 or 6 membered heteroaryl.

In another aspect provided herein the compounds are compounds comprising formula (I). In another aspect provided herein the compounds are compounds comprising formula (II).

In one embodiment, ring A comprises a 3 to 6 membered heterocycloalkyl. In certain embodiments, where ring a comprises a 3 to 6 membered heterocycloalkyl. In another embodiment, ring A is 6 membered heterocycloalkyl. In still another embodiment, ring A is a 6 membered heterocycloalkyl comprising one or more nitrogen atoms. In still another embodiment, ring A is azetindyl, oxetanyl, pyrrolyl, furanyl, pyridinyl, piperidinyl, piperazinyl, pyrimidinyl, pyradizinyl, tetrahydropyranyl, or pyranyl. In one embodiment, ring a is piperidinyl.

In certain embodiments, each R¹² is independently halogen or C1-3 alkyl. In one embodiment, each R¹² is independently halogen (e.g. F or Cl). In another embodiment, each R¹² is independently F, where n is 1 or 2. In another embodiment, each R¹² is independently methyl, where n is 1 or 2. In still another embodiment, n is 2 where each R¹² is independently F and methyl. In such embodiments, two R¹² moieties can be geminal on ring A. In another embodiment, ring A is piperidinyl substituted at the 3-position.

In one embodiment, ring A has the structure:

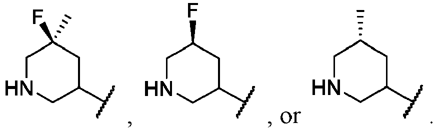

In one embodiment, ring A has the structure:

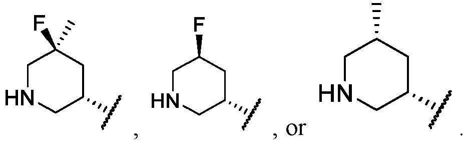

In one embodiment, ring A has the structure:

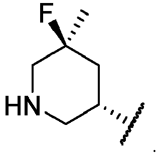

R² can be hydrogen, halogen, or -CN. In another embodiment, R² is R¹⁰-substituted or unsubstituted C₁₋₆ alkoxy, or R¹⁰-substituted or unsubstituted C₁₋₆ alkyl. In one embodiment, R² is hydrogen or unsubstituted C₁₋₃ alkyl. In one embodiment, R² is hydrogen. In one embodiment, R² is methyl.

R³ can be hydrogen, halogen, -CN, -NO₂, -NRaR^{b}, -NR^{a}C(O)R^{b}, -NR^{a}C(O)R^{b},-NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a},-OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b},-P(O)R^{a}R^{b}, where R^{a} and R^{b} are as defined herein. In one embodiment, R^{a} and R^{b} are independently hydrogen or methyl. In one embodiment, R³ is R¹⁰-substituted or unsubstituted C₁₋₆ alkoxy, R¹⁰-substituted or unsubstituted C₁₋₆ haloalkyl, or R¹⁰-substituted or unsubstituted C₁₋₆ alkyl. In another embodiment, R³ is R¹⁰-substituted or unsubstituted C₃₋₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heteroaryl. In one embodiment, R³ is hydrogen. In another embodiment, R³ is methyl.

In another embodiment, R³ is -L¹-R¹. In such embodiments, L¹ is -NHS(O)₂- or-NHC(O)-. R¹ can be hydrogen, halogen, R¹⁰-substituted or unsubstituted C₁₋₆ alkyl, R¹⁰-substituted or unsubstituted C₁₋₆ haloalkyl, R¹⁰-substituted or unsubstituted C₃₋C₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heterocycloalkyl. In one embodiment, where R³ is -L¹-R¹, L¹ is -NHS(O)₂- or -NHC(O)- and R¹ is R¹⁰-substituted or unsubstituted C₁₋₆ alkyl, R¹⁰-substituted or unsubstituted C₃₋C₇ cycloalkyl, or R¹⁰-substituted or unsubstituted C₅₋₆ aryl.

R⁴ can be halogen, -CN, -NO₂, -NR^{a}R^{b}, -NR^{a}C(O)R^{b}, -NR^{a}C(O)R^{b}, -NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a}, -OC(O)R^{a},-OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b}, -P(O)R^{a}R^{b}, where R^{a} and R^{b} are as defined herein. In one embodiment, R^{a} and R^{b} are independently hydrogen or methyl. In another embodiment, R⁴ is R¹⁰-substituted or unsubstituted C₁₋₆ alkoxy, R¹⁰-substituted or unsubstituted C₁₋₆ haloalkyl, or R¹⁰-substituted or unsubstituted C₁₋₆ alkyl. In still another embodiment, R⁴ is R¹⁰-substituted or unsubstituted C₃₋₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heteroaryl.

In one aspect provided herein R⁴ is -L²-R⁹-. In one embodiment, L² is -NHSO₂-,-NHC(O)-. In another embodiment, L² is -NH-, -O-, -S-, or -SO₂-. In one embodiment, L² is-NHSO₂-. In another embodiment, L² is -NHC(O)-. Where R⁴ is -L²-R⁹-, R⁹ can be R¹⁰-substituted phenyl and R¹⁰ is halogen. In one embodiment, R⁹ is phenyl substituted with one Cl or F. In another embodiment, R⁹ is phenyl disubstituted with 2 halogens (e.g. Cl or F).

In another embodiment, where R⁴ is -L²-R⁹-, R⁹ is R¹⁰-substituted or unsubstituted C₃₋₆ cycloalkyl. In one embodiment, R⁹ is unsubstituted C₃₋₆ cycloalkyl. In another embodiment, R⁹ is unsubstituted cyclopropanyl.

In another embodiment, where R⁴ is -L²-R⁹-, R⁹ is 2 to 6 membered unsubstituted haloalkyl. In one embodiment, R⁹ is a 4 membered unsubstituted haloalkyl, where the halogen atom is Cl or F. In another embodiment, R⁹ is 3,3 difluorobutyl.

In another embodiment, where R⁴ is -L²-R⁹-, R⁹ is R¹⁰-substituted or unsubstituted C₁₋₃ alkyl. In one embodiment, In another embodiment, R⁹ is unsubstituted C₁₋₃ alkyl. In still another embodiment, R⁹ is methyl or propyl.

In one embodiment, each R¹⁰ is independently halogen, -N₃, -CF₃, -CN, -NO₂,-NR^{a}R^{b}, -NR^{a}C(O)R^{b}, -NR^{a}C(O)R^{b}, -NR^{a}C(O)OR^{b}, -NR^{a}C(O)NR^{b}, -S(O)₂NR^{a}, -C(O)R^{a},-C(O)OR^{a}, -C(O)NR^{a}R^{b}, -OR^{a}, -OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b}, or -P(O)R^{a}R^{b}, where R^{a} and R^{b} are as defined herein. In one embodiment, R^{a} and R^{b} are independently hydrogen or methyl. In another embodiment, R¹⁰ is R¹¹-substituted or unsubstituted C₁₋₆ alkyl, R¹¹-substituted or unsubstituted 2 to 6 membered heteroalkyl. In still another embodiment, R¹⁰ is R¹¹-substituted or unsubstituted C₃₋₇ cycloalkyl, R¹¹-substituted or unsubstituted 3 to 7 membered heterocycloalkyl, R¹¹-substituted or unsubstituted C₅₋₆ aryl, or R¹¹-substituted or unsubstituted 5 to 6 membered heteroaryl.

In one embodiment, each R¹¹ is independently halogen, -N₃, -CF₃, -CCl₃, -CBr₃,-CI₃, -CN, -CHO, -OH, -NH₂, -COOH, -CONH₂, -NO₂, -SH, -SO₂Cl, -SO₃H, -SO₄H, or-SO₂NH₂. In another embodiment, each R¹¹ is independently unsubstituted C₁₋₆ alkyl, unsubstituted 2 to 6 membered heteroalkyl, unsubstituted C₃₋₇ cycloalkyl, unsubstituted 3 to 7 membered heterocycloalkyl, unsubstituted C₅₋₆ aryl, or unsubstituted 5 to 6 membered heteroaryl. In one embodiment, each R¹¹ is independently halogen. In another embodiment, each R¹¹ is independently -OH, -NH₂, -COOH, -CONH₂, or -SH. In another embodiment, each R¹¹ is independently unsubstituted C₁₋₆ alkyl (e.g. methyl, ethyl, or propyl). In still another embodiment, each R¹¹ is independently unsubstituted C₃₋₇ cycloalkyl or unsubstituted C₅₋₆ aryl. For example, each R¹¹ can independently be cyclopropyl, cyclobutyl, cyclopentyl, cyclohexyl, or phenyl.

In one embodiment, R⁵ is hydrogen or halogen. In another embodiment, R⁵ is R¹⁰-substituted or unsubstituted C₁₋₃ alkyl. In another embodiment, R⁵ is hydrogen. In still another embodiment, R⁵ is unsubstituted C₁₋₃ alkyl. In still another embodiment, R⁵ is methyl. In still another embodiment, R⁵ is halogen (e.g. Cl or F).

In one embodiment, each R⁶ is independently hydrogen, halogen, -CN, -NO₂, -OR^{a}, -NR^{a}R^{b}, -C(O)R^{a}, -C(O)OR^{a}, -SR^{a}, -S(O)₂R^{a}, or -P(O)R^{a}R^{b}. In another embodiment, each R⁶ is independently R¹⁰-substituted or unsubstituted C₁₋₆ alkoxy, R¹⁰-substituted or unsubstituted C₁₋₆ haloalkyl, or R¹⁰-substituted or unsubstituted C₁₋₆ alkyl. In another embodiment, each R⁶ is independently R¹⁰-substituted or unsubstituted C₃₋₆ cycloalkyl, -SO₂(C₁₋₆ alkyl), or-SO₂(C₁₋₆ haloalkyl). In one embodiment, each R⁶ is independently hydrogen. In another embodiment, each R⁶ is independently halogen (e.g. Cl or F). In another embodiment, each R⁶ is independently -CN. In still another embodiment, each R⁶ is independently R¹⁰-substituted or unsubstituted C₁₋₆ alkyl. In one embodiment, each R⁶ is independently methyl. In embodiments, m is 1.

In one embodiment, R⁷ is hydrogen, halogen, -CN, or R¹⁰-substituted or unsubstituted C₁₋₆ alkyl. In another embodiment, R⁷ is R¹⁰-substituted or unsubstituted C₁₋₆ alkoxy or R¹⁰-substituted or unsubstituted C₁₋₆ haloalkyl. In still another embodiment, R⁷ is R¹⁰-substituted or unsubstituted C₃₋₆ cycloalkyl. In certain embodiments, R⁷ is hydrogen. In one embodiment, R⁷ is halogen (e.g. F or Cl). In one embodiment, R⁷ is methyl. In another embodiment, R⁷ is unsubstituted cyclopropyl.

In one embodiment, each R⁸ is independently hydrogen or halogen. In another embodiment, each R⁸ is independently R¹⁰-substituted or unsubstituted C₁₋₃ alkyl. In certain embodiments, each R⁸ is independently hydrogen. In one embodiment, each R⁸ is independently F. In another embodiment, each R⁸ is independently methyl. In one embodiment, n is 1.

In one embodiment, each R¹² is independently hydrogen, halogen, or a nitrogen protecting group (NPG). In another embodiment, each R¹² is independently R¹⁰-substituted or unsubstituted C₁₋₃ alkyl. In one embodiment, the NPG comprises, for example, tert-Butyloxycarbonyl (Boc), 9-fluorenylmethoxycarbonyl (Fmoc), benzyloxycarbonyl (Cbz), trityl (Trt), or allylozycarbonyl (Alloc), α,α-Dimethyl-3,5-dimethoxybenzyloxycarbonyl (Ddz), 2-(4-Biphenyl)isopropoxycarbonyl (Bpoc), 2-Nitrophenylsulfenyl (Nps), 2-(4-Nitrophenylsulfonyl)ethoxycarbonyl (Nsc), 1,1-Dioxobenzo[b]thiophene-2-ylmethyloxycarbonyl (Bsmoc), - (1-(4,4-Dimethyl-2,6-dioxocyclohex-1-ylidene)-3-ethyl) (Dde), Tetrachlorophthaloyl (TCP), 2-[Phenyl(methyl)sulfonio]ethyloxycarbonyl tetrafluoroborate (Pms), Ethanesulfonylethoxycarbonyl (Esc), 2-(4-Sulfophenylsulfonyl)ethoxycarbonyl (Sps), 2,4-Dinitrobenzenesulfonyl (dNBS), Benzothiazole-2-sulfonyl (Bts), - 2,2,2-Trichloroethyloxycarbonyl (Troc), or p-Nitrobenzyloxycarbonyl (pNZ). In one embodiment, the NPG is Boc. In another embodiment, the NPG is yl (Trt), α,α-Dimethyl-3,5-dimethoxybenzyloxycarbonyl (Ddz), 2-(4-Biphenyl)isopropoxycarbonyl (Bpoc), or 2-Nitrophenylsulfenyl (Nps).

In another aspect provided herein are compounds having the formula; 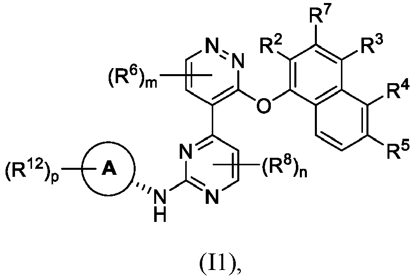 or 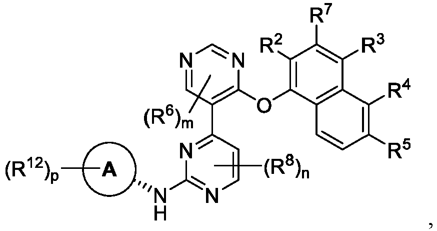 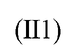 wherein R², R³, R⁴, R⁵, R⁶, R⁷, R⁸, R¹², m, n, p, and ring A are as defined herein.

In certain embodiments, compounds of formula (I) have formula: 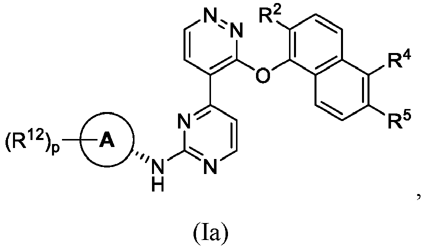 or a pharmaceutically acceptable salt thereof, where R², R⁴, R⁵, R¹², p, and ring A are as defined herein.

In another embodiment, compounds of formula (I) have the formula: 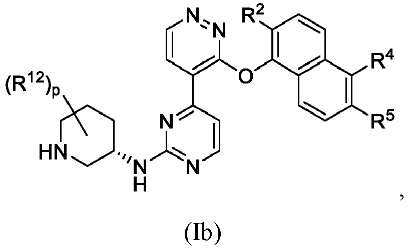 or a pharmaceutically acceptable salt thereof, where R², R⁴, R⁵, R¹², p, and ring A are as defined herein

In another embodiment, compounds of formula (I) have the formula: 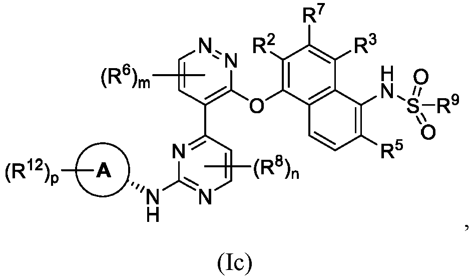 or a pharmaceutically acceptable salt thereof, where R², R³, R⁵, R⁶, R⁷, R⁸, R⁹, R¹², m, n, p, and ring A are as defined herein.

In another embodiment, compounds of formula (I) have the formula: 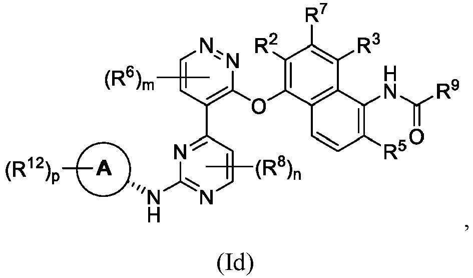 or a pharmaceutically acceptable salt thereof, where R², R³, R⁵, R⁶, R⁷, R⁸, R⁹, R¹², m, n, p, and ring A are as defined herein.

In another embodiment, compounds of formula (I) have the formula: 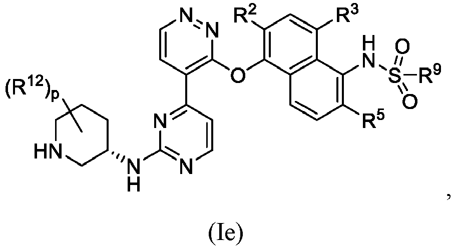 or a pharmaceutically acceptable salt thereof, where R², R³, R⁵, R⁹, R¹², and p are as defined herein.

In another embodiment, compounds of formula (I) have the formula: 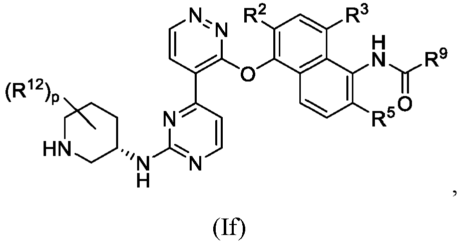 or a pharmaceutically acceptable salt thereof, where R², R³, R⁵, R⁹, R¹², and p are as defined herein.

Also provided herein are compounds of formula (II) having the formula: 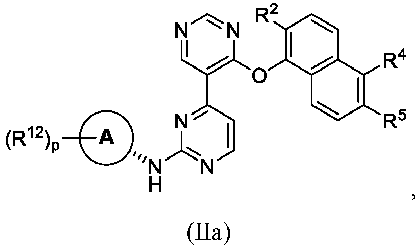 or a pharmaceutically acceptable salt thereof, where R², R⁴, R⁵, R¹², p, and ring A are as defined herein.

In another embodiment, compounds of formula (II) have the formula: 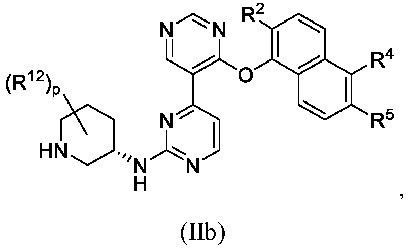 or a pharmaceutically acceptable salt thereof, where R², R⁴, R⁵, R¹², and p are as defined herein.

In another embodiment, compounds of formula (II) have the formula: 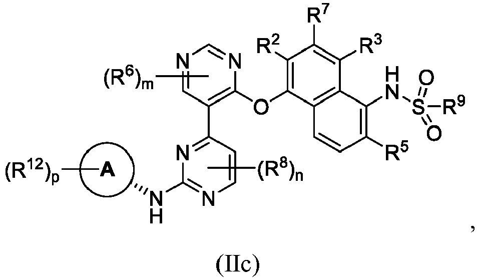 or a pharmaceutically acceptable salt thereof, where R², R³, R⁵, R⁶, R⁷, R⁸, R⁹, R¹², m, n, p, and ring A are as defined herein.

In another embodiment, compounds of formula (II) have the formula: 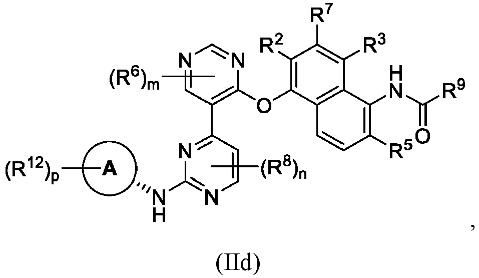 or a pharmaceutically acceptable salt thereof, where R², R³, R⁵, R⁶, R⁷, R⁸, R⁹, R¹², m, n, p, and ring A are as defined herein.

In another embodiment, compounds of formula (II) have the formula: 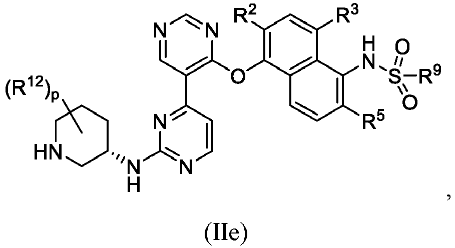 or a pharmaceutically acceptable salt thereof, where R², R³, R⁵, R⁹, R¹², and p are as defined herein.

In another embodiment, compounds of formula (II) have the formula: 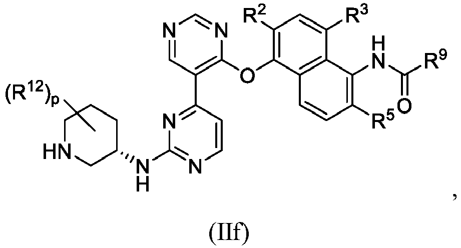 or a pharmaceutically acceptable salt thereof, where R², R³, R⁵, R⁹, R¹², and p are as defined herein.

In one embodiment, the compound of Formula (I) or Formula (II) is a compound set forth in Table 1. It is understood that individual enatiomers and diastereomers are included in the table below by Compound No. and Compound Name, and their corresponding structures can be readily determined therefrom. In some instances, the enantiomers or diastereomers are identified by their respective perperties, for example, retention times on a chiral HPLC or its biological activities.

**Table 1**

| No | Structure | Name |
|---|---|---|
| 1 | 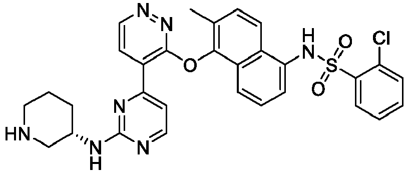 | (*S*)-2-chloro-*N*-(6-methyl-5-((4-(2-(piperidin-3-ylamino)pyrimidin-4-yl)pyridazin-3-yl)oxy)naphthalen-1-yl)benzenesulfonamide |
| 2 | 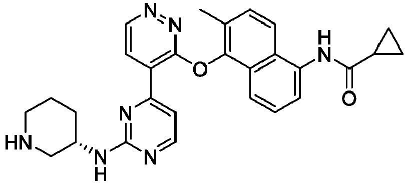 | (*S*)-*N*-(6-methyl-5-((4-(2-(piperidin-3-ylamino)pyrimidin-4-yl)pyridazin-3-yl)oxy)naphthalen-1-yl)cyclopropanecarboxamide |
| 3 | 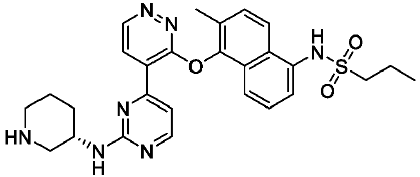 | (*S*)-*N*-(6-methyl-5-((4-(2-(piperidin-3-ylamino)pyrimidin-4-yl)pyridazin-3-yl)oxy)naphthalen-1-yl)propane-1-sulfonamide |
| 4 | 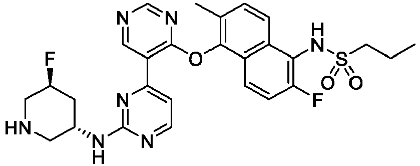 | *N*-(2-fluoro-5-((2-(((3*S*,5*S*)-5-fluoropiperidin-3-yl)amino)-[4,5'-bipyrimidin]-4'-yl)oxy)-6-methylnaphthalen-1-yl)propane-1-sulfonamide |
| 5 | 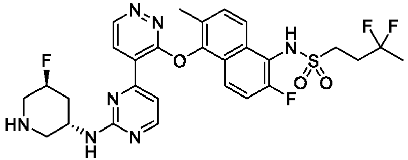 | 3,3-difluoro-*N*-[2-fluoro-6-methyl-5-[4-[2-[[-(3*S*,5*S*)-5-fluoro-3-piperidyl]amino] pyrimidin-4-yl]pyridazin-3-yl]oxy-1-naphthyl]butane-1-sulfonamide |
| 6 | 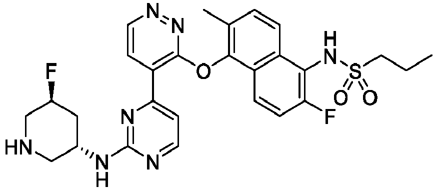 | N-(2-fluoro-5-((4-(2-(((3S,5S)-5-fluoropiperidin-3-yl)amino)pyrimidin-4-yl)pyridazin-3-yl)oxy)-6-methylnaphthalen-1-yl)propane-1-sulfonamide |
| 7 | 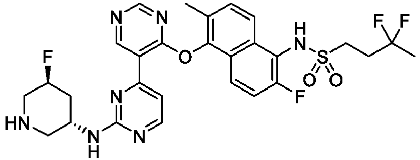 | 3,3-difluoro-*N*-(2-fluoro-5-((2-(((3*S*,5S)-5-fluoropiperidin-3-yl)amino)-[4,5'-bipyrimidin]-4'-yl)oxy)-6-methylnaphthalen-1-yl)butane-1-sulfonamide |
| 8 | 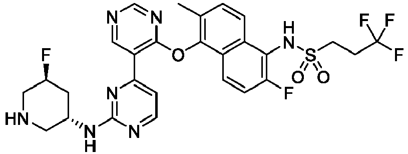 | 3,3,3-trifluoro-*N*-(2-fluoro-5-((2-(((3*S*,5*S*)-5-fluoropiperidin-3-yl)amino)-[4,5'-bipyrimidin]-4'-yl)oxy)-6-methylnaphthalen-1-yl)propane-1-sulfonamide |
| 9 | 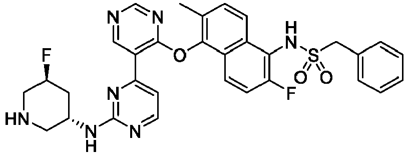 | *N*-(2-fluoro-5-((2-(((3*S*,5*S*)-5-fluoropiperidin-3-yl)amino)-[4,5'-bipyrimidin]-4'-yl)oxy)-6-methylnaphthalen-1-yl)-1-phenylmethanesulfonamide |

Thus, in another aspect provided herein is a compound selected from Table 1, or a pharmaceutically acceptable salt thereof.

In one embodiment, the compound of Formula (I) or Formula (II) is a compound set forth in Table 2. It is understood that individual enatiomers and diastereomers are included in the table below by Compound No. and Compound Name, and their corresponding structures can be readily determined therefrom. In some instances, the enantiomers or diastereomers are identified by their respective perperties, for example, retention times on a chiral HPLC or its biological activities, and the absolute stereo configurations of the chiral centers are arbitrarily assigned.

**Table 2:**

| No | Structure | Name |
|---|---|---|
| 10 | 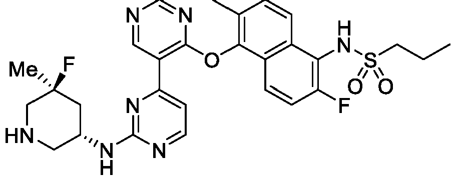 | *N*-(2-fluoro-5-((2-(((3*S*,5*S*)-5-fluoro-5-methylpiperidin-3-yl)amino)-[4,5'-bipyrimidin]-4'-yl)oxy)-6-methylnaphthalen-1-yl)propane-1-sulfonamide |
| 11 | 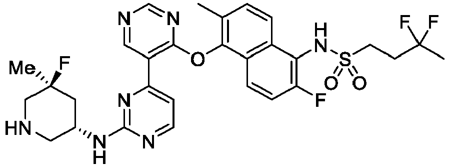 | 3,3-difluoro-*N*-(2-fluoro-5-((2-(((3*S*,5*S*)-5-fluoro-5-methylpiperidin-3-yl)amino)-[4,5'-bipyrimidin]-4'-yl)oxy)-6-methylnaphthalen-1-yl)butane-1-sulfonamide |
| 12 | 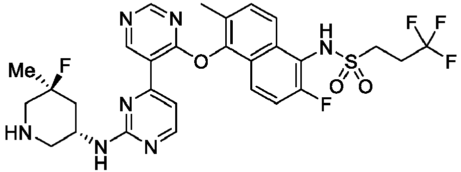 | 3,3,3-trifluoro-*N*-(2-fluoro-5-((2-(((3*S*,5*S*)-5-fluoro-5-methylpiperidin-3-yl)amino)-[4,5'-bipyrimidin]-4'-yl)oxy)-6-methylnaphthalen-1-yl)propane-1-sulfonamide |
| 13 | 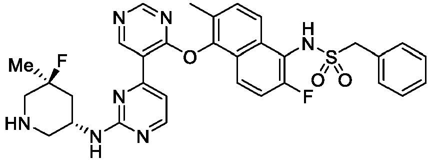 | *N*-(2-fluoro-5-((2-(((3*S*,5*S*)-5-fluoro-5-methylpiperidin-3-yl)amino)-[4,5'-bipyrimidin]-4'-yl)oxy)-6-methylnaphthalen-1-yl)-1-phenylmethanesulfonamide |
| 14 | 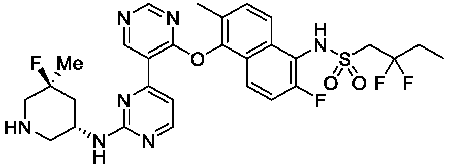 | 2,2-difluoro-*N*-(2-fluoro-5-((2-(((3*S*,5*S*)-5-fluoro-5-methylpiperidin-3-yl)amino)-[4,5'-bipyrimidin]-4'-yl)oxy)-6-methylnaphthalen-1-yl)butane-1-sulfonamide |
| 15 | 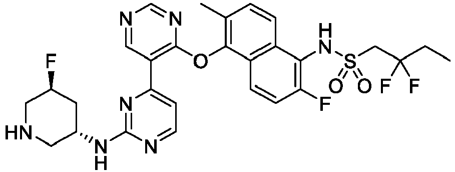 | 2,2-difluoro-*N*-(2-fluoro-5-((2-(((3*S*,5*S*)-5-fluoropiperidin-3-yl)amino)-[4,5'-bipyrimidin]-4'-yl)oxy)-6-methylnaphthalen-1-yl)butane-1-sulfonamide |
| 16 | 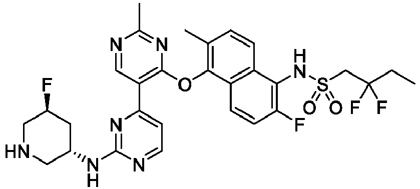 | 2,2-difluoro-*N*-(2-fluoro-5-((2-(((3*S*,5*S*)-5-fluoropiperidin-3-yl)amino)-2'-methyl-[4,5'-bipyrimidin]-4'-yl)oxy)-6-methylnaphthalen-1-yl)butane-1-sulfonamide |
| 17 | 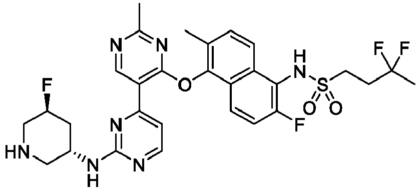 | 3,3-difluoro-*N*-(2-fluoro-5-((2-(((3*S*,5*S*)-5-fluoropiperidin-3-yl)amino)-2'-methyl-[4,5'-bipyrimidin]-4'-yl)oxy)-6-methylnaphthalen-1-yl)butane-1-sulfonamide |
| 18 | 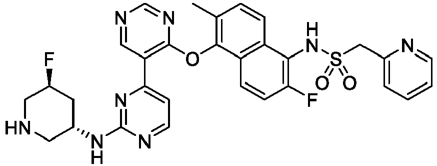 | *N*-(2-fluoro-5-((2-(((3*S*,5*S*)-5-fluoropiperidin-3-yl)amino)-[4,5'-bipyrimidin]-4'-yl)oxy)-6-methylnaphthalen-1-yl)-1-(pyridin-2-yl)methanesulfonamide |
| 19 | 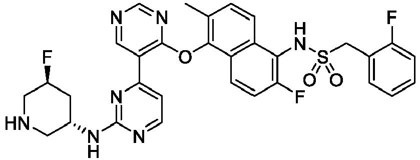 | *N*-(2-fluoro-5-((2-(((3*S*,5*S*)-5-fluoropiperidin-3-yl)amino)-[4,5'-bipyrimidin]-4'-yl)oxy)-6-methylnaphthalen-1-yl)-1-(2-fluorophenyl)methanesulfonamide |
| 20 | 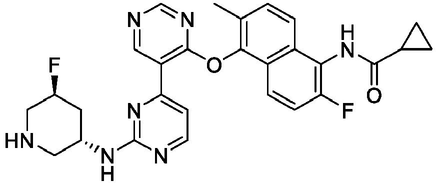 | *N*-(2-fluoro-5-((2-(((3*S*,5*S*)-5-fluoropiperidin-3-yl)amino)-[4,5'-bipyrimidin]-4'-yl)oxy)-6-methylnaphthalen-1-yl)cyclopropanecarboxamide |
| 21 | 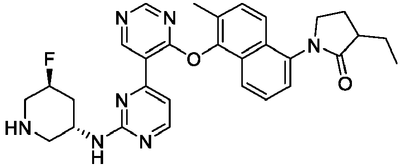 | 3-ethyl-1-(5-((2-(((3*S*,5*S*)-5-fluoropiperidin-3-yl)amino)-[4,5'-bipyrimidin]-4'-yl)oxy)-6-methylnaphthalen-1-yl)pyrrolidin-2-one |
| 22 | 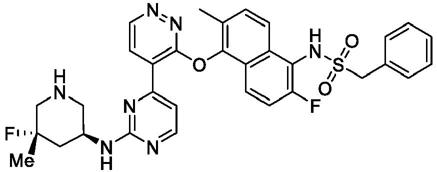 | *N*-(2-fluoro-5-((4-(2-(((3*S*,5*S*)-5-fluoro-5-methylpiperidin-3-yl)amino)pyrimidin-4-yl)pyridazin-3-yl)oxy)-6-methylnaphthalen-1-yl)-1-phenylmethanesulfonamide |
| 23 | 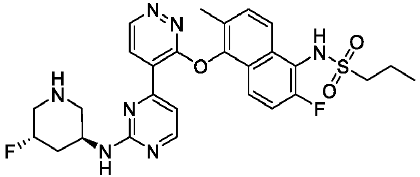 | *N*-(2-fluoro-5-((4-(2-(((3*S*,5S)-5-fluoropiperidin-3-yl)amino)pyrimidin-4-yl)pyridazin-3-yl)oxy)-6-methylnaphthalen-1-yl)propane-1-sulfonamide |
| 24 | 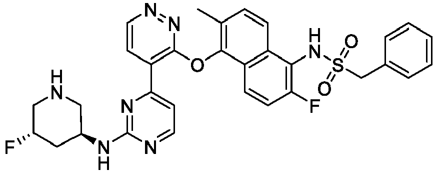 | *N*-(2-fluoro-5-((4-(2-(((3*S*,5*S*)-5-fluoropiperidin-3-yl)amino)pyrimidin-4-yl)pyridazin-3-yl)oxy)-6-methylnaphthalen-1-yl)-1-phenylmethanesulfonamide |
| 25 | 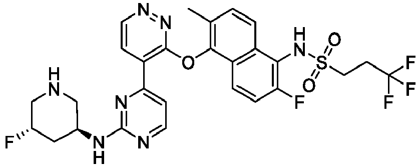 | 3,3,3-trifluoro-*N-*(2-fluoro-5-((4-(2-(((3*S*,5*S*)-5-fluoropiperidin-3-yl)amino)pyrimidin-4-yl)pyridazin-3-yl)oxy)-6-methylnaphthalen-1-yl)propane-1-sulfonamide |

Thus, in another aspect provided herein is a compound selected from Table 2, or a pharmaceutically acceptable salt thereof.

### Preparation of Compounds

Compounds described herein can be synthesized by synthetic routes that include processes analogous to those well-known in the chemical arts, particularly in light of the description contained herein, and those for other heterocycles described in: Comprehensive Heterocyclic Chemistry II, Editors Katritzky and Rees, Elsevier, 1997, e.g. Volume 3; Liebigs Annalen der Chemie, (9):1910-16, (1985); Helvetica Chimica Acta, 41:1052-60, (1958); Arzneimittel-Forschung, 40(12):1328-31, (1990). Starting materials are generally available from commercial sources such as Aldrich Chemicals (Milwaukee, WI) or are readily prepared using methods (e.g., prepared by methods generally described in Louis F. Fieser and Mary Fieser, Reagents for Organic Synthesis, v. 1-23, Wiley, N.Y. (1967-2006 ed.), or Beilsteins Handbuch der organischen Chemie, 4, Aufl. ed. Springer-Verlag, Berlin, including supplements (also available via the Beilstein online database). Formula I compounds can also be made following the procedures found in US 8476434, US 7880000, WO 2005/113494, US 7868177, and WO 2007/100646.

Synthetic chemistry transformations and protecting group methodologies (protection and deprotection) useful in synthesizing compounds described herein and necessary reagents and intermediates include, for example, those described in R. Larock, Comprehensive Organic Transformations, VCH Publishers (1989); T. W. Greene and P. G. M. Wuts, Protective Groups in Organic Synthesis, 3rd Ed., John Wiley and Sons (1999); and L. Paquette, ed., Encyclopedia of Reagents for Organic Synthesis, John Wiley and Sons (1995) and subsequent editions thereof.

Compounds described herein can be prepared singly or as compound libraries comprising at least 2, for example 5 to 1,000 compounds, or 10 to 100 compounds. Libraries comprising compounds described herein can be prepared by a combinatorial 'split and mix' approach or by multiple parallel syntheses using, for example, either solution phase or solid phase chemistry. Thus according to a further aspect provided herein is a compound library comprising at least 2 compounds described herein, or pharmaceutically acceptable salts thereof.

The Examples provide exemplary methods for preparing compounds described herein. Those skilled in the art will appreciate that other synthetic routes can be used to synthesize the compounds described herein. Although specific starting materials and reagents are depicted and discussed in the Examples, other starting materials and reagents can be easily substituted to provide a variety of derivatives and/or reaction conditions. In addition, many of the exemplary compounds prepared by the described methods can be further modified in light of this disclosure using conventional chemistry.

In preparing compounds described herein, protection of remote functionality (e.g., primary or secondary amine) of intermediates can be necessary. The need for such protection will vary depending on the nature of the remote functionality and the conditions of the preparation methods. Suitable amino-protecting groups include acetyl, trifluoroacetyl, t-butoxycarbonyl (BOC), benzyloxycarbonyl (CBz) and 9-fluorenylmethyleneoxycarbonyl (Fmoc). The need for such protection can be readily determined. For a general description of protecting groups and their use, see T. W. Greene, Protective Groups in Organic Synthesis, John Wiley & Sons, New York, 1991.

In the methods of preparing compounds described herein, it can be advantageous to separate reaction products from one another and/or from starting materials. The desired products of each step or series of steps is separated and/or purified to the desired degree of homogeneity by the techniques common in the art. Typically such separations involve multiphase extraction, crystallization from a solvent or solvent mixture, distillation, sublimation, or chromatography. Chromatography can involve any number of methods including, for example: reverse-phase and normal phase; size exclusion; ion exchange; high, medium and low pressure liquid chromatography methods and apparatus; small scale analytical; simulated moving bed (SMB) and preparative thin or thick layer chromatography, as well as techniques of small scale thin layer and flash chromatography.

Another class of separation methods involves treatment of a mixture with a reagent selected to bind to or render otherwise separable a desired product, unreacted starting material, reaction by product, or the like. Such reagents include adsorbents or absorbents such as activated carbon, molecular sieves, ion exchange media, or the like. Alternatively, the reagents can be acids in the case of a basic material, bases in the case of an acidic material, binding reagents such as antibodies, binding proteins, selective chelators such as crown ethers, liquid/liquid ion extraction reagents (LIX), or the like. Selection of appropriate methods of separation depends on the nature of the materials involved, such as, boiling point and molecular weight in distillation and sublimation, presence or absence of polar functional groups in chromatography, stability of materials in acidic and basic media in multiphase extraction, and the like.

Diastereomeric mixtures can be separated into their individual diastereomers on the basis of their physical chemical differences by methods such as by chromatography and/or fractional crystallization. Enantiomers can be separated by converting the enantiomeric mixture into a diastereomeric mixture by reaction with an appropriate optically active compound (e.g., chiral auxiliary such as a chiral alcohol or Mosher's acid chloride), separating the diastereomers and converting (e.g., hydrolyzing) the individual diastereoisomers to the corresponding pure enantiomers. Also, some of the compounds or pharmaceutically acceptable salts thereof as described herein can be atropisomers (e.g., substituted biaryls) and included herein. Enantiomers can also be separated by use of a chiral HPLC column.

A single stereoisomer, e.g., an enantiomer, substantially free of its stereoisomer can be obtained by resolution of the racemic mixture using a method such as formation of diastereomers using optically active resolving agents (Eliel, E. and Wilen, S. "Stereochemistry of Organic Compounds," John Wiley & Sons, Inc., New York, 1994; Lochmuller, C. H., (1975) J. Chromatogr., 113(3):283-302). Racemic mixtures of chiral compounds or pharmaceutically acceptable salts thereof as described herein can be separated and isolated by any suitable method, including: (1) formation of ionic, diastereomeric salts with chiral compounds and separation by fractional crystallization or other methods, (2) formation of diastereomeric compounds with chiral derivatizing reagents, separation of the diastereomers, and conversion to the pure stereoisomers, and (3) separation of the substantially pure or enriched stereoisomers directly under chiral conditions. See: "Drug Stereochemistry, Analytical Methods and Pharmacology," Irving W. Wainer, Ed., Marcel Dekker, Inc., New York (1993).

Under method (1), diastereomeric salts can be formed by reaction of enantiomerically pure chiral bases such as brucine, quinine, ephedrine, strychnine, α-methyl-β-phenylethylamine (amphetamine), and the like with asymmetric compounds bearing acidic functionality, such as carboxylic acid and sulfonic acid. The diastereomeric salts can be induced to separate by fractional crystallization or ionic chromatography. For separation of the optical isomers of amino compounds, addition of chiral carboxylic or sulfonic acids, such as camphorsulfonic acid, tartaric acid, mandelic acid, or lactic acid can result in formation of the diastereomeric salts.

Alternatively, by method (2), the substrate to be resolved is reacted with one enantiomer of a chiral compound to form a diastereomeric pair (E. and Wilen, S. "Stereochemistry of Organic Compounds", John Wiley & Sons, Inc., 1994, p. 322). Diastereomeric compounds can be formed by reacting asymmetric compounds with enantiomerically pure chiral derivatizing reagents, such as menthyl derivatives, followed by separation of the diastereomers and hydrolysis to yield the pure or enriched enantiomer. A method of determining optical purity involves making chiral esters, such as a menthyl ester, e.g., (-) menthyl chloroformate in the presence of base, or Mosher ester, α-methoxy-α-(trifluoromethyl)phenyl acetate (Jacob III. J. Org. Chem. (1982) 47:4165), of the racemic mixture, and analyzing the ¹H NMR spectrum for the presence of the two atropisomeric enantiomers or diastereomers. Stable diastereomers of atropisomeric compounds can be separated and isolated by normal- and reverse-phase chromatography following methods for separation of atropisomeric naphthyl-isoquinolines (WO 96/15111). By method (3), a racemic mixture of two enantiomers can be separated by chromatography using a chiral stationary phase ("Chiral Liquid Chromatography" (1989) W. J. Lough, Ed., Chapman and Hall, New York; Okamoto, J. Chromatogr., (1990) 513:375-378). Enriched or purified enantiomers can be distinguished by methods used to distinguish other chiral molecules with asymmetric carbon atoms, such as optical rotation and circular dichroism.

### Biological Evaluation

The relative efficacies of Formula (I) or Formula (II) compounds as inhibitors of an enzyme activity (or other biological activity) can be established by determining the concentrations at which each compound inhibits the activity to a predefined extent and then comparing the results. Typically, the preferred determination is the concentration that inhibits 50% of the activity in a biochemical assay, i.e., the 50% inhibitory concentration or "IC₅₀". Determination of IC₅₀ values can be accomplished using conventional techniques. In general, an IC₅₀ can be determined by measuring the activity of a given enzyme in the presence of a range of concentrations of the inhibitor under study. The experimentally obtained values of enzyme activity then are plotted against the inhibitor concentrations used. The concentration of the inhibitor that shows 50% enzyme activity (as compared to the activity in the absence of any inhibitor) is taken as the IC₅₀ value. Analogously, other inhibitory concentrations can be defined through appropriate determinations of activity. For example, in some settings it can be desirable to establish a 90% inhibitory concentration, i.e., IC₉₀, etc.

Inhibition of IRE1α RNase activity was determined in an enzyme assay that measured cleavage of the XBP1 stem loop by autophosphorylated IRE1α. This assay format was chosen to ensure that inhibitors of either the IRE1α kinase or the RNase domains would be identified. Binding to the ATP pocket and inhibition of IRE1α kinase activity are not necessarily required to inhibit the RNase activity. Compounds were also profiled in cellular assays by direct measurement of XBP1s (B-DNA assay) or by quantification of the luciferase signal in HT1080 XBP1-Luc, which carries a luciferase fusion that is only in frame and expressed from the spliced XBP1 transcript. In the IRE1α enzyme and XPB1-Luc assays, compounds described herein (e.g., compounds in Table 1) demonstrated activity.

Cell proliferation, cytotoxicity, and cell viability of the Formula (I) or Formula (II) compounds can be measured by the CellTiter-Glo^{®} Luminescent Cell Viability Assay (Promega Corp.). The CellTiter-Glo^{®} Luminescent Cell Viability Assay is a homogeneous method of determining the number of viable cells in culture based on quantitation of the ATP present, an indicator of metabolically active cells. The CellTiter-Glo^{®} Assay is designed for use with multiwell formats, making it ideal for automated high-throughput screening (HTS), cell proliferation and cytotoxicity assays. The homogeneous assay procedure involves adding the single reagent (CellTiter-Glo^{®} Reagent) directly to cells cultured in serum-supplemented medium. Cell washing, removal of medium and multiple pipetting steps are not required. The system detects as few as 15 cells/well in a 384-well format in 10 minutes after adding reagent and mixing.

Biological activity of Formula (I) or Formula (II) compounds was measured by an IRE1 biochemical binding assay (Example 7), a biochemical RNase assay (Example 8), a cellular PD assay, XBP1s-LUC reporter (Example 9), and an IRE1α-based inhibition of multiple myeloma (MM) cell proliferation assay.

### Administration of Compounds

Compounds described herein can be administered by any route appropriate to the condition to be treated. Suitable routes include oral, parenteral (including subcutaneous, intramuscular, intravenous, intraarterial, intradermal, intrathecal and epidural), transdermal, rectal, nasal, topical (including buccal and sublingual), vaginal, intraperitoneal, intrapulmonary and intranasal. For local immunosuppressive treatment, the compounds can be administered by intralesional administration, including perfusing or otherwise contacting the graft with the inhibitor before transplantation. It will be appreciated that the preferred route can vary with for example the condition of the recipient. Where the compound is administered orally, it can be formulated as a pill, capsule, tablet, etc. with a pharmaceutically acceptable carrier or excipient. Where the compound is administered parenterally, it can be formulated with a pharmaceutically acceptable parenteral vehicle and in a unit dosage injectable form, as detailed below.

Thus, in one aspect provided herein is a pharmaceutical composition comprising a compound or pharmaceutically acceptable salt thereof as described herein and one or more pharmaceutically acceptable excipients. In one embodiment, compounds described herein are administered as pharmaceutical compositions capable of being administered to a subject orally or parenterally. The compounds described herein can be formulated for topical or parenteral use where the compound is dissolved or otherwise suspended in a solution suitable for injections, suspensions, syrups, creams, ointments, gels, sprays, solutions and emulsions.

Oral administration can promote patient compliance in taking the compound (e.g. formulated as a pharmaceutical composition), thereby increasing compliance and efficacy. Oral pharmaceutical compositions comprising a compound described herein include, but are not limited to, tablets (e.g. coated, non-coated and chewable) and capsules (e.g. hard gelatin capsules, soft gelatin capsules, enteric coated capsules, and sustained release capsules). Tablets can be prepared by direct compression, by wet granulation, or by dry granulation. Oral pharmaceutical compositions comprising a compound described herein can be formulated for delayed or prolonged release.

A dose to treat human patients can range from about 10 mg to about 1000 mg of Formula (I) or Formula (II) compound. A typical dose can be about 100 mg to about 300 mg of the compound. A dose can be administered once a day (QID), twice per day (BID), or more frequently, depending on the pharmacokinetic and pharmacodynamic properties, including absorption, distribution, metabolism, and excretion of the particular compound. In addition, toxicity factors can influence the dosage and administration regimen. When administered orally, the pill, capsule, or tablet can be ingested daily or less frequently for a specified period of time. The regimen can be repeated for a number of cycles of therapy.

### Methods of Treatment

In one aspect provided herein, the compounds provided herein are useful for treating cancer. The cancer can be associated with the unfolded protein response (UPR) pathway. Thus, provided herein are methods of treating cancer by administering to a patient having cancer an effective amount of a compound or pharmaceutically acceptable salt thereof described herein. It is to be understood that the methods provided herein include administration of compounds or pharmaceutically acceptable salts thereof formulated as a pharmaceutical composition as set forth herein. Furthermore, as already noted, all references herein to a particular method of treatment are, in the context of the present invention, to be interpreted as being directed to the relevant compound, pharmaceutical salt thereof, or pharmaceutical composition for use in the specified method. For instance, references to a particular embodiment of a method are to be interpreted as being directed to an embodiment of the relevant compound, pharmaceutical salt thereof, or pharmaceutical composition for use in the method being specifically described.

The methods provided herein include treatment of solid tumors/cancers. For example, adminstration of a compound or pharmaceutically acceptable salt thereof described herein can be performed for patients having breast cancer, ovary cancer, cervix cancer, prostate cancer, testis cancer, genitourinary tract cancer, esophagus cancer, larynx cancer, glioblastoma, neuroblastoma, stomach cancer, skin cancer, keratoacanthoma, lung cancer, epidermoid carcinoma, large cell cancer, non-small cell lung cancer (NSCLC), small cell carcinoma, lung adenocarcinoma, bone cancer, colon cancer, adenoma, pancreatic cancer, adenocarcinoma, thyroid cancer, follicular carcinoma, undifferentiated carcinoma, papillary carcinoma, seminoma, melanoma, sarcoma, bladder carcinoma, liver carcinoma and biliary passages, kidney carcinoma, buccal cavity cancer, naso-pharyngeal cancer, pharynx cancer, lip cancer, tongue cancer, mouth cancer, small intestine cancer, colon-rectum cancer, large intestine cancer, rectum cancer, bronchial cancer, hepatocellular cancer, gastric cancer, endometrial cancer, melanoma, renal cancer, urinary bladder cancer, uterine corpus cancer, and uterine cervix cancer.

In another embodiment, the methods provided herein include treatment of cancer by administering to a patient having cancer an effective amount of a compound or pharmaceutically acceptable salt thereof where the cancer comprises squamous cell carcinoma, small-cell lung cancer, non-small cell lung cancer (NSCLC), lung adenocarcinoma, squamous cell lung cancer, peritoneum cancer, hepatocellular cancer, stomach cancer, gastrointestinal cancer, esophageal cancer, pancreatic cancer, glioblastoma, cervical cancer, ovarian cancer, liver cancer, bladder cancer, breast cancer, colon cancer, rectal cancer, colorectal cancer, endometrial cancer, uterine cancer, salivary gland carcinoma, renal cancer, prostate cancer, vulval cancer, thyroid cancer, hepatocellular carcinoma (HCC), anal carcinoma, penile carcinoma, or head and neck cancer.

In certain embodiments, the cancer is breast cancer. The breast cancer can be Stage I, II, III, or IV as understood in the art. In one embodiment, the breast cancer is triple negative breast cancer (TNBC). In another embodiment, the breast cancer is Her2 negative breast cancer.

In another aspect provided herein are methods of treating hematological cancers such as, for example, lymphoma, lymphocytic leukemia (acute (ALL) and chronic (CLL), multiple myeloma (MM), acute myelogenous leukemia (AML), chronic myelogenous leukemia (CML), myelodysplastic syndrome (MDS), myeloproliferative disease (MPD), or non-Hodgkin lymphoma. In one embodiment, the methods herein include treatment of lymphoma, lymphocytic leukemia, multiple myeloma (MM), acute myelogenous leukemia (AML), chronic myelogenous leukemia (CML), myelodysplastic syndrome (MDS), or myeloproliferative disease (MPD) by administering an effective amount of a compound described herein.

In one embodiment, the method is a method of treating MM by administering to a patient having MM an effective amount of compound described herein.

In one embodiment, the cancer is an Ire1-mediated cancer (i.e. a cancer having abnormal expression or activity of Ire1 relative to a control). In one embodiment, the Ire1-mediated cancer has increased expression of Ire1. In another embodiment, the Ire1-mediated cancer has increased activity of Ire1. Such increases can be measured against a control (e.g. against a patient having predetermined Ire1 function, expression, activity; or for example measure in a single patient before, during, or after treatment with a compound or pharmaceutically acceptable salt thereof described herein).

Thus, in one aspect provided herein is a method of treating a disease caused by abnormal levels of Ire1 activity in a human or animal patient having such abnormal levels of Ire1 activity by administering a compound or pharmaceutically acceptable salt thereof described herein. The disease can be caused by an amount of Ire1 activity that is too low or too high. For example, the disease can be caused by a deficiency in Ire1 activity or by abnormally high Ire1 activity (e.g., hyperactivity of Ire1). The method includes administering to the patient a therapeutically effective amount of an Ire1 modulator Formula (I) or Formula (II) compound.

Ire1 deficiency is a decreased amount of Ire1 activity compared to normal levels of Ire1 activity in a particular subject or a population of healthy subjects. The decreased amount of Ire1 activity results in excessive amounts of misfolded protein accumulation thereby causing the disease state.

Ire1 hyperactivity is an increased amount of Ire1 activity compared to normal levels of Ire1 activity in a particular subject or a population of healthy subjects. The increased amount of Ire1 activity can result in, for example, excessive amounts of cell proliferation thereby causing the disease state.

In one embodiment, the disease is associated with Ire1 deficiency. Such diseases include, but are not limited to, cystic fibrosis, retinitis pigmentosa, diabetes, or a neurodegenerative disease. The neurodegenerative disease can include Alexander's disease, Alper's disease, Alzheimer's disease, Amyotrophic lateral sclerosis, Ataxia telangiectasia, Batten disease (also known as Spielmeyer-Vogt-Sjogren-Batten disease). Bovine spongiform encephalopathy (BSF), Canavan disease, Cockayne syndrome, Corticobasal degeneration, Creutzfeldt-Jakob disease, Huntington's disease, HIV-associated dementia, Kennedy's disease, Krabbe's disease, Lewy body dementia, Machado-Joseph disease (Spinocerebellar ataxia type 3), Multiple sclerosis, Multiple System Atrophy, Narcolepsy, Neuroborreliosis, Parkinson's disease, Pelizaeus-Merzbacher Disease, Pick's disease, Primary lateral sclerosis, Prion diseases, Refsum's disease, Sandhoffs disease, Schilder's disease, Subacute combined degeneration of spinal cord secondary to Pernicious Anaemia, Schizophrenia, Spinocerebellar ataxia (multiple types with varying characteristics), Spinal muscular atrophy, Steele-Richardson-Olszewski disease, or Tabes dorsalis.

In other embodiments, the disease is associated with abnormally high Ire1. Such diseases include, but are not limited, to cancers, inflammatory diseases, and autoimmune diseases. Exemplary cancers include, but am not limited to, breast cancer and multiple myeloma. In one embodiment, the disease is multiple myeloma. In one embodiment, the disease is a triple-negative breast cancer. Exemplary inflammatory diseases include, but are not limited to, asthma, chronic inflammation, chronic prostatitis, glomerulonephritis, hypersensitivities, inflammatory bowel diseases, pelvic inflammatory disease; reperfusion injury, rheumatoid arthritis, transplant rejection, and vasculitis. Exemplary autoimmune diseases include, but are not limited to, XBP1-linked Crohn's disease, Coeliac disease, diabetes mellitus type 1 (IDDM), systemic lupus erythematosus (SLE), Sjogren's syndrome, Churg-Strauss Syndrome, Hashimoto's thyroiditis, Graves' disease, idiopathic thrombocytopenic purpura, and rheumatoid arthritis. In one embodiment, the disease is XBP1-linked. Crohn's disease.

In one aspect provided herein is a method of treating atherosclerosis or the progression of atherosclerosis by administering an effective amount of a compound or pharmaceutically acceptable salt thereof described herein. In one embodiment, administration of a compound or pharmaceutically acceptable salt thereof described herein reduces the number of macrophages in an atherosclerotic lesion. Such reduction can be imparted, in some embodiments, without altering apoptosis state. In another embodiment, administration of a compound or pharmaceutically acceptable salt thereof as described herein inhibits or reduces the production of IL-1β, CCL2, and chemokine receptor 2.

### Pharmaceutical Formulations

Compounds or pharmaceutically acceptable salts thereof as described herein can be formulated in accordance with standard pharmaceutical practice as a pharmaceutical composition. Thus, further provided herein is a pharmaceutical composition comprising a compound or pharmaceutically acceptable salt thereof and one or more pharmaceutically acceptable excipients.

A typical formulation is prepared by mixing a compound or pharmaceutically acceptable salt thereof as described herein and an excipient. Suitable carriers, diluents and excipients include, but are not limited to, materials such as carbohydrates, waxes, water soluble and/or swellable polymers, hydrophilic or hydrophobic materials, gelatin, oils, solvents, water and the like. The particular excipient used will depend upon the means and purpose for which the compound or pharmaceutically acceptable salt thereof as described herein is being applied. Solvents are generally selected based on solvents recognized as safe (GRAS) to be administered to a mammal. In general, safe solvents are non-toxic aqueous solvents such as water and other non-toxic solvents that are soluble or miscible in water. Suitable aqueous solvents include water, ethanol, propylene glycol, polyethylene glycols (e.g., PEG 400, PEG 300), etc. and mixtures thereof. The formulations can also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents and other known additives to provide an elegant presentation of the drug (i.e., a compound described herein or pharmaceutical composition thereof) or aid in the manufacturing of the pharmaceutical product (i.e., medicament).

The formulations can be prepared using conventional dissolution and mixing procedures. For example, the bulk drug substance (i.e., compound or pharmaceutically acceptable salt thereof as described herein or stabilized form thereof (e.g., complex with a cyclodextrin derivative or other known complexation agent) is dissolved in a suitable solvent in the presence of one or more of the excipients described above. The compound or pharmaceutically acceptable salt thereof as described herein is typically formulated into pharmaceutical dosage forms to provide an easily controllable dosage of the drug and to enable patient compliance with the prescribed regimen.

The pharmaceutical composition (or formulation) for application can be packaged in a variety of ways depending upon the method used for administering the drug. Generally, an article for distribution includes a container having deposited therein the pharmaceutical formulation in an appropriate form. Suitable containers include materials such as bottles (plastic and glass), sachets, ampoules, plastic bags, metal cylinders, and the like. The container can also include a tamper-proof assemblage to prevent indiscreet access to the contents of the package. In addition, the container has deposited thereon a label that describes the contents of the container. The label can also include appropriate warnings.

Pharmaceutical formulations of the compounds or pharmaceutically acceptable salts thereof as described herein can be prepared for various routes and types of administration. For example, a compound or pharmaceutically acceptable salt thereof as described herein having the desired degree of purity can optionally be mixed with one or more pharmaceutically acceptable excipients (Remington's Pharmaceutical Sciences (1980) 16th edition, Osol, A. Ed.), in the form of a lyophilized formulation, milled powder, or an aqueous solution. Formulation can be conducted by mixing at ambient temperature at the appropriate pH, and at the desired degree of purity, with physiologically acceptable carriers, i.e., carriers that are non-toxic to recipients at the dosages and concentrations employed. The pH of the formulation depends mainly on the particular use and the concentration of compound, but can range from about 3 to about 8. For example, formulation in an acetate buffer at pH 5 can be a suitable embodiment.

The pharmaceutical composition ordinarily can be stored as a solid composition, a lyophilized formulation or as an aqueous solution.

The pharmaceutical compositions described herein can be formulated, dosed and administered in a fashion, i.e., amounts, concentrations, schedules, course, vehicles and route of administration, consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The effective amount of the compound to be administered will be governed by such considerations, and is the minimum amount necessary to ameliorate, or treat the hyperproliferative disorder.

As a general proposition, the initial pharmaceutically effective amount of the inhibitor administered parenterally per dose will be in the range of about 0.01-100 mg/kg, namely about 0.1 to 20 mg/kg of patient body weight per day, with the typical initial range of compound used being 0.3 to 15 mg/kg/day.

Acceptable pharmaceutically acceptable excipients are nontoxic to recipients at the dosages and concentrations employed, and include buffers such as phosphate, citrate and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG). The active pharmaceutical ingredients can also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations of compounds or pharmaceutically acceptable salts thereof as described herein may be prepared. Suitable examples of sustained-release preparations include semipermeable matrices of solid hydrophobic polymers containing a compound or pharmaceutically acceptable salt thereof as described herein , which matrices are in the form of shaped articles, e.g., films, or microcapsules. Examples of sustained-release matrices include polyesters, hydrogels (for example, poly(2-hydroxyethyl-methacrylate), or poly(vinyl alcohol)), polylactides (US 3773919), copolymers of L-glutamic acid and gamma-ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate) and poly-D-(-)-3-hydroxybutyric acid.

The formulations include those suitable for the administration routes detailed herein. The formulations can conveniently be presented in unit dosage form and can be prepared by any methods. Techniques and formulations generally are found in Remington's Pharmaceutical Sciences (Mack Publishing Co., Easton, PA). Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of a compound or pharmaceutically acceptable salt thereof as described herein suitable for oral administration can be prepared as discrete units such as pills, capsules, cachets or tablets each containing a predetermined amount of such compound or pharmaceutically acceptable salt thereof. Compressed tablets can be prepared by compressing in a suitable machine the active ingredient in a free-flowing form such as a powder or granules, optionally mixed with a binder, lubricant, inert diluent, preservative, surface active or dispersing agent. Molded tablets can be made by molding in a suitable machine a mixture of the powdered active ingredient moistened with an inert liquid diluent. The tablets can optionally be coated or scored and optionally are formulated so as to provide slow or controlled release of the active ingredient therefrom. Tablets, troches, lozenges, aqueous or oil suspensions, dispersible powders or granules, emulsions, hard or soft capsules, e.g., gelatin capsules, syrups or elixirs can be prepared for oral use. Formulations of compounds or pharmaceutically acceptable salts thereof as described herein intended for oral use can be prepared according to any method for the manufacture of pharmaceutical compositions and such compositions can contain one or more agents including sweetening agents, flavoring agents, coloring agents and preserving agents, in order to provide a palatable preparation. Tablets containing the active ingredient in admixture with non-toxic pharmaceutically acceptable excipient which are suitable for manufacture of tablets are acceptable. These excipients can be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents, such as magnesium stearate, stearic acid or talc. Tablets can be uncoated or can be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax can be employed.

For treatment of the eye or other external tissues, e.g., mouth and skin, the formulations are preferably applied as a topical ointment or cream containing the active ingredient(s) in an amount of, for example, 0.075 to 20% w/w. When formulated in an ointment, the active ingredients can be employed with either a paraffinic or a water-miscible ointment base. Alternatively, the active ingredients can be formulated in a cream with an oil-in-water cream base. If desired, the aqueous phase of the cream base can include a polyhydric alcohol, i.e., an alcohol having two or more hydroxyl groups such as propylene glycol, butane 1,3-diol, mannitol, sorbitol, glycerol and polyethylene glycol (including PEG 400) and mixtures thereof. The topical formulations can desirably include a compound which enhances absorption or penetration of the active ingredient through the skin or other affected areas. Examples of such dermal penetration enhancers include dimethyl sulfoxide and related analogs. The oily phase of the emulsions of compositions provided herein can be constituted from known ingredients in a known manner. While the phase can comprise merely an emulsifier, it desirably comprises a mixture of at least one emulsifier with a fat or an oil or with both a fat and an oil. Preferably, a hydrophilic emulsifier is included together with a lipophilic emulsifier which acts as a stabilizer. It is also preferred to include both an oil and a fat. Together, the emulsifier(s) with or without stabilizer(s) make up the so-called emulsifying wax, and the wax together with the oil and fat make up the so-called emulsifying ointment base which forms the oily dispersed phase of the cream formulations. Emulsifiers and emulsion stabilizers suitable for use in the formulation of described herein include Tween^{®} 60, Span^{®} 80, cetostearyl alcohol, benzyl alcohol, myristyl alcohol, glyceryl monostearate and sodium lauryl sulfate.

Aqueous suspensions comprising compounds or pharmaceutically acceptable salts thereof as described herein can contain the active materials in admixture with excipients suitable for the manufacture of aqueous suspensions. Such excipients include a suspending agent, such as sodium carboxymethylcellulose, croscarmellose, povidone, methylcellulose, hydroxypropyl methylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethyleneoxycetanol), a condensation product of ethylene oxide with a partial ester derived from a fatty acid and a hexitol anhydride (e.g., polyoxyethylene sorbitan monooleate). The aqueous suspension can also contain one or more preservatives such as ethyl or n-propyl p-hydroxybenzoate, one or more coloring agents, one or more flavoring agents and one or more sweetening agents, such as sucrose or saccharin.

The pharmaceutical compositions of compounds or pharmaceutically acceptable salts thereof as described herein can be in the form of a sterile injectable preparation, such as a sterile injectable aqueous or oleaginous suspension. This suspension can be formulated using suitable dispersing or wetting agents and suspending agents which have been mentioned above. The sterile injectable preparation can also be a sterile injectable solution or suspension in a non-toxic parenterally acceptable diluent or solvent, such as a solution in 1,3-butanediol or prepared as a lyophilized powder. Among the acceptable vehicles and solvents that can be employed are water, Ringer's solution and isotonic sodium chloride solution. In addition, sterile fixed oils can conventionally be employed as a solvent or suspending medium. For this purpose any bland fixed oil can be employed including synthetic mono- or diglycerides. In addition, fatty acids such as oleic acid can likewise be used in the preparation of injectables.

The amount of active ingredient that can be combined with the carrier material to produce a single dosage form will vary depending upon the host treated and the particular mode of administration. For example, a time-release formulation intended for oral administration to humans can contain approximately 1 to 1000 mg of active material compounded with an appropriate and convenient amount of carrier material which can vary from about 5 to about 95% of the total compositions (weight:weight). The pharmaceutical composition can be prepared to provide easily measurable amounts for administration. For example, an aqueous solution intended for intravenous infusion can contain from about 3 to 500 µg of the active ingredient per milliliter of solution in order that infusion of a suitable volume at a rate of about 30 mL/hr can occur.

Formulations suitable for parenteral administration include aqueous and non-aqueous sterile injection solutions which can contain anti-oxidants, buffers, bacteriostats and solutes which render the formulation isotonic with the blood of the intended recipient; and aqueous and non-aqueous sterile suspensions which can include suspending agents and thickening agents.

Formulations suitable for topical administration to the eye also include eye drops wherein the active ingredient is dissolved or suspended in a suitable carrier, especially an aqueous solvent for the active ingredient. The active ingredient is preferably present in such formulations in a concentration of about 0.5 to 20% w/w, for example about 0.5 to 10% w/w, for example about 1.5% w/w.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration can be presented as a suppository with a suitable base comprising for example cocoa butter or a salicylate.

Formulations suitable for intrapulmonary or nasal administration have a particle size for example in the range of 0.1 to 500 microns (including particle sizes in a range between 0.1 and 500 microns in increments microns such as 0.5, 1, 30 microns, 35 microns, etc.), which is administered by rapid inhalation through the nasal passage or by inhalation through the mouth so as to reach the alveolar sacs. Suitable formulations include aqueous or oily solutions of the active ingredient. Formulations suitable for aerosol or dry powder administration can be prepared according to conventional methods and can be delivered with other therapeutic agents such as compounds heretofore used in the treatment or prophylaxis disorders as described below.

Formulations suitable for vaginal administration can be presented as pessaries, tampons, creams, gels, pastes, foams or spray formulations containing in addition to the active ingredient such carriers considered to be appropriate.

The formulations can be packaged in unit-dose or multi-dose containers, for example sealed ampoules and vials, and can be stored in a freeze-dried (lyophilized) condition requiring only the addition of the sterile liquid carrier, for example water, for injection immediately prior to use. Extemporaneous injection solutions and suspensions are prepared from sterile powders, granules and tablets of the kind previously described. Preferred unit dosage formulations are those containing a daily dose or unit daily sub-dose, as herein above recited, or an appropriate fraction thereof, of the active ingredient.

The compounds or pharmaceutically acceptable salts thereof as described herein can be used in veterinary compositions comprising at least one active ingredient as above defined together with a veterinary carrier. Veterinary carriers are materials useful for the purpose of administering the composition and can be solid, liquid or gaseous materials which are otherwise inert or acceptable in the veterinary field and are compatible with the active ingredient. These veterinary compositions can be administered parenterally, orally or by any other desired route.

### Combination Therapy

The compounds or pharmaceutically acceptable salts thereof as described herein can be employed alone or in combination with additional therapeutic agents for the treatment of a disease or disorder described herein, such as inflammation or a hyperproliferative disorder (e.g., cancer). In certain embodiments, a compound or pharmaceutically acceptable salt thereof as described herein is combined in a pharmaceutical combination formulation, or dosing regimen as combination therapy, with an additional, second therapeutic compound that has anti-inflammatory or anti-hyperproliferative properties or that is useful for treating an inflammation, immune-response disorder, or hyperproliferative disorder (e.g., cancer). The additional therapeutic can be a Bcl-2 inhibitor, a JAK inhibitor, a PI3K inhibitor, an mTOR inhibitor, an anti-inflammatory agent, an immunomodulatory agent, anti-cancer agent as described herein, an apoptosis-enhancer, a neurotropic factor, an agent for treating cardiovascular disease, an agent for treating liver disease, an anti-viral agent, an agent for treating blood disorders, an agent for treating diabetes, and an agent for treating immunodeficiency disorders. The second therapeutic agent can be an NSAID anti-inflammatory agent. The second therapeutic agent can be a anti-cancer agent as described herein. The second compound of the pharmaceutical combination formulation or dosing regimen preferably has complementary activities to the compound or pharmaceutically acceptable salt thereof as described herein such that they do not adversely affect each other. Such compounds or pharmaceutically acceptable salts thereof are suitably present in combination in amounts that are effective for the purpose intended. In one embodiment, a composition comprises a compound or pharmaceutically acceptable salt thereof as described herein, in combination with a therapeutic agent such as an NSAID.

The combination therapy can be administered as a simultaneous or sequential regimen. When administered sequentially, the combination can be administered in two or more administrations. The combined administration includes coadministration, using separate formulations or a single pharmaceutical formulation, and consecutive administration in either order, wherein preferably there is a time period while both (or all) active agents simultaneously exert their biological activities.

Suitable dosages for any of the above coadministered agents are those presently used and can be lowered due to the combined action (synergy) of the newly identified agent and other therapeutic agents or treatments.

The combination therapy can provide "synergy" and prove "synergistic", i.e., the effect achieved when the active ingredients used together is greater than the sum of the effects that results from using the compounds separately. A synergistic effect can be attained when the active ingredients are: (1) co-formulated and administered or delivered simultaneously in a combined, unit dosage formulation; (2) delivered by alternation or in parallel as separate formulations; or (3) by some other regimen. When delivered in alternation therapy, a synergistic effect can be attained when the compounds are administered or delivered sequentially, e.g., by different injections in separate syringes, separate pills or capsules, or separate infusions. In general, during alternation therapy, an effective dosage of each active ingredient is administered sequentially, i.e., serially, whereas in combination therapy, effective dosages of two or more active ingredients are administered together.

In a particular embodiment of therapy, a compound or pharmaceutically acceptable salt thereof as described herein , can be combined with other therapeutic, hormonal or antibody agents such as those described herein, as well as combined with surgical therapy and radiotherapy. Combination therapies described herein can comprise the administration of at least one compound or pharmaceutically acceptable salt thereof as described herein, and the use of at least one other cancer treatment method. The amounts of the compound or pharmaceutically acceptable salt thereof as described herein, and the other pharmaceutically active therapeutic agent(s) and the relative timings of administration will be selected in order to achieve the desired combined therapeutic effect.

In one embodiment, a compound or pharmaceutically acceptable salt thereof as described herein, is used in combination with an aromatase inhibitor, a phosphoinositide 3-kinase (PI3K)/mTOR pathway inhibitor, a CDK 4/6 inhibitor, a HER-2 inhibitor, a SERM, a SERD, an EGFR inhibitor, a PD-1 inhibitor, poly ADP-ribose polymerase (PARP) inhibitor, a histone deacetylase (HDAC) inhibitor, an HSP90 inhibitor, a VEGFR inhibitor, an AKT inhibitor, chemotherapy, or any combination thereof.

In one embodiment, a pharmaceutical composition comprising a compound or pharmaceutically acceptable salt thereof as described herein is administered in combination with a therapeutic agent selected from paclitaxel, anastrozole, exemestane, cyclophosphamide, epirubicin, fulvestrant, letrozole, palbociclib, gemcitabine, trastuzumab, trastuzumab emtansine, pegfilgrastim, filgrastim, tamoxifen, docetaxel, toremifene, vinorelbine, capecitabine, and ixabepilone.

In one embodiment, a compound or pharmaceutically acceptable salt thereof as described herein is used in combination with hormone blocking therapy, chemotherapy, radiation therapy, monoclonal antibodies, or combinations thereof.

### Metabolites of Compounds of Formula I or Formula II

The compounds or pharmaceutically acceptable salts thereof as described herein may be capable of forming *in vivo* metabolic products. Such products can result for example from the oxidation, reduction, hydrolysis, amidation, deamidation, esterification, deesterification, enzymatic cleavage, and the like, of the administered compound. For instance, such compounds may be produced by a process comprising contacting a compound or pharmaceutically acceptable salt thereof as described herein with a mammal for a period of time sufficient to yield a metabolic product thereof.

Metabolite products typically are identified by preparing a radiolabelled (e.g., 14C or 3H) isotope of a compound or pharmaceutically acceptable salt thereof as described herein, administering it parenterally in a detectable dose (e.g., greater than about 0.5 mg/kg) to an animal such as rat, mouse, guinea pig, monkey, or to man, allowing sufficient time for metabolism to occur (typically about 30 seconds to 30 hours) and isolating its conversion products from the urine, blood or other biological samples. These products are easily isolated since they are labeled (others are isolated by the use of antibodies capable of binding epitopes surviving in the metabolite). The metabolite structures are determined in conventional fashion, e.g., by MS, LC/MS or NMR analysis. In general, analysis of metabolites is done in the same way as conventional drug metabolism studies. The metabolite products, so long as they are not otherwise found *in vivo,* are useful in diagnostic assays for therapeutic dosing of the compounds or pharmaceutically acceptable salts thereof as described herein.

### Articles of Manufacture

An article of manufacture, or kit, containing materials useful for the treatment of the diseases and disorders described above is now described. In one example, the kit comprises a container comprising a compound or pharmaceutically acceptable salt thereof as described herein. The kit can further comprise a label or package insert on or associated with the container. Suitable containers include, for example, bottles, vials, syringes, blister pack, etc. The container can be formed from a variety of materials such as glass or plastic. The container can hold a compound or pharmaceutically acceptable salt thereof as described herein, or a formulation thereof which is effective for treating the condition and can have a sterile access port (for example, the container can be an intravenous solution bag or a vial having a stopper pierceable by a hypodermic injection needle). At least one active agent in the composition is a compound or pharmaceutically acceptable salt thereof as described herein. The label or package insert indicates that the composition is used for treating the condition of choice, such as cancer. In addition, the label or package insert can indicate that the patient to be treated is one having a disorder such as atherosclerosis, a hyperproliferative disorder, neurodegeneration, cardiac hypertrophy, pain, migraine or a neurotraumatic disease or event. In one example, the label or package inserts indicates that the composition comprising a compound or pharmaceutically acceptable salt thereof as described herein can be used to treat a disorder resulting from abnormal cell growth. The label or package insert can also indicate that the composition can be used to treat other disorders. Alternatively, or additionally, the article of manufacture can further comprise a second container comprising a pharmaceutically acceptable buffer, such as bacteriostatic water for injection (BWFI), phosphate-buffered saline, Ringer's solution and dextrose solution. It can further include other materials desirable from a commercial and user standpoint, including other buffers, diluents, filters, needles, and syringes.

The kit can further comprise directions for the administration of the compound or pharmaceutically acceptable salt thereof as described herein and, if present, a second pharmaceutical formulation. For example, if the kit comprises a first composition comprising a compound or pharmaceutically acceptable salt thereof as described herein and a second pharmaceutical formulation, the kit can further comprise directions for the simultaneous, sequential or separate administration of the first and second pharmaceutical compositions to a patient in need thereof.

In another example, the kits are suitable for the delivery of solid oral forms of a compound or pharmaceutically acceptable salt thereof as described herein, such as tablets or capsules. Such a kit preferably includes a number of unit dosages. Such kits can include a card having the dosages oriented in the order of their intended use. An example of such a kit is a blister pack. Blister packs are well known in the packaging industry and are widely used for packaging pharmaceutical unit dosage forms. If desired, a memory aid can be provided, for example in the form of numbers, letters, or other markings or with a calendar insert, designating the days in the treatment schedule in which the dosages can be administered.

According to one example, a kit can comprise (a) a first container with a compound or pharmaceutically acceptable salt thereof as described herein contained therein; and optionally (b) a second container with a second pharmaceutical formulation contained therein, wherein the second pharmaceutical formulation comprises a second compound with anti-hyperproliferative activity. Alternatively, or additionally, the kit can further comprise a third container comprising a pharmaceutically-acceptable buffer, such as bacteriostatic water for injection (BWFI), phosphate-buffered saline, Ringer's solution and dextrose solution. It can further include other materials desirable from a commercial and user standpoint, including other buffers, diluents, filters, needles, and syringes.

In certain other examples wherein the kit comprises a composition of compound or pharmaceutically acceptable salt thereof as described herein and a second therapeutic agent, the kit can comprise a container for containing the separate compositions such as a divided bottle or a divided foil packet, however, the separate compositions can also be contained within a single, undivided container. Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

### EMBODIMENTS

Embodiment 1: A compound having formula (I) or formula (II); 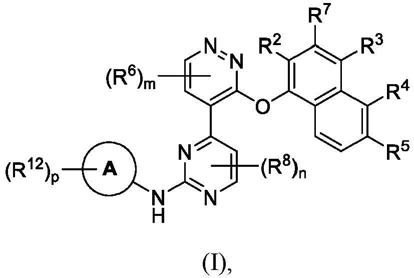 or 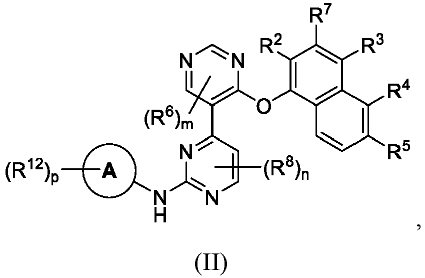 or a pharmaceutically acceptable salt thereof,
wherein;
R¹ is hydrogen, halogen, -CN, -NO₂, -NR^{a}R^{b}, -NR^{a}C(O)R^{b}, -NR^{a}C(O)R^{b}, - NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a}, - OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b}, - P(O)R^{a}R^{b}, R¹⁰-substituted or unsubstituted C₁₋₆ alkyl, R¹⁰-substituted or unsubstituted 2 to 6 membered heteroalkyl, R¹⁰-substituted or unsubstituted C₁₋₆ haloalkyl, R¹⁰-substituted or unsubstituted C₃₋C₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heterocycloalkyl;
R² is hydrogen, halogen, -CN, R¹⁰-substituted or unsubstituted C₁₋₆ alkoxy, or R¹⁰-substituted or unsubstituted C₁₋₆ alkyl;
R³ is -L¹-R¹, hydrogen, halogen, -CN, -NO₂, -NR^{a}R^{b}, -NR^{a}C(O)R^{b}, -NR^{a}C(O)R^{b}, -NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a}, - OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b}, - P(O)R^{a}R^{b}, R¹⁰-substituted or unsubstituted C₁₋₆ alkoxy, R¹⁰-substituted or unsubstituted C₁₋₆ haloalkyl, R¹⁰-substituted or unsubstituted C₁₋₆ alkyl, R¹⁰-substituted or unsubstituted C₃₋₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heteroaryl;
R⁴ is -L²-R⁹-, halogen, -CN, -NO₂, -NR^{a}R^{b}, -NR^{a}C(O)R^{b}, -NR^{a}C(O)R^{b}, - NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a}, - OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b}, - P(O)R^{a}R^{b}, R¹⁰-substituted or unsubstituted C₁₋₆ alkoxy, R¹⁰-substituted or unsubstituted C₁₋₆ haloalkyl, R¹⁰-substituted or unsubstituted C₁₋₆ alkyl, R¹⁰-substituted or unsubstituted 2 to 6 membered heteroalkyl, R¹⁰-substituted or unsubstituted C₃₋₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heteroaryl;
R⁵ is hydrogen, halogen, or R¹⁰-substituted or unsubstituted C₁₋₃ alkyl;
each R⁶ is independently hydrogen, halogen, -CN, -NO₂, -OR^{a}, -NR^{a}R^{b}, -C(O)R^{a}, - C(O)OR^{a}, -SR^{a}, -S(O)₂R^{a}, -P(O)R^{a}R^{b}, R¹⁰-substituted or unsubstituted C₁₋₆ alkoxy, R¹⁰-substituted or unsubstituted C₁₋₆ haloalkyl, R¹⁰-substituted or unsubstituted C₁₋₆ alkyl, R¹⁰-substituted or unsubstituted C₃₋₆ cycloalkyl, -SO₂(C₁₋₆ alkyl), or -SO₂(C₁₋₆ haloalkyl);
R⁷ is hydrogen, halogen, -CN, R¹⁰-substituted or unsubstituted C₁₋₆ alkyl, R¹⁰-substituted or unsubstituted C₁₋₆ alkoxy, R¹⁰-substituted or unsubstituted C₁₋₆ haloalkyl, or R¹⁰-substituted or unsubstituted C₃₋₆ cycloalkyl;
each R⁸ is independently hydrogen, halogen, or R¹⁰-substituted or unsubstituted C₁₋₃ alkyl;
R⁹ is hydrogen, halogen, R¹⁰-substituted or unsubstituted C₁₋₆ alkyl, R¹⁰-substituted or unsubstituted 2 to 6 membered heteroalkyl, R¹⁰-substituted or unsubstituted C₁₋₆ haloalkyl, R¹⁰-substituted or unsubstituted C₃₋C₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heterocycloalkyl;
each R¹⁰ is independently halogen, -N₃, -CF₃, -CN, -NO₂, -NR^{a}R^{b}, -NR^{a}C(O)R^{b}, -NR^{a}C(O)R^{b}, -NR^{a}C(O)OR^{b}, -NR^{a}C(O)NR^{b}, -S(O)₂NR^{a}, -C(O)R^{a}, -C(O)OR^{a}, -C(O)NR^{a}R^{b}, - OR^{a}, -OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, - S(O)₂NR^{a}R^{b}, -P(O)R^{a}R^{b}, R¹¹-substituted or unsubstituted C₁₋₆ alkyl, R¹¹-substituted or unsubstituted 2 to 6 membered heteroalkyl, R¹¹-substituted or unsubstituted C₃₋₇ cycloalkyl, R¹¹-substituted or unsubstituted 3 to 7 membered heterocycloalkyl, R¹¹-substituted or unsubstituted C₅₋₆ aryl, or R¹¹-substituted or unsubstituted 5 to 6 membered heteroaryl;
each R¹¹ is independently halogen, -N₃, -CF₃, -CCl₃, -CBr₃, -CI₃, -CN, -CHO, -OH, -NH₂, -COOH, -CONH₂, -NO₂, -SH, -SO₂Cl, -SO₃H, -SO₄H, -SO₂NH₂, unsubstituted C₁₋₆ alkyl, unsubstituted 2 to 6 membered heteroalkyl, unsubstituted C₃₋₇ cycloalkyl, unsubstituted 3 to 7 membered heterocycloalkyl, unsubstituted C₅₋₆ aryl, or unsubstituted 5 to 6 membered heteroaryl;
each R¹² is independently hydrogen, halogen, a NPG, or R¹⁰-substituted or unsubstituted C₁₋₃ alkyl;
L¹ is -NHSO₂-, -NHC(O)-, NHCO(O)-, -NHC(O)NH-, -NH-, -O-, -S-, or -SO₂-;
L² is -NHSO₂-, -NHC(O)-, NHCO(O)-, -NHC(O)NH-, -NH-, -O-, -S-, or -SO₂-;
each R^{a} and R^{b} is independently hydrogen, unsubstituted C₁₋₆ alkyl, unsubstituted C₁₋₆ haloalkyl, unsubstituted C₁₋₆ alkoxy, unsubstituted C₂₋₆ alkenyl, unsubstituted C₂₋₆ alkynyl, unsubstituted C₃₋₇ cycloalkyl, unsubstituted 3 to 7 membered heterocycloalkyl, unsubstituted C₅₋₆ aryl, or unsubstituted 5 or 6 membered heteroaryl;
m is 1 or 2;
n is 1 or 2;
p is 1, 2, 3, 4, 5 or 6; and
Ring A is C₃₋₆ cycloalkyl, 3 to 6 membered heterocycloalkyl, C₅₋₆ aryl, or 5 or 6 membered heteroaryl.

Embodiment 2: The compound of embodiment 1, wherein the compound or a pharmaceutically acceptable salt thereof comprises formula (I).

Embodiment 3: The compound of embodiment 1, wherein the compound or a pharmaceutically acceptable salt thereof comprises formula (II).

Embodiment 4: The compound of any one of embodiments 1 to 3, wherein ring A is 6 membered heterocycloalkyl.

Embodiment 5: The compound of any one of embodiments 1 to 4, wherein ring A is piperidinyl.

Embodiment 6: The compound of embodiment 4 or embodiment 5, wherein R¹² is halogen or C₁₋₃ alkyl.

Embodiment 7: The compound of embodiment 6, wherein n is p is 1 or 2.

Embodiment 8: The compound of embodiment 6 or embodiment 7, wherein R¹² is -F.

Embodiment 9: The compound of embodiment 6 or embodiment 7, wherein p is 2 and R¹² is -F and methyl.

Embodiment 10: The compound of any one of embodiments 1 to 9, wherein ring A has the structure:

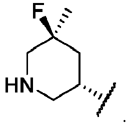

Embodiment 11: The compound of any one of embodiments 1 to 10, wherein R⁶ is hydrogen.

Embodiment 12: The compound of any one of embodiments 1 to 11, wherein R⁸ is hydrogen.

Embodiment 13: The compound of any one of embodiments 1 to 12, wherein R² is unsubstituted C₁₋₃ alkyl.

Embodiment 14: The compound of any one of embodiments 1 to 13, wherein R² is methyl.

Embodiment 15: The compound of any one of embodiments 1 to 14, wherein R³ is hydrogen.

Embodiment 16: The compound of any one of embodiments 1 to 15, wherein R⁴ is -L²-R⁹-.

Embodiment 17: The compound of embodiment 16, wherein L² is -NHSO₂-.

Embodiment 18: The compound of embodiment 16, wherein L² is -NHC(O)-.

Embodiment 19: The compound of any one of embodiments 16 to 18, wherein R⁹ is R¹⁰-substituted or unsubstituted phenyl.

Embodiment 20: The compound of embodiment 19, wherein R⁹ is R¹⁰-substituted phenyl and R¹⁰ is halogen.

Embodiment 21: The compound of any one of embodiments 16 to 18, wherein R⁹ is R¹⁰-substituted or unsubstituted C₃₋₆ cycloalkyl.

Embodiment 22: The compound of embodiment 21, wherein R⁹ is unsubstituted cyclopropanyl.

Embodiment 23: The compound of any one of embodiments 16 to 18, wherein R⁹ is 2 to 6 membered unsubstituted haloalkyl.

Embodiment 24: The compound of embodiment 23, wherein the haloalkyl is 3,3 difluorobutyl.

Embodiment 25: The compound of any one of embodiments 16 to 18, wherein R⁹ is R¹⁰-substituted or unsubstituted C₁₋₃ alkyl.

Embodiment 26: The compound of embodiment 25, wherein R⁹ is unsubstituted C₁₋₃ alkyl.

Embodiment 27: The compound of any one of embodiments 1 to 27, wherein R⁵ is halogen.

Embodiment 28: The compound of any one of embodiments 1 to 27, wherein R⁵ is hydrogen.

Embodiment 29: The compound of any one of embodiments 1 to 27, wherein R⁵ is unsubstituted C₁₋₃ alkyl.

Embodiment 30: The compound of embodiment 1 having formula: 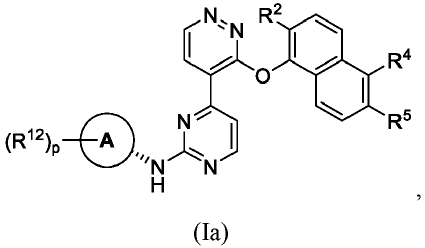 or a pharmaceutically acceptable salt thereof.

Embodiment 31: The compound of embodiment 1 having formula: 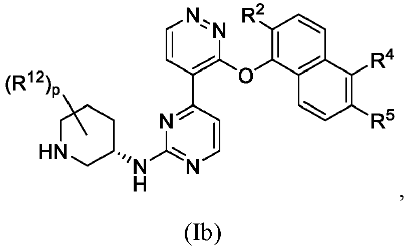 or a pharmaceutically acceptable salt thereof.

Embodiment 32: The compound of embodiment 1 having formula: 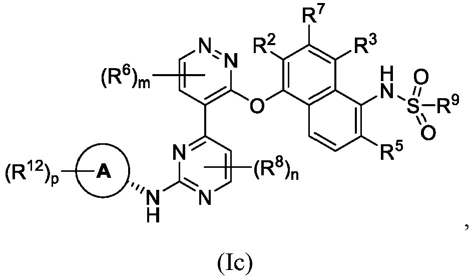 or a pharmaceutically acceptable salt thereof.

Embodiment 33: The compound of embodiment 1 having formula: 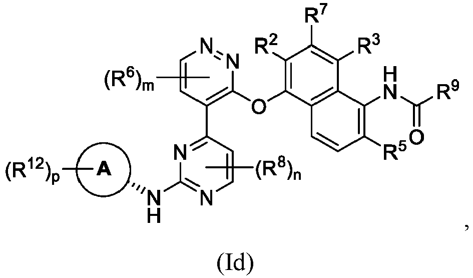 or a pharmaceutically acceptable salt thereof.

Embodiment 34: The compound of embodiment 1 having formula: 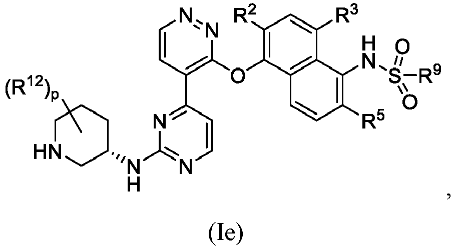 or a pharmaceutically acceptable salt thereof.

Embodiment 35: The compound of embodiment 1 having formula: 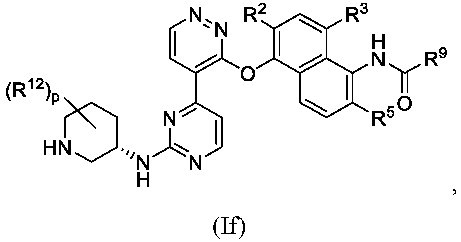 or a pharmaceutically acceptable salt thereof.

Embodiment 36: The compound of embodiment 1 having formula: 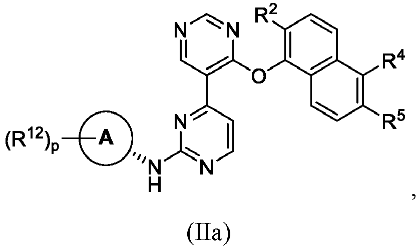 or a pharmaceutically acceptable salt thereof.

Embodiment 37: The compound of embodiment 1 having formula: 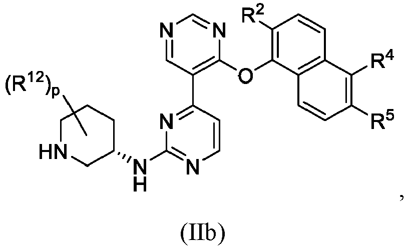 or a pharmaceutically acceptable salt thereof.

Embodiment 38: The compound of embodiment 1 having formula: 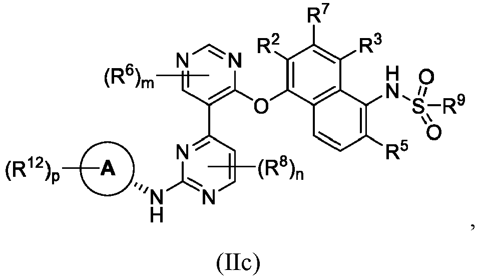 or a pharmaceutically acceptable salt thereof.

Embodiment 39: The compound of embodiment 1 having formula: 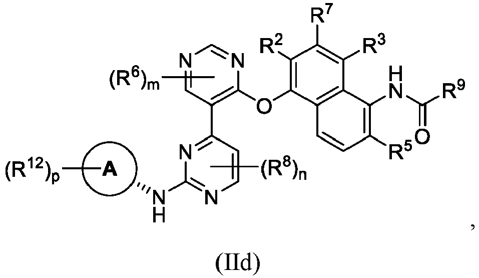 or a pharmaceutically acceptable salt thereof.

Embodiment 40: The compound of embodiment 1 having formula: 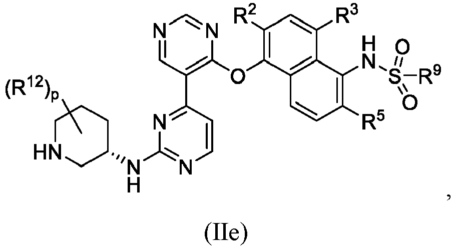 or a pharmaceutically acceptable salt thereof.

Embodiment 41: The compound of embodiment 1 having formula: 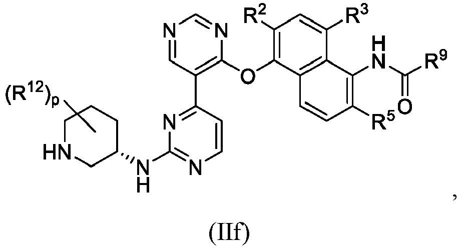 or a pharmaceutically acceptable salt thereof.

Embodiment 42: The compound or pharmaceutically acceptable salt thereof of embodiment 1, wherein the compound comprises a compound of Table 1.

Embodiment 43: A pharmaceutical composition comprising a compound or pharmaceutically acceptable salt thereof of any one of embodiments 1 to 42 and one or more pharmaceutically acceptable excipients.

Embodiment 44: A compound or pharmaceutically acceptable salt thereof of any one of embodiments 1 to 42 or a pharmaceutical composition of embodiment 43 for use in a method of treating cancer.

Embodiment 45: The compound, pharmaceutically acceptable salt, or pharmaceutical composition for use of embodiment 44, wherein the cancer is squamous cell carcinoma, small-cell lung cancer, non-small cell lung cancer (NSCLC), lung adenocarcinoma, squamous cell lung cancer, peritoneum cancer, hepatocellular cancer, stomach cancer, gastrointestinal cancer, esophageal cancer, pancreatic cancer, glioblastoma, cervical cancer, ovarian cancer, liver cancer, bladder cancer, breast cancer, colon cancer, rectal cancer, colorectal cancer, endometrial cancer, uterine cancer, salivary gland carcinoma, renal cancer, prostate cancer, vulval cancer, thyroid cancer, hepatocellular carcinoma (HCC), anal carcinoma, penile carcinoma, or head and neck cancer.

Embodiment 46: The compound, pharmaceutically acceptable salt, or pharmaceutical composition for use of embodiment 44, wherein the cancer is lymphoma, lymphocytic leukemia, multiple myeloma (MM), acute myelogenous leukemia (AML), chronic myelogenous leukemia (CML), myelodysplastic syndrome (MDS), or myeloproliferative disease (MPD).

Embodiment 47: The compound, pharmaceutically acceptable salt, or pharmaceutical composition for use of embodiment 44, wherein the cancer is multiple myeloma.

Embodiment 48: The compound, pharmaceutically acceptable salt, or pharmaceutical composition for use of embodiment 44, wherein the cancer is breast cancer.

Embodiment 49: The compound, pharmaceutically acceptable salt, or pharmaceutical composition for use of embodiment 48, wherein the breast cancer is triple-negative breast cancer (TNBC).

Embodiment 50: The compound, pharmaceutically acceptable salt, or pharmaceutical composition of any one of embodiments 44 to 49, wherein the cancer is an Ire1-mediated cancer.

Embodiment 51: The compound, pharmaceutically acceptable salt, or pharmaceutical composition for use of embodiment 50, wherein the cancer is characterized by an increased expression of Ire1.

Embodiment 52: The compound, pharmaceutically acceptable salt, or pharmaceutical composition for use of any one of embodiments 44 to 51, the method further comprising administering one or more additional anti-cancer therapies in combination with the compound or pharmaceutically acceptable salt thereof or pharmaceutical composition.

Embodiment 53: The compound, pharmaceutically acceptable salt, or pharmaceutical composition for use of embodiment 52, wherein the anti-cancer therapy is administered before said compound or pharmaceutical composition.

Embodiment 54: The compound, pharmaceutically acceptable salt, or pharmaceutical composition for use of embodiment 52, wherein the anti-cancer therapy is administered after said compound or pharmaceutical composition.

Embodiment 55: The compound, pharmaceutically acceptable salt, or pharmaceutical composition for use of embodiment 52, wherein the anti-cancer therapy is administered concurrently with said compound or pharmaceutical composition.

Embodiment 56: The compound, pharmaceutically acceptable salt, or pharmaceutical composition for use of any one of embodiments 52 to 55, wherein the anti-cancer therapy comprises a corticosteroid, a proteasome inhibitor, an immunomodulatory agent, an anti-CD38 antibody, an anti-VEGF-A antibody, an anti-PD-1 antibody, an anti-PD-L1 antibody, or an anti-interleukin-6 antibody, or a combination thereof.

Embodiment 57: The compound, pharmaceutically acceptable salt, or pharmaceutical composition for use of embodiment 56, wherein the corticosteroid comprises dexamethasone.

Embodiment 58: The compound, pharmaceutically acceptable salt, or pharmaceutical composition for use of embodiment 56, wherein the proteasome inhibitor comprises carfilzomib, ixazomib or bortezomib.

Embodiment 59: The compound, pharmaceutically acceptable salt, or pharmaceutical composition for use of embodiment 56, wherein the immunomodulatory agent comprises lenalidomide or pomalidomide.

Embodiment 60: The compound, pharmaceutically acceptable salt, or pharmaceutical composition for use of embodiment 56, wherein the anti-PD-L1 antibody comprises, avelumab, durvalumab, or atezolizumab.

Embodiment 61: The compound, pharmaceutically acceptable salt, or pharmaceutical composition for use of embodiment 56, wherein the anti-PD-1 antibody comprises pembrolizumab or nivolumab.

Embodiment 62: The compound, pharmaceutically acceptable salt, or pharmaceutical composition for use of any one of embodiments 44 to 62, wherein the treatment further comprises administering radiotherapy.

Embodiment 63: An in vitro method of inhibiting or killing a cancer cell expressing Ire1, the method comprising contacting the cancer cell expressing Ire1 with a compound or pharmaceutically acceptable salt thereof, of any one of embodiments 1 to 42 or a pharmaceutical composition of embodiment 43.

Embodiment 64: The compound or pharmaceutically acceptable salt thereof of any one of embodiments 1 to 42 or a pharmaceutical composition of embodiment 43 for use in a method of inhibiting or killing a cancer cell expressing Ire1, the method comprising contacting the cancer cell expressing Ire1 with the compound, pharmaceutically acceptable salt, or pharmaceutical composition, wherein the inhibiting or killing is performed in vivo.

Embodiment 65A: The method of embodiment 63, wherein the cancer cell expressing Ire1 is in a human.
Embodiment 65B: The compound, pharmaceutically acceptable salt, or pharmaceutical composition for use of embodiment 64, wherein the cancer cell expressing Ire1 is in a human.

Embodiment 66: An vitro method of modulating Ire1 activity, the method comprising contacting Ire1 with a compound or pharmaceutically acceptable salt thereof of any one of embodiments 1 to 42 or a pharmaceutical composition of embodiment 43.

Embodiment 67: The method of embodiment 66, wherein the Ire1 is in a cancer cell.

Embodiment 68: The compound or pharmaceutically acceptable salt thereof of any one of embodiments 1 to 42 or a pharmaceutical composition of embodiment 43 for use in a method of medical treatment comprising modulating Ire1 activity, the method comprising contacting Ire1 with the compound, pharmaceutically acceptable salt, or pharmaceutical composition.

Embodiment 69: The compound, pharmaceutically acceptable salt, or pharmaceutical composition for use of embodiment 68, wherein the Ire1 is in a cancer cell.

Embodiment 70: The method of embodiment 69, wherein the cancer cell is in a human.

Embodiment 71: A kit for treating cancer, comprising:
a) a pharmaceutical composition of embodiment 43; and
b) instructions for use.

The following examples are offered by way of illustration and not by way of limitation.

### EXAMPLES

### Synthetic Examples

Exemplary Formula (I) or Formula (II) compounds in Table 1 were made, characterized, and tested for binding to IRE1α (alpha). Where more than one name is associated with a Formula (I) or Formula (II) compound or intermediate, the chemical structure shall define the compound.

### Abbreviations:

ACN: acetonitrile
DCM: dichloromethane
DMF: N,N-dimethylformamide
DMSO: dimethyl sulfoxide
EtOAc: ethyl acetate
EtOH: ethanol
h: hour
HCl: hydrochloric acid
HPLC: High-performance liquid chromatography
IPA: isopropyl alcohol
LCMS: Liquid chromatography-mass spectrometry
MeCN: acetonitrile
THF: tetrahydrofuran

### Example 1

### (S)-2-Chloro-N-(6-methyl-5-((4-(2-(piperidin-3-ylamino)pyrimidin-4-yl)pyridazin-3-yl)oxy)naphthalen-1-yl)benzenesulfonamide hydrochloride

### Step 1: 3-Methoxy-4-(tributylstannyl)pyridazine

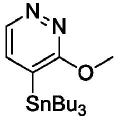

To a dry flask under nitrogen was charged diisopropylamine (3.83 mL, 27.2 mmol), and anhydrous THF (120 mL) and the mixture was cooled at -78 °C. Butyllithium (2.5 M in hexane, 10.9 mL, 27.2 mmol) was added and the mixture was warmed to 0 °C and left to stir for 0.5 h. This mixture was then cooled to -78 °C and a solution of 3-methoxypyridazine (2.0 g, 18.2 mmol) in anhydrous THF (36 mL) was added slowly over 10 min. The reaction was stirred for a further 0.5 h. Tributyltin chloride (7.88 mL, 9.46 g, 29.1 mmol) was then added at -78 °C and the mixture was stirred in the cooling bath for 1.5 h. A solution of saturated aqueous ammonium chloride was then added and the mixture was warmed to room temperature. The phases were separated and the organic phase was washed with saturated aqueous ammonium chloride and brine. The organic phase was dried over anhydrous magnesium sulfate, filtered and concentrated *in vacuo.* The crude was purified by silica flash chromatography through (0-25% EtOAc/hexanes) to provide 3.07 g (42% yield) of the title compound as a colorless oil. LCMS (ESI) [M+H]⁺= 401.1; ¹H NMR (500 MHz, CDCl₃) δ 8.74 - 8.66 (m, 1H), 7.51 - 7.36 (m, 1H), 4.10 (s, 3H), 1.59 - 1.43 (m, 6H), 1.37 - 1.28 (m, 6H), 1.20 - 1.05 (m, 6H), 0.89 (t, *J* = 7.3 Hz, 9H).

### Step 2: 4-(2-Chloropyrimidin-4-yl)-3-methoxypyridazine

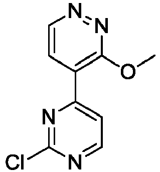

To a flask containing 3-methoxy-4-(tributylstannyl)pyridazine (2.34 g, 5.87 mmol) was added 2,4-dichloropyrimidine (875 mg, 5.87 mmol), copper(I) iodide (112 mg, 0.59 mmol), tetrakis(triphenylphosphine)palladium(0) (689 mg, 0.59 mmol) and DMF (24 mL) in that order. The solution was sparged with N₂ for 15 minutes then placed in a 100 °C oil bath. After 16 hours the mixture was cooled to room temperature and purified directly by C18 reverse phase flash chromatography (0-50% MeCN/10 mM aqueous ammonium formate, pH = 3.8) to provide 850 mg (65% yield) of the title compound as a red solid. LCMS (ESI) [M+H]⁺= 222.9; ¹H NMR (500 MHz, CDCl₃) δ 9.12 (s, 1H), 8.77 (d, *J* = 5.2 Hz, 1H), 8.35 (s, 1H), 8.18 (d, *J* = 5.2 Hz, 1H), 4.33 (s, 3H).

### Step 3: (S)-tert-Butyl 3-((4-(3-methoxypyridazin-4-yl)pyrimidin-2-yl)amino)piperidine-1-carboxylate

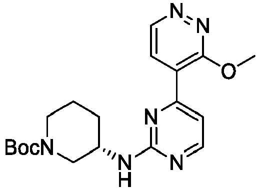

To a flask containing 4-(2-chloropyrimidin-4-yl)-3-methoxypyridazine (500 mg, 2.25 mmol) was added (*S*)-*tert*-butyl 3-aminopiperidine-1-carboxylate (900 mg, 4.49 mmol), triethylamine (1.24 mL, 8.98 mmol) and DMSO (4.5 mL) in that order. Nitrogen was bubbled through the solution for 15 min then the mixture was placed in a 100 °C oil bath. After 4 h, the mixture was cooled to room temperature, formic acid (0.7 ml) was added and the mixture was directly purified by C18 reverse phase chromatography (10-50% MeCN/10 mM aqueous ammonium formate, pH = 3.8) to provide 766 mg (88% yield) of the title compound as a yellow solid. LCMS (ESI) [M+H]⁺= 387.2; ¹H NMR (500 MHz, CDCl₃) δ 9.02 (d, *J* = 4.7 Hz, 1H), 8.40 (d, *J* = 4.7 Hz, 1H), 8.14 (br s, 1H), 7.42 (s, 1H), 5.38 (s, 1H), 4.28 (s, 3H), 4.06 - 3.96 (m, 1H), 3.89 - 2.97 (m, 4H), 2.05 - 1.96 (m, 1H), 1.82 - 1.73 (m, 1H), 1.68 - 1.57 (m, 2H), 1.50 - 1.34 (m, 9H).

### Step 4: (S)-4-(2-(Piperidin-3-ylamino)pyrimidin-4-yl)pyridazin-3-ol hydrobromide

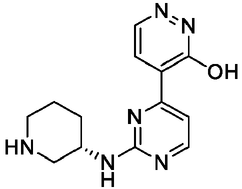

To a flask containing (*S*)-*tert*-butyl 3-((4-(3-methoxypyridazin-4-yl)pyrimidin-2-yl)amino)piperidine-1-carboxylate (950 mg, 2.46 mmol) was added a solution of 33% HBr in acetic acid (10 ml) and the mixture was heated to 100 °C. After 30 min, the reaction mixture was cooled to room temperature and concentrated *in vacuo.* 1,4-Dioxane was added and the mixture was concentrated *in vacuo* (repeated x 2), followed by addition of methanol and concentrated under reduced pressure (repeated x 2) to provide 868 mg (100% crude yield) of the title compound as a pale yellow solid. LCMS (ESI) [M+H]⁺= 273.0; ¹H NMR (500 MHz, CD₃OH) δ 8.64 (s, 1H), 8.56 (d, *J* = 4.7 Hz, 1H), 8.32 - 8.20 (br s, 1H), 8.21 (d, *J* = 4.3 Hz, 1H), 4.63 (s, 1H), 3.67 (dd, *J* = 11.9, 3.1 Hz, 1H), 3.42 (d, *J* = 12.6 Hz, 1H), 3.12 - 3.03 (m, 2H), 2.28 - 2.19 (m, 1H), 2.17 - 2.09 (m, 1H), 2.09 - 1.96 (m, 1H), 1.92 - 1.83 (m, 1H).

### Step 5: (S)-tert-Butyl 3-((4-(3-chloropyridazin-4-yl)pyrimidin-2-yl)amino)piperidine-1-carboxylate

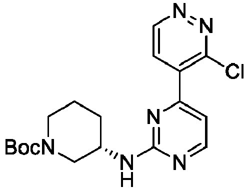

To a flask containing crude (*S*)-4-(2-(piperidin-3-ylamino)pyrimidin-4-yl)pyridazin-3-ol hydrobromide (868 mg, 2.46 mmol) was added phosphorus oxide chloride (11.5 mL, 0.12 mol) and the mixture was concentrated *in vacuo.* A further portion of phosphorus oxide chloride (11.5 mL, 0.12 mmol) was added and the reaction was heated to 80 °C. After 24 h, phosphorus oxide chloride was removed by concentration under reduced pressure and 1,4-dioxane was then added and the mixture was again concentrated *in vacuo.* To the crude material thus obtained was added THF (2.5 mL), saturated aqueous sodium carbonate (5 mL) followed by addition of a solution of di-*tert*-butyldicarbonate (1.07 g, 4.92 mmol) in THF (2.5 mL) and the mixture was stirred at rt. After 1 h, the mixture was diluted with ethyl acetate (50 mL) and the phases were separated. The organic phase was dried over anydrous magnesium sulfate, filtered and concentrated under reduced pressure. The crude material was purified by silica flash column chromatography (0-100% acetone/CH₂Cl₂) to provide 744 mg (77% yield) of the title compound as a pale pink solid. LCMS (ESI) [M-tBu+H]⁺ = 335.0, [M-Boc+H]⁺= 291.0; ¹H NMR (500 MHz, CDCl₃) δ 9.28 (d, *J* = 5.0 Hz, 1H), 8.47 (d, *J* = 4.8 Hz, 1H), 7.81 (br s, 1H), 7.11 (s, 1H), 5.47 (s, 1H), 4.03 (s, 1H), 3.93 - 2.94 (br m, 4H), 2.05 - 1.94 (m, 1H), 1.84 - 1.73 (m, 1H), 1.73-1.66 (m, 1H), 1.66 - 1.57 (m, 1H), 1.42 (s, 9H).

### Step 6: (S)-tert-Butyl 3-((4-(3-((5-amino-2-methylnaphthalen-1-yl)oxy)pyridazin-4-yl)pyrimidin-2-yl)amino)piperidine-1-carboxylate

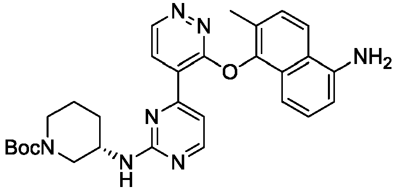

(*S*)-*tert*-Butyl 3-((4-(3-chloropyridazin-4-yl)pyrimidin-2-yl)amino)piperidine-1-carboxylate (200 mg, 0.510 mmol), 5-amino-2-methylnaphthalen-1-ol hydrochloride (129 mg, 0.610 mmol) and cesium carbonate (503 mg, 1.54 mmol) and *N*-methyl-pyrrolidinone (1.5 mL) were combined in a flask and the mixture was flushed with nitrogen for 10 min. The reaction mixture was then heated to 120 °C. After 1.5 hour, another portion of 5-amino-2-methylnaphthalen-1-ol hydrochloride (128 mg, 0.61 mmol), *N*-methyl-pyrrolidinone (1.5 mL) and cesium carbonate (503 mg, 1.54 mmol) were added. After 2 h at 120 °C the reaction mixture was cooled to room temperature and was directly purified by C18 reverse phase chromatography (10-70% MeCN/10 mM aqueous ammonium formate, pH = 3.8) to provide 149 mg (55% yield) of the title compound as a pale brown solid. LCMS (ESI) [M+H]⁺ = 528.2; ¹H NMR (500 MHz, CDCl₃) δ 9.07 (d, *J* = 4.8 Hz, 1H), 8.82-8.25 (br s, 1H), 8.44 (s, 1H), 7.81 (s, 1H), 7.70 (d, *J* = 8.6 Hz, 1H), 7.38 (d, *J* = 8.7 Hz, 1H), 7.23 - 7.10 (m, 2H), 6.72 (dd, *J* = 7.0, 1.0 Hz, 1H), 5.59 (br s, 1H), 4.52-3.97 (m, 1H), 4.09 (s, 1H), 3.96 - 2.88 (m, 5H), 2.31 (s, 3H), 2.08 - 2.04 (m, 1H), 1.85 - 1.78 (m, 1H), 1.72 (dd, *J* = 18.5, 9.5 Hz, 1H), 1.68 - 1.61 (m, 1H), 1.44 (s, 9H).

### Step 7: (S)-tert-Butyl 3-((4-(3-((5-(2-chlorophenylsulfonamido)-2-methylnaphthalen-1-yl)oxy)pyridazin-4-yl)pyrimidin-2-yl)amino)piperidine-1-carboxylate

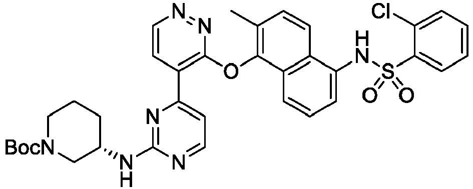

To a solution of (*S*)-*tert*-butyl 3-((4-(3-((5-amino-2-methylnaphthalen-1-yl)oxy)pyridazin-4-yl)pyrimidin-2-yl)amino)piperidine-1-carboxylate (50 mg, 0.090 mmol) in pyridine (0.50 mL) was added a solution of 2-chlorobenzenesulfonyl chloride (24 mg, 0.11 mmol) in pyridine (0.50 mL). The reaction mixture was stirred at rt. After 2 h, the mixture was directly purified by C18 reverse phase chromatography (30-80% acetonitrile/10 mM aqueous ammonium formate, pH = 3.8) to provide 53 mg (80% yield) of the title compound as a tan solid. LCMS (ESI) [M+H]+ = 702.1; ¹H NMR (500 MHz, CDCl₃) δ 9.07 (s, 1H), 8.81-8.16 (m, 2H), 8.04 (d, *J* = 8.7 Hz, 1H), 7.94 (dd, *J* = 7.9, 1.6 Hz, 1H), 7.71 (s, 1H), 7.60 - 7.51 (m, 3H), 7.51 - 7.43 (m, 2H), 7.30 (td, *J* = 7.8, 1.2 Hz, 1H), 7.24 (d, *J* = 7.0 Hz, 1H), 7.18 (t, *J* = 7.9 Hz, 1H), 6.08 - 5.21 (m, 1H), 4.07 (s, 1H), 3.94 - 2.83 (m, 4H), 2.28 (s, 3H), 2.06 (s, 1H), 1.88 - 1.77 (m, 1H), 1.77 - 1.59 (m, 2H), 1.44 (s, 9H).

### Step 8: (S)-2-Chloro-N-(6-methyl-5-((4-(2-(piperidin-3-ylamino)pyrimidin-4-yl)pyridazin-3-yl)oxy)naphthalen-1-yl)benzenesulfonamide hydrochloride

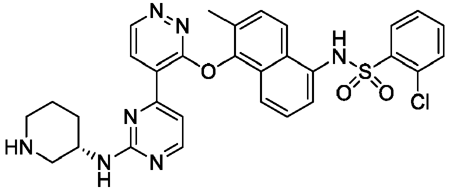

To a solution of (*S*)-*tert*-butyl 3-((4-(3-((5-(2-chlorophenylsulfonamido)-2-methylnaphthalen-1-yl)oxy)pyridazin-4-yl)pyrimidin-2-yl)amino)piperidine-1-carboxylate (53 mg, 0.080 mmol) in 1,4-dioxane (1 mL) was added 4M HCl in 1,4-dioxane (0.5 mL, 2 mmol) and the mixture stirred at rt. After 1 h, the resulting solids were filtered off and washed with 1,4-dioxane, then with heptane, then 1,4-dioxane again. The solids were dissolved in water and lyophilized to provide 36 mg (75% yield) of the title compound as a yellow solid. LCMS (ESI) [M+H]+ = 602.1; ¹H NMR (500 MHz, *d₆*-DMSO) δ 10.68 (s, 1H), 9.38 (br s, 1H), 9.15 (d, *J* = 4.7 Hz, 2H), 8.62 (br s, 1H), 8.55 (d, *J* = 5.1 Hz, 1H), 8.11 (d, *J* = 8.7 Hz, 1H), 7.86 (dd, *J* = 7.9, 1.2 Hz, 1H), 7.69 (d, *J* = 7.4 Hz, 2H), 7.65 - 7.54 (m, 3H), 7.51 (d, *J* = 8.8 Hz, 1H), 7.43 (t, *J* = 7.4 Hz, 1H), 7.33 (t, *J* = 8.0 Hz, 1H), 7.19 (d, *J* = 7.4 Hz, 1H), 4.35 (s, 1H), 3.42 (s, 1H), 3.20 (d, *J* = 10.5 Hz, 1H), 2.92 - 2.74 (m, 2H), 2.20 (s, 3H), 2.00 (d, *J* = 9.4 Hz, 1H), 1.90 (d, *J* = 14.3 Hz, 1H), 1.77 (q, *J* = 9.4 Hz, 1H), 1.63 (dd, *J* = 19.4, 9.3 Hz, 1H).

### Example 2

### (S)-N-(6-methyl-5-((4-(2-(piperidin-3-ylamino)pyrimidin-4-yl)pyridazin-3-yl)oxy)naphthalen-1-yl)cyclopropanecarboxamide hydrochloride

### Step 1: (S)-tert-Butyl 3-((4-(3-((5-(cyclopropanecarboxamido)-2-methylnaphthalen-1-yl)oxy)pyridazin-4-yl)pyrimidin-2-yl)amino)piperidine-1-carboxylate

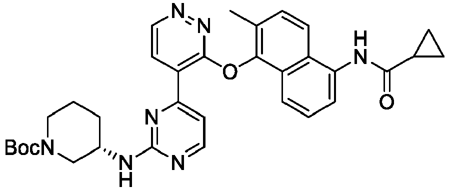

To a solution of (*S*)-*tert*-butyl 3-((4-(3-((5-amino-2-methylnaphthalen-1-yl)oxy)pyridazin-4-yl)pyrimidin-2-yl)amino)piperidine-1-carboxylate (30 mg, 0.06 mmol) in pyridine (0.50 mL) was added a solution of cyclopropanecarbonyl chloride (7.1 mg, 0.07 mmol) in pyridine (0.50 mL) and the reaction mixture was stirred at rt. After 2 h, the mixture was concentrated *in vacuo* to remove pyridine and the crude material was partitioned between EtOAc (40 mL) and saturated aqueous NaCl (10 mL). The phases were separated and the organic phase was dried over anhydrous magnesium sulfate, filtered and concentrated *in vacuo.* The crude material obtained was purified by silica flash column chromatography (0 - 100% EtOAc/CH₂Cl₂) to provide 18 mg (53% yield) of the title compound as an off-white solid. LCMS (ESI) [M+H]+ = 596.3; ¹H NMR (500 MHz, CDCl₃) δ 9.05 (s, 1H), 8.96 - 8.20 (m, 2H), 8.04 (s, 1H), 7.93 - 7.62 (m, 3H), 7.50 (s, 1H), 7.41 (s, 1H), 7.32 (s, 1H), 6.02 (br s, 1H), 4.06 (s, 1H), 3.93 - 2.84 (m, 4H), 2.29 (s, 3H), 2.06 (s, 1H), 1.80 (s, 1H), 1.75 - 1.59 (m, 3H), 1.54 (br s, 9H), 1.18 - 1.09 (m, 2H), 1.09 - 0.99 (m, 1H), 0.93 - 0.87 (m, 1H).

### Step 2: (S)-N-(6-methyl-5-((4-(2-(piperidin-3-ylamino)pyrimidin-4-yl)pyridazin-3-yl)oxy)naphthalen-1-yl)cyclopropanecarboxamide hydrochloride

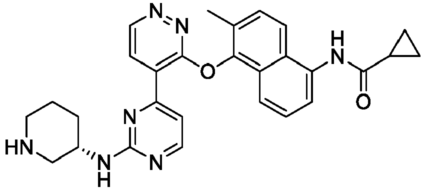

To a solution of (*S*)-*tert*-butyl 3-((4-(3-((5-(cyclopropanecarboxamido)-2-methylnaphthalen-1-yl)oxy)pyridazin-4-yl)pyrimidin-2-yl)amino)piperidine-1-carboxylate (18 mg, 0.03 mmol) in 1,4-dioxane (1 mL) was added 4M HCl in 1,4-dioxane (0.2 mL, 0.8 mmol) and the mixture stirred at rt. After 1 h, the resulting solids were filtered off and washed with 1,4-dioxane, then with heptane, then 1,4-dioxane again. The solids were dissolved in water and lyophilized to provide 11 mg (68% yield) of the title compound as an off-white solid. LCMS (ESI) [M+H]+ = 496.2; ¹H NMR (500 MHz, *d₆*-DMSO) δ 10.26 (s, 1H), 9.32-9.01 (m, 1H), 9.17 (d, *J* = 4.9 Hz, 1H), 9.00-8.77 (m, 1H), 8.63-8.14 (m, 1H), 8.58 (d, *J*= 5.1 Hz, 1H), 8.05 (d, *J* = 8.7 Hz, 1H), 7.72 (d, *J* = 7.4 Hz, 1H), 7.69 (d, *J* = 7.5 Hz, 1H), 7.62 (s, 1H), 7.57 (d, *J* = 8.8 Hz, 1H), 7.50 (d, *J* = 8.5 Hz, 1H), 7.45 - 7.38 (m, 1H), 4.30 (s, 1H), 3.43 (m, 1H), 3.21 (d, *J* = 12.1 Hz, 1H), 2.92 - 2.75 (m, 2H), 2.24 (s, 3H), 2.11 (s, 1H), 2.01 (dd, *J* = 8.6, 4.0 Hz, 1H), 1.91 (dd, *J* = 10.3, 4.2 Hz, 1H), 1.76 (dd, *J* = 21.4, 10.4 Hz, 1H), 1.63 (dd, *J* = 21.9, 11.5 Hz, 1H), 0.86 (d, *J* = 4.8 Hz, 4H).

### Example 3

### (S)-N-(6-Methyl-5-((4-(2-(piperidin-3-ylamino)pyrimidin-4-yl)pyridazin-3-yl)oxy)naphthalen-1-yl)propane-1-sulfonamide hydrochloride

### Step 1: (S)-tert-Butyl 3-((4-(3-((2-methyl-5-(propylsulfonamido)naphthalen-1-yl)oxy)pyridazin-4-yl)pyrimidin-2-yl)amino)piperidine-1-carboxylate

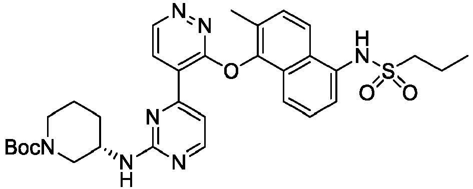

To a solution of (*S*)-*tert*-butyl 3-((4-(3-((5-amino-2-methylnaphthalen-1-yl)oxy)pyridazin-4-yl)pyrimidin-2-yl)amino)piperidine-1-carboxylate (40 mg, 0.076 mmol) in pyridine (0.50 mL) was added a solution of 1-propanesulfonyl chloride (13 mg, 0.091 mmol) in pyridine (0.50 mL) and the mixture was stirred at rt. After 24 h, a further portion of 1-propanesulfonyl chloride (13 mg, 0.091 mmol) in pyridine (0.20 mL) was added and after a further 24 h at rt the mixture was concentrated *in vacuo* to remove pyridine. The crude material was partitioned between ethyl acetate (40 mL) and saturated aqueous NaCl (10 mL). The phases were separated and the organic phase was dried over anhydrous magnesium sulfate, filtered and concentrated *in vacuo.* The crude material obtained was purified by silica flash column chromatography (0 - 100% ethyl acetate/dichloromethane) to provide 23 mg (48% yield) of the title compound as a pale yellow solid. LCMS (ESI) [M+H]⁺ = 634.3; ¹H NMR (500 MHz, CDCl₃) δ 9.09 (s, 1H), 8.91 - 8.05 (m, 2H), 7.89 (d, *J* = 8.7 Hz, 1H), 7.74 (s, 1H), 7.61 (s, 1H), 7.57 - 7.48 (m, *J* = 7.4 Hz, 2H), 7.39 (d, *J* = 8.7 Hz, 1H), 7.30 (t, *J* = 8.0 Hz, 1H), 6.18 - 5.42 (m, 1H), 4.08 (s, 1H), 3.86 - 2.89 (m, 4H), 3.09 (m, 2H), 2.32 (s, 3H), 2.07 (s, 1H), 1.90 - 1.80 (m, 3H), 1.72 (m, *J* = 8.6 Hz, 1H), 1.67 - 1.60 (m, 1H), 1.45 (s, 9H), 0.98 (t, *J* = 7.5 Hz, 3H).

### Step 2: (S)-N-(6-Methyl-5-((4-(2-(piperidin-3-ylamino)pyrimidin-4-yl)pyridazin-3-yl)oxy)naphthalen-1-yl)propane-1-sulfonamide hydrochloride

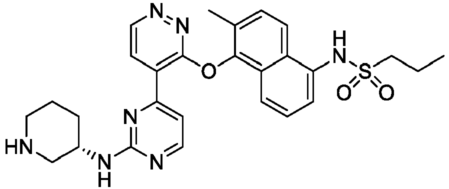

To a solution of (*S*)-*tert*-butyl 3-((4-(3-((2-methyl-5-(propylsulfonamido)naphthalen-1-yl)oxy)pyridazin-4-yl)pyrimidin-2-yl)amino)piperidine-1-carboxylate (23 mg, 0.04 mmol) in 1,4-dioxane (1 mL) was added 4 M HCl in 1,4-dioxane (0.24 mL, 0.96 mmol) and the mixture stirred at rt. After 1 h, the resulting solids were filtered off and washed with 1,4-dioxane, then with heptane, then 1,4-dioxane again. The solids were dissolved in water and lyophilized to provide 19 mg (92% yield) of the title compound as an off-white solid. LCMS (ESI) [M+H]⁺ = 534.2; ¹H NMR (500 MHz, *d₆*-DMSO) δ 9.87 (s, 1H), 9.27 - 8.82 (m, 2H), 9.17 (d, *J* = 4.9 Hz, 1H), 8.72-8.20 (m, 1H), 8.57 (d, *J* = 5.1 Hz, 1H), 8.19 (d, *J* = 8.8 Hz, 1H), 7.69 (d, *J* = 7.5 Hz, 1H), 7.59 (dd, *J* = 8.5, 5.4 Hz, 3H), 7.49 (d, *J* = 6.6 Hz, 1H), 7.48 - 7.41 (m, 1H), 4.32 (s, 1H), 3.41 - 3.36 (m, 1H), 3.21 (d, *J* = 11.2 Hz, 1H), 3.16 - 3.08 (m, 2H), 2.93 - 2.72 (m, 2H), 2.24 (s, 3H), 2.08 - 1.97 (m, 1H), 1.91 (dd, *J* = 10.3, 4.1 Hz, 1H), 1.76 (dq, *J* = 15.0, 7.5 Hz, 3H), 1.63 (dd, *J* = 20.8, 10.1 Hz, 1H), 0.96 (t, *J* = 7.4 Hz, 3H).

### Example 4

### N-(2-Fluoro-5-((2-(((3S,5S)-5-fluoropiperidin-3-yl)amino)-[4,5'-bipyrimidin]-4'-yl)oxy)-6-methylnaphthalen-1-yl)propane-1-sulfonamide

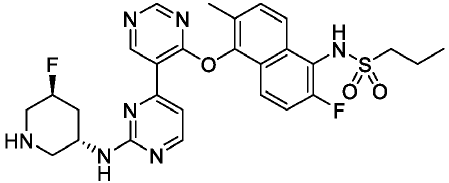

### Step 1: 4-Chloro-5-(2-methylsulfanylpyrimidin-4-yl)pyrimidine

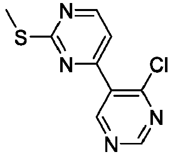

Under nitrogen, a mixture of 4-chloro-5-iodopyrimidine (2.0 g, 8.32 mmol), 2-(methylthio)-4-(tributylstannyl)pyrimidine (3.8 g, 9.15 mmol) and bis(triphenylphosphine)palladium(II) chloride (1.12 g, 1.60 mmol) in toluene (70 mL) was stirred for 16 h at 95 °C. The reaction was quenched with a saturated solution of potassium fluoride. The mixture was extracted with ethyl acetate and washed with brine. The organic layers were combined, dried over anhydrous sodium sulfate and concentrated under vacuum. The residue was purified by silica flash chromatography (ethyl acetate/petroleum ether 20:80) to afford the title compound (534 mg, 26.9% yield) as a yellow solid. LCMS (ESI): [M+H]⁺ = 239.1

### Step 2: 2-Fluoro-6-methyl-5-(5-(2-methylsulfanylpyrimidin-4-yl)pyrimidin-4-yl)oxy-naphthalen-1-amine

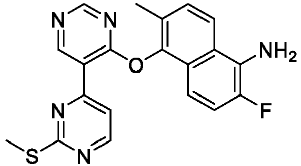

Under nitrogen, a mixture of 4-chloro-5-(2-methylsulfanylpyrimidin-4-yl)pyrimidine (0.30 g, 1.26 mmol), 5-amino-6-fluoro-2-methyl-naphthalen-1-ol hydrochloride (0.26 g, 1.13 mmol) and cesium carbonate (0.82 g, 2.51 mmol) in dimethyl sulfoxide (8 mL) was stirred for 1 h at 100 °C. The reaction mixture was quenched with water and extracted with ethyl acetate. The organic layers were combined. The organic layer was washed with brine and dried over anhydrous sodium sulfate. The solvent was concentrated under vacuum. The residue was purified by silica flash chromatography (ethyl acetate/petroleum ether, 1:1) to afford the title compound (350 mg, 70.8% yield) as a brown solid. LCMS (ESI): [M+H]⁺ = 394.1

### Step 3: 2-Fluoro-6-methyl-5-[5-(2-methylsulfinylpyrimidin-4-yl)pyrimidin-4-yl]oxy-naphthalen-1-amine

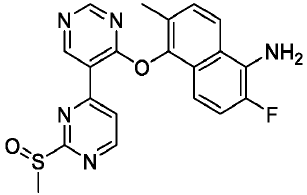

To a solution of 2-fluoro-6-methyl-5-(5-(2-methylsulfanylpyrimidin-4-yl)pyrimidin-4-yl]oxy-naphthalen-1-amine (0.32 g, 0.8100 mmol) in tetrahydrofuran (5 mL) and water (2 mL) was added potassium peroxymonosulfate (1.0 g, 1.63 mmol) and stirred for 1 h at room temperature. The reaction was quenched with a saturated solution of sodium bisulfite and extracted with ethyl acetate. The organic layer was washed with a saturated solution of sodium bicarbonate and was concentrated under reduced pressure. The title compound was used directly in the next step. LCMS (ESI) [M+H]⁺ = 410.1

### Step 4: Benzyl (3S,SS)-3-((4'-((5-amino-6-fluoro-2-methylnaphthalen-1-yl)oxy)-[4,5'-bipyrimidin]-2-yl)amino)-5-fluoropiperidine-1-carboxylate

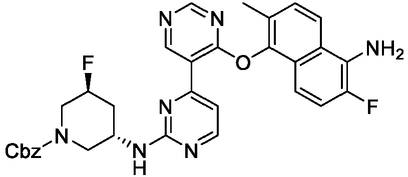

Under nitrogen, to a solution of benzyl (3*S*,5*S*)-3-amino-5-fluoro-piperidine-1-carboxylate hydrochloride (0.22 g, 0.75 mmol) and 2-fluoro-6-methyl-5-(5-(2-methylsulfinylpyrimidin-4-yl)pyrimidin-4-yl)oxy-naphthalen-1-amine (0.3 g, 0.7300 mmol) in 1,4-dioxane (5 mL) was added *N*,*N-*diisopropylethylamine (0.38 mL, 2.32 mmol) and stirred for 36 h at 110 °C. The solvent was removed under vacuum. The residue was purified by silica flash chromatography (ethyl acetate/petroleum ether 1:1) to afford the title compound (179 mg, 40.9% yield) as a brown solid. LCMS (ESI): [M+H]⁺ = 598.3

### Step 5: Benzyl (3S,5S)-3-fluoro-5-((4'-((6-fluoro-2-methyl-5-(propylsulfonamido)naphthalen-1-yl)oxy)-[4,5'-bipyrimidin]-2-yl)amino)piperidine-1-carboxylate

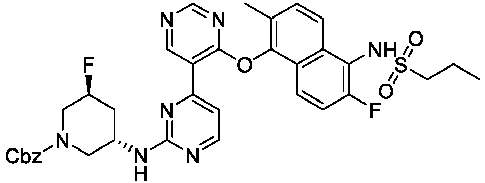

To a solution of benzyl (3*S*,5*S*)-3-((4'-((5-amino-6-fluoro-2-methylnaphthalen-1-yl)oxy)-[4,5'-bipyrimidin]-2-yl)amino)-5-fluoropiperidine-1-carboxylate (60 mg, 0.10 mmol) in pyridine (1 mL) was added 1-propanesulfonyl chloride (57 mg, 0.40 mmol), the reaction mixture was stirred for 36 h at room temperature. The reaction was quenched with water and extracted with ethyl acetate and washed with brine. The organic layer was concentrated under vacuum. The residue was purified by silica flash chromatography gel (ethyl acetate/petroleum ether 3:1) to afford the title compound (62 mg, 87.7% yield) as a black solid. LCMS (ESI): [M+H]⁺ = 704.2

### Step 6: N-(2-Fluoro-5-((2-(((3S,5S)-5-fluoropiperidin-3-yl)amino)-[4,5'-bipyrimidin]-4'-yl)oxy)-6-methylnaphthalen-1-yl)propane-1-sulfonamide

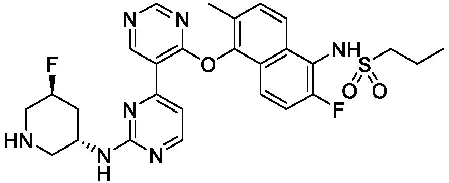

To a solution of benzyl (3*S*,5*S*)-3-fluoro-5-((4'-((6-fluoro-2-methyl-5-(propylsulfonamido)naphthalen-1-yl)oxy)-[4,5'-bipyrimidin]-2-yl)amino)piperidine-1-carboxylate (60 mg, 0.0900 mmol) in dichloromethane (3 mL) was added a solution of 33% HBr in acetic acid (1 mL). The reaction mixture was stirred for 1 h at room temperature. The solvent was removed under vacuum and the residue was dissolved in dichloromethane and washed with a saturated solution of sodium bicarbonate. The solvent was removed under vacuum and the crude product was purified by Prep-HPLC to afford the title compound (2.8 mg, 5.8% yield) as a white solid. LCMS (ESI): [M+H]⁺ = 570.2; ¹H NMR (300 MHz, CD₃OD) δ 9.46 (s, 1H), 8.60 (s, 1H), 8.46 (d, *J* = 5.2 Hz, 1H), 8.26 (d, *J* = 8.8 Hz, 1H), 7.80 - 7.69 (m, 1H), 7.59 (t, *J* = 6.9 Hz, 2H), 7.37 (t, *J* = 9.7 Hz, 1H), 4.88 (d, *J* = 47.4 Hz, 1H), 4.42 (s, 1H), 3.31 - 3.13 (m, 4H), 2.90 - 2.70 (m, 1H), 2.61 - 2.37 (m, 2H), 2.30 (s, 3H), 2.10 - 1.65 (m, 3H), 1.11 (t, *J* = 7.4 Hz, 3H).

### Example 5

### 3,3-Difluoro-N-(2-fluoro-5-((4-(2-(((3S,5S)-5-fluoropiperidin-3-yl)amino)pyrimidin-4-yl)pyridazin-3-yl)oxy)-6-methylnaphthalen-1-yl)butane-1-sulfonamide

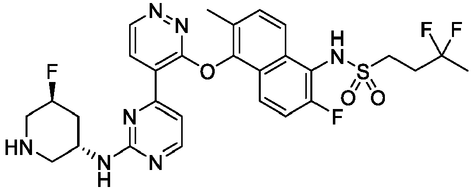

### Step 1: 4-Chloro-2-(4-methoxybenzyl)pyridazin-3(2H)-one

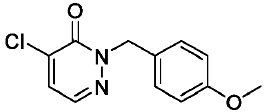

To a solution of 5-chloro-1*H*-pyridazin-6-one (1.0 g, 7.66 mmol) in *N,N-*dimethylformamide (20 mL) was added sodium hydride (0.50 g, 12.5 mmol) at 0 °C and the reaction mixture was stirred for 0.5 h at 0 °C. 4-Methoxybenzyl chloride (1.5 g, 9.58 mmol) was then added and stirred for 16 h at 25 °C. The reaction mixture was quenched with brine and extracted with ethyl acetate. The organic layers were combined and washed with brine. The solvent was removed. The residue was purified by silica flash chromatography eluting with ethyl acetate/petroleum ether (1/10) to afford the title compound (860 mg, 44.8% yield) as a yellow oil. LCMS (ESI): [M+H]⁺ = 251.2

### Step 2: 2-(4-Methoxybenzyl)-4-(2-(methylthio)pyrimidin-4-yl)pyridazin-3(2H)-one

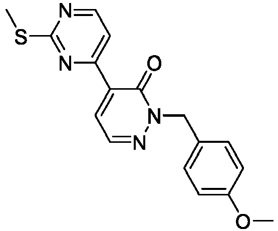

Under nitrogen, a solution of 4-chloro-2-(4-methoxybenzyl)pyridazin-3(2H)-one (0.86 g, 3.43 mmol), 2-(methylthio)-4-(tributylstannyl)pyrimidine (1.4 g, 3.37mmol) and tetrakis(triphenylphosphine)palladium (0.35 g, 0.30mmol) in *N,N-*dimethylformamide (15mL) was stirred for 18 h at 100 °C. The reaction was quenched with a saturated solution of potassium fluoride and extracted with ethyl acetate. The organic layers were washed with brine and the solvent was removed under vacuum. The residue was purified by silica flash chromatography (ethyl acetate/petroleum ether 1:5) to afford the title compound (700 mg, 59.9% yield) as a white solid. LCMS (ESI): [M+H]⁺ = 341.2

### Step 3: 4-(2-(Methylthio)pyrimidin-4-yl)pyridazin-3(2H)-one

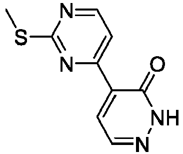

A solution of 2-(4-methoxybenzyl)-4-(2-(methylthio)pyrimidin-4-yl)pyridazin-3(2*H*)-one (0.7 g, 2.06 mmol) in trifluoroacetic acid (10 mL) and trifluoromethanesulfonic acid (1 mL) was stirred for 2 h at room temperature. The reaction was quenched with ice/water and extracted with ethyl acetate. The organic layer was washed with brine. The solvent was removed under vacuum to afford the title compound (380 mg, 83.9% yield) as an orange solid. LCMS (ESI): [M+H]⁺ = 221.2.

### Step 4: 3-Chloro-4-(2-(methylthio)pyrimidin-4-yl)pyridazine

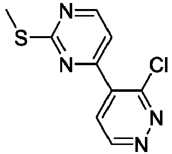

A solution of 4-(2-(methylthio)pyrimidin-4-yl)pyridazin-3(2*H*)-one (380 mg, 1.73 mmol) in phosphorus oxychloride (10 mL) was stirred for 4 h at 105 °C. The solvent was removed under vacuum. The reaction was quenched with ice/water and extracted with ethyl acetate. The solvent was removed under vacuum. The residue was purified by silica flash chromatography (ethyl acetate/petroleum ether 1:1) to afford the title compound (340 mg, 82.6% yield) as a yellow solid. LCMS (ESI): [M+H]⁺ = 239.1.

### Step 5: 2-Fluoro-6-methyl-5-((4-(2-(methylthio)pyrimidin-4-yl)pyridazin-3-yl)oxy)naphthalen-1-amine

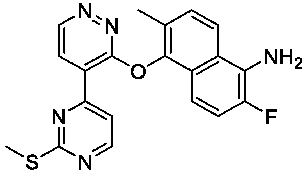

Under nitrogen, a mixture of 5-amino-6-fluoro-2-methyl-naphthalen-1-ol (315 mg, 1.65 mmol), 3-chloro-4-(2-(methylthio)pyrimidin-4-yl)pyridazine (350 mg, 1.47 mmol), cesium carbonate (875 mg, 2.68 mmol) in 1-methyl-2-pyrrolidinone (7 mL) was stirred for 2 h at 80 °C. The reaction mixture was diluted with water and extracted with ethyl acetate. The organic layers were combined. The organic layer was washed with brine and dried over anhydrous sodium sulfate. The solvent was removed under vacuum. The residue was purified by silica flash chromatography eluting with ethyl acetate/petroleum ether (1/1) to afford the title compound (350 mg, 57.6% yield) as a brown solid. LCMS (ESI) [M+H]⁺ = 394.1.

### Step 6: 2-Fluoro-6-methyl-5-((4-(2-(methylsulfinyl)pyrimidin-4-yl)pyridazin-3-yl)oxy)naphthalen-1-amine

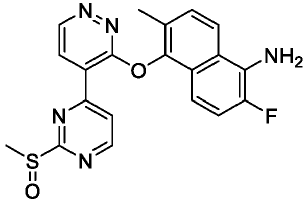

To a solution of 2-fluoro-6-methyl-5-((4-(2-(methylthio)pyrimidin-4-yl)pyridazin-3-yl)oxy)naphthalen-1-amine (350 mg, 0.89 mmol) in tetrahydrofuran (7 mL) and water (3 mL) was added potassium peroxymonosulfate (531 mg, 3.14 mmol) and stirred for 1 h at 25 °C. The reaction was quenched with a saturated solution of sodium bisulfite and extracted with ethyl acetate. The organic layer was washed with a saturated solution of sodium bicarbonate. The solvent was removed under vacuum to afford the title compound (200 mg, 52.2% yield) as an off-white solid. The crude product would be directly used in the next step without purification. LCMS (ESI): [M+H]⁺ = 410.1

### Step 7: Benzyl (3S,5S)-3-((4-(3-((5-amino-6-fluoro-2-methylnaphthalen-1-yl)oxy)pyridazin-4-yl)pyrimidin-2-yl)amino)-5-fluoropiperidine-1-carboxylate

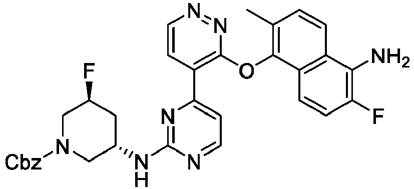

Under nitrogen, a mixture of benzyl (3*S*,5*S*)-3-amino-5-fluoro-piperidine-1-carboxylate hydrochloride (170 mg, 0.67 mmol), 2-fluoro-6-methyl-5-((4-(2-(methylsulfinyl)pyrimidin-4-yl)pyridazin-3-yl)oxy)naphthalen-1-amine (200 mg, 0.49 mmol), *N,N-*diisopropylethylamine (0.45 mL, 2.71 mmol), in 1,4-dioxane (4 mL) was stirred for 2 h at 115 °C. The solvent was removed under vacuum. The residue was purified by silica flash chromatography eluting with methanol /dichloromethane (6%) to afford the title compound (160 mg, 52.1% yield)] as a brown solid. LCMS (ESI): [M+H]⁺ = 598.2

### Step 8: Benzyl (3S,5S)-3-((4-(3-((5-((3,3-difluorobutyl)sulfonamido)-6-fluoro-2-methylnaphthalen-1-yl)oxy)pyridazin-4-yl)pyrimidin-2-yl)amino)-5-fluoropiperidine-1-carboxylate

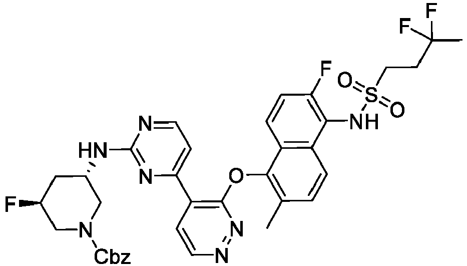

Under nitrogen, a solution of benzyl (3*S*,5*S*)-3-((4-(3-((5-amino-6-fluoro-2-methylnaphthalen-1-yl)oxy)pyridazin-4-yl)pyrimidin-2-yl)amino)-5-fluoropiperidine-1-carboxylate (50 mg, 0.08 mmol) and *N*-methylmorpholine (50 mg, 0.49 mmol) in dichloromethane (1 mL) was added 3,3-difluorobutane-1-sulfonyl chloride (40 mg, 0.21 mmol) and stirred for 1 h at 25 °C. The reaction mixture was diluted with water and extracted with ethyl acetate. The organic layers were combined. The organic layer was washed with brine. The organic layer was dried over anhydrous sodium sulfate and concentrated under vacuum.

To a solution of the residue in dichloromethane (1 mL) was added a saturated solution of lithium hydroxide (2 mL). The resulting solution was stirred for 1 h at room temperature. The reaction mixture was diluted with water and extracted with ethyl acetate. The organic layer was dried over anhydrous sodium sulfate and concentrated under vacuum. The residue was purified by silica flash chromatography eluting with methanol/dichloromethane (6%) to afford the title compound (45 mg, 67.8% yield) as a brown solid. LCMS (ESI): [M+H]+ = 754.2.

### Step 9: 3,3-Difluoro-N-(2-fluoro-5-((4-(2-(((3S,5S)-5-fluoropiperidin-3-yl)amino)pyrimidin-4-yl)pyridazin-3-yl)oxy)-6-methylnaphthalen-1-yl)butane-1-sulfonamide

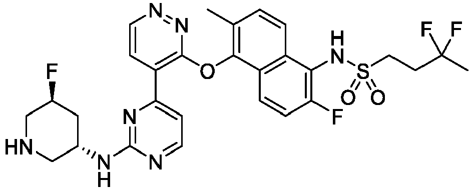

To a solution of benzyl (3*S*,5*S*)-3-((4-(3-((5-((3,3-difluorobutyl)sulfonamido)-6-fluoro-2-methylnaphthalen-1-yl)oxy)pyridazin-4-yl)pyrimidin-2-yl)amino)-5-fluoropiperidine-1-carboxylate (45 mg, 0.06 mmol) in dichloromethane (0.50 mL) was added 33% HBr in acetic acid (0.1 mL) and stirred for 1 h at room temperature. The solvent was removed under vacuum. The crude product was purified by Prep-HPLC to afford the title compound (10 mg, 24.3% yield) as a white solid. LCMS (ESI): [M+H]⁺ = 620.2; ¹H NMR (300 MHz, CD₃OD) δ 9.09 (d, *J* = 4.9 Hz, 1H), 8.51 (d, *J* = 5.1 Hz, 2H), 8.22 (d, *J* = 8.7 Hz, 1H), 7.85 (dd, *J* = 9.3, 5.1 Hz, 1H), 7.70 - 7.59 (m, 2H), 7.39 (t, *J* = 9.5 Hz, 1H), 4.89 (d, *J* = 47.2 Hz, 1H), 4.43 (s, 1H), 3.49 - 3.38 (m, 3H), 3.14 (t, *J* = 12.8 Hz, 1H), 2.90 - 2.68 (m, 1H), 2.65 - 2.36 (m, 4H), 2.31 (s, 3H), 1.92 (s, 1H), 1.71 (t, *J* = 18.5 Hz, 3H).

### Example 6

### N-(2-Fluoro-5-((4-(2-(((3S,5S)-5-fluoropiperidin-3-yl)amino)pyrimidin-4-yl)pyridazin-3-yl)oxy)-6-methylnaphthalen-1-yl)propane-1-sulfonamide

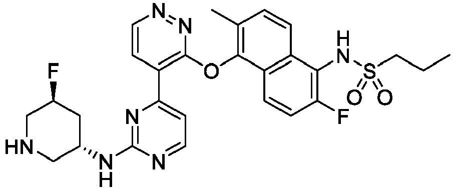

### Step 1: Benzyl (3S,SS)-3-fluoro-5-((4-(3-((6-fluoro-2-methyl-5-(propylsulfonamido)naphthalen-1-yl)oxy)pyridazin-4-yl)pyrimidin-2-yl)amino)piperidine-1-carboxylate

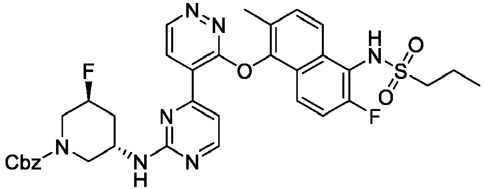

Under nitrogen, to a solution of benzyl (3*S*,5*S*)-3-((4-(3-((5-amino-6-fluoro-2-methylnaphthalen-1 -yl)oxy)pyridazin-4-yl)pyrimidin-2-yl)amino)-5-fluoropiperidine-1 - carboxylate (45.0 mg, 0.08 mmol) in pyridine (0.25 mL) was added 1-propanesulfonyl chloride (26 mg, 0.18 mmol) and the reaction mixture was stirred for 2 h at 25 °C. The reaction mixture was diluted with water and extracted with ethyl acetate, washed with brine, dried over anhydrous sodium sulfate and concentrated under vacuum. The residue was purified by silica flash chromatography eluting with methanol/dichloromethane (5:95) to afford the tittle compound (45 mg, 80.7% yield) as a brown solid. LCMS (ESI): [M+H]⁺ = 704.2.

### Step 2: N-(2-Fluoro-5-((4-(2-(((3S,5S)-5 -fluoropiperidin-3-yl)amino)pyrimidin-4-yl)pyridazin-3-yl)oxy)-6-methylnaphthalen-1-yl)propane-1-sulfonamide

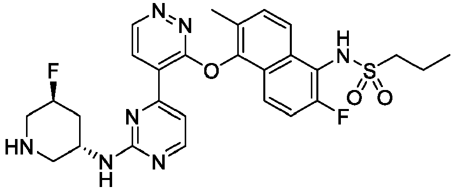

To a solution of benzyl (*3*S,5*S*)-3-fluoro-5-((4-(3-((6-fluoro-2-methyl-5-(propylsulfonamido)naphthalen-1-yl)oxy)pyridazin-4-yl)pyrimidin-2-yl)amino)piperidine-1-carboxylate (45 mg, 0.06 mmol) in dichloromethane (0.50 mL) was added 33% HBr in acetic acid (1 mL). The reaction mixture was then stirred at 25 °C for 1 h and the organic layer was concentrated under vacuum. The crude product was purified by Prep-HPLC to the title compound (12.3 mg, 32.9% yield) as an off-white solid. LCMS (ESI): [M+H]⁺ = 570.2; ¹H NMR (400 MHz, CD₃OD) δ 9.09 (d, *J* = 4.9 Hz, 1H), 8.52 (d, *J* = 5.1 Hz, 2H), 8.23 (d, *J* = 8.7 Hz, 1H), 7.84 (dd, *J* = 9.4, 5.2 Hz, 1H), 7.64 (dd, *J* = 16.9, 7.0 Hz, 2H), 7.38 (t, *J* = 9.5 Hz, 1H), 4.92 (d, *J* = 47.7 Hz, 1H), 4.45 (s, 1H), 3.37 (s, 1H), 3.27 - 3.13 (m, 3H), 2.83 (dd, *J* = 37.1, 14.2 Hz, 1H), 2.62 - 2.38 (m, 2H), 2.31 (s, 3H), 2.05 - 1.93 (m, 2H), 1.93 - 1.75 (m, 1H) 1.12 (t, *J* = 7.5 Hz, 3H).

### Biological Examples

Exemplary compounds of Formula (I) or Formula (II) were tested to assess compound inhibition of IRE1. The Kᵢ for each exemplary compound was determined.

### Example 7: IRE1α TR-FRET Competition Binding Assay

To determine the affinity of compound binding to the kinase domain of IRE1 alpha, a Time Resolved Fluorescence Resonance Energy Transfer (TR-FRET) competition assay was used. A His-tagged IRE1 alpha kinase dead construct containing the kinase and RNase domains (KR, AA G547-L977, D688N) was expressed in Sf9 insect cells. The purified protein (final concentration 0.006 µM micromolar) was pre-incubated with anti-His Europium labeled antibody (Life Technologies PV5596, final concentration 0.002 µM micromolar) for one hour at 4 °C in 1X TR-FRET Assay Buffer (50 mM HEPES, pH 7.5, 10 mM MgCl₂, 0.083 mM Brij 35, 1 mM DTT, and 0.1% bovine gamma globulin) prior to addition to test compounds. A fluorescent labeled probe based on an ATP competitive inhibitor (Kinase Tracer 236, Life Technologies PV5592) is added to a final concentration of 0.1 µM (micromolar). Reactions were carried out for one hour at room temperature in a final volume of 20 µL (microliter) in 384 well white ProxiPlates (Perkin Elmer 6008289). Binding of the tracer to the IRE1 protein alpha was detected in an Envision instrument (PerkinElmer) equipped with a TRF laser option and a LANCE/Delfia DualBias D400/D630 mirror (Ex 347 nm, 1^{st} Em 665 nm, 2^{nd} Em 615 nm).
Exemplary compounds depicted in Table 1 were tested in the IRE1α binding assays. Exemplary IC₅₀ values determined are listed in Table 3.

**Table 3**

| Compound No. | IRE1α TR-FRET IC₅₀ (µM) |
|---|---|
| 1 | 0.0061 |
| 2 | 0.038 |
| 3 | 0.012 |
| 4 | 0.0033 |
| 5 | 0.0031 |
| 6 | 0.0071 |

### Example 8: IRE1 alpha RNase Activity Assay

Inhibitors of the RNase activity of IRE1α were assessed by Fluorescence (Forster) resonance energy transfer (FRET) using a mini-XBP-1 stem-loop RNA as a substrate for the IRE1α RNase activity. A 5'-Carboxyfluorescein (FAM)-and 3'-Black Hole Quencher (BHQ)-labeled XBP1 single stem-loop mini-substrate oligonucleotide, TAQMAN^{®} (Roche Molecular Systems) probe (Kutyavin et al (2000) Nucleic Acids Research,. 28(2):655-661) is cleaved by IRE1α. When the oligonucleotide is intact, the fluorescence signal is quenched by BHQ. Upon cleavage, the fluorescence is no longer quenched and can be quantified.

An IRE1 alpha construct corresponding to the linker, kinase and RNase domains (LKR, AA Q470-L977) was expressed in Sf9 insect cells. All reagent preparation and procedures are done under RNase free conditions. Test compounds and purified enzyme were combined in RNase Assay Buffer (20 mM HEPES, pH 7.5, 50 mM KAc, 1 mM MgAc, 1 mM DTT, and 0.05% Triton X-100) in a 384 well white ProxiPlate (Perkin Elmer 6008289). Upon addition of the RNA substrate (final assay volume 20 µL, microliter), the plates were placed into a Flexstation 3 instrument (Molecular Devices) for kinetic fluorescence reading at 2 minute intervals (Ex 485, Em 535). The velocity of the reaction, using the first 50 minutes, was used to calculate the RNase activity and inhibition of test compounds.

Exemplary compounds in Table 1 had activity in the IRE1α RNase activity.

### Example 9: IRE1 alpha Ribonuclease Luciferase Reporter Assay

HEK293 cells expressing a pBABE.puro HA-2xXBP1delta DBD firefly luciferase reporter (Mendez etal., (2015) "Endoplasmic reticulum stress-independent activation of unfolded protein response kinases by a small molecule ATP-mimic", eLife;4:e05434) were cultured in DMEM high glucose media containing L-glutamine, 10% fetal bovine serum, 100 units/mL of penicillin and 100 µg/mL (microgram per milliliter) of streptomycin, plus 2 µg/ml puromycin to maintain selective pressure. Upon stimulation of IRE1 and activation of the endogenous RNase activity, a 26 nt intron is removed from XBP1 resulting in a frame shift allowing the transcription of the luciferase.

Cells were seeded without puromycin at 10,000/well in 384 well clear bottom white tissue culture plates (Corning 3707), 25 µL volume. The following morning, test compounds were added and incubated for one hour at 37°C prior to stimulation of the cells with thapsigargin at 50 µM (micromolar) final concentration for an additional 5 hours. After equilibration to room temperature, 25 µL (microliters) of One-Glo^{®} luciferase detection reagent (Promega cat # E6120) was added, plates sealed and shaken for 5 minutes to lyse cells, then luciferase quantified by luminescence detection using an Envision instrument (PerkinElmer).

Exemplary compounds in Table 1 had activity in the XBP1s-LUC reporter assay described herein.

It is to be noted that the term "a" or "an" entity refers to one or more of that entity; for example, "a polypeptide" is understood to represent one or more polypeptides. As such, the terms "a" (or "an"), "one or more," and "at least one" can be used interchangeably herein.

All technical and scientific terms used herein have the same meaning. Efforts have been made to ensure accuracy with respect to numbers used (e.g. amounts, temperature, etc.) but some experimental errors and deviations should be accounted for.

Throughout this specification and the claims, the words "comprise," "comprises," and "comprising" are used in a non-exclusive sense, except where the context requires otherwise. It is understood that embodiments described herein include "consisting of" and/or "consisting essentially of" embodiments.

As used herein, the term "about," when referring to a value is meant to encompass variations of, in some embodiments ± 50%, in some embodiments ± 20%, in some embodiments ± 10%, in some embodiments ± 5%, in some embodiments ± 1%, in some embodiments ± 0.5%, and in some embodiments ± 0.1% from the specified amount, as such variations are appropriate to perform the disclosed methods or employ the disclosed compositions.

Where a range of values is provided, it is understood that each intervening value, to the tenth of the unit of the lower limit, unless the context clearly dictates otherwise, between the upper and lower limit of the range and any other stated or intervening value in that stated range, is encompassed herein. The upper and lower limits of these small ranges which can independently be included in the smaller rangers is also encompassed herein, subject to any specifically excluded limit in the stated range. Where the stated range includes one or both of the limits, ranges excluding either or both of those included limits are also included herein.

## Claims

1. A compound having formula (I) or formula (II); 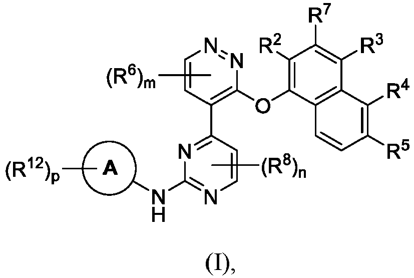 or 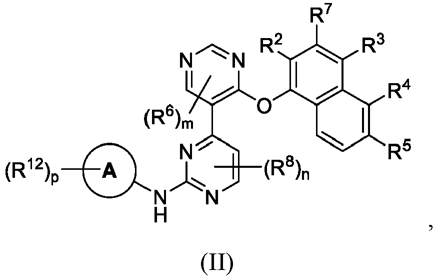
or a pharmaceutically acceptable salt thereof,
wherein;
R¹ is hydrogen, halogen, -CN, -NO₂, -NR^{a}R^{b}, -NR^{a}C(O)R^{b}, -NR^{a}C(O)R^{b}, - NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a}, - OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b}, - P(O)R^{a}R^{b}, R¹⁰-substituted or unsubstituted C₁₋₆ alkyl, R¹⁰-substituted or unsubstituted 2 to 6 membered heteroalkyl, R¹⁰-substituted or unsubstituted C₁₋₆ haloalkyl, R¹⁰-substituted or unsubstituted C₃₋C₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heterocycloalkyl;
R² is hydrogen, halogen, -CN, R¹⁰-substituted or unsubstituted C₁₋₆ alkoxy, or R¹⁰-substituted or unsubstituted C₁₋₆ alkyl;
R³ is -L¹-R¹, hydrogen, halogen, -CN, -NO₂, -NR^{a}R^{b}, -NR^{a}C(O)R^{b}, -NR^{a}C(O)R^{b}, -NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a}, - OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b}, - P(O)R^{a}R^{b}, R¹⁰-substituted or unsubstituted C₁₋₆ alkoxy, R¹⁰-substituted or unsubstituted C₁₋₆ haloalkyl, R¹⁰-substituted or unsubstituted C₁₋₆ alkyl, R¹⁰-substituted or unsubstituted C₃₋₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heteroaryl;
R⁴ is -L²-R⁹-, halogen, -CN, -NO₂, -NR^{a}R^{b}, -NR^{a}C(O)R^{b}, -NR^{a}C(O)R^{b}, - NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a}, - OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b}, - P(O)R^{a}R^{b}, R¹⁰-substituted or unsubstituted C₁₋₆ alkoxy, R¹⁰-substituted or unsubstituted C₁₋₆ haloalkyl, R¹⁰-substituted or unsubstituted C₁₋₆ alkyl, R¹⁰-substituted or unsubstituted 2 to 6 membered heteroalkyl, R¹⁰-substituted or unsubstituted C₃₋₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heteroaryl;
R⁵ is hydrogen, halogen, or R¹⁰-substituted or unsubstituted C₁₋₃ alkyl;
each R⁶ is independently hydrogen, halogen, -CN, -NO₂, -OR^{a}, -NR^{a}R^{b}, -C(O)R^{a}, - C(O)OR^{a}, -SR^{a}, -S(O)₂R^{a}, -P(O)R^{a}R^{b}, R¹⁰-substituted or unsubstituted C₁₋₆ alkoxy, R¹⁰-substituted or unsubstituted C₁₋₆ haloalkyl, R¹⁰-substituted or unsubstituted C₁₋₆ alkyl, R¹⁰-substituted or unsubstituted C₃₋₆ cycloalkyl, -SO₂(C₁₋₆ alkyl), or -SO₂(C₁₋₆ haloalkyl);
R⁷ is hydrogen, halogen, -CN, R¹⁰-substituted or unsubstituted C₁₋₆ alkyl, R¹⁰-substituted or unsubstituted C₁₋₆ alkoxy, R¹⁰-substituted or unsubstituted C₁₋₆ haloalkyl, or R¹⁰-substituted or unsubstituted C₃₋₆ cycloalkyl;
each R⁸ is independently hydrogen, halogen, or R¹⁰-substituted or unsubstituted C₁₋₃ alkyl;
R⁹ is hydrogen, halogen, R¹⁰-substituted or unsubstituted C₁₋₆ alkyl, R¹⁰-substituted or unsubstituted 2 to 6 membered heteroalkyl, R¹⁰-substituted or unsubstituted C₁₋₆ haloalkyl, R¹⁰-substituted or unsubstituted C₃₋C₇ cycloalkyl, R¹⁰-substituted or unsubstituted 3 to 7 membered heterocycloalkyl, R¹⁰-substituted or unsubstituted C₅₋₆ aryl, or R¹⁰-substituted or unsubstituted 5 or 6 membered heterocycloalkyl;
each R¹⁰ is independently halogen, -N₃, -CF₃, -CN, -NO₂, -NR^{a}R^{b}, -NR^{a}C(O)R^{b}, -NR^{a}C(O)R^{b}, -NR^{a}C(O)OR^{b}, -NR^{a}C(O)NR^{b}, -S(O)₂NR^{a}, -C(O)R^{a}, -C(O)OR^{a}, -C(O)NR^{a}R^{b}, - OR^{a}, -OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, - S(O)₂NR^{a}R^{b}, -P(O)R^{a}R^{b}, R¹¹-substituted or unsubstituted C₁₋₆ alkyl, R¹¹-substituted or unsubstituted 2 to 6 membered heteroalkyl, R¹¹-substituted or unsubstituted C₃₋₇ cycloalkyl, R¹¹-substituted or unsubstituted 3 to 7 membered heterocycloalkyl, R¹¹-substituted or unsubstituted C₅₋₆ aryl, or R¹¹-substituted or unsubstituted 5 to 6 membered heteroaryl;
each R¹¹ is independently halogen, -N₃, -CF₃, -CCl₃, -CBr₃, -CI₃, -CN, -CHO, -OH, -NH₂, -COOH, -CONH₂, -NO₂, -SH, -SO₂Cl, -SO₃H, -SO₄H, -SO₂NH₂, unsubstituted C₁₋₆ alkyl, unsubstituted 2 to 6 membered heteroalkyl, unsubstituted C₃₋₇ cycloalkyl, unsubstituted 3 to 7 membered heterocycloalkyl, unsubstituted C₅₋₆ aryl, or unsubstituted 5 to 6 membered heteroaryl;
each R¹² is independently hydrogen, halogen, a NPG, or R¹⁰-substituted or unsubstituted C₁₋₃ alkyl;
L¹ is -NHSO₂-, -NHC(O)-, NHCO(O)-, -NHC(O)NH-, -NH-, -O-, -S-, or -SO₂-;
L² is -NHSO₂-, -NHC(O)-, NHCO(O)-, -NHC(O)NH-, -NH-, -O-, -S-, or -SO₂-;
each R^{a} and R^{b} is independently hydrogen, unsubstituted C₁₋₆ alkyl, unsubstituted C₁₋₆ haloalkyl, unsubstituted C₁₋₆ alkoxy, unsubstituted C₂₋₆ alkenyl, unsubstituted C₂₋₆ alkynyl, unsubstituted C₃₋₇ cycloalkyl, unsubstituted 3 to 7 membered heterocycloalkyl, unsubstituted C₅₋₆ aryl, or unsubstituted 5 or 6 membered heteroaryl;
m is 1 or 2;
n is 1 or 2;
p is 1, 2, 3, 4, 5 or 6; and
Ring A is C₃₋₆ cycloalkyl, 3 to 6 membered heterocycloalkyl, C₅₋₆ aryl, or 5 or 6 membered heteroaryl.

2. The compound of claim 1, wherein:
(i) ring A is 6 membered heterocycloalkyl; or
(ii) ring A is piperidinyl.

3. The compound of claim 2, wherein R¹² is halogen or C₁₋₃ alkyl, and optionally further wherein:
- n is p is 1 or 2; or
- R¹² is -F; or
- n is p is 1 or 2, and R¹² is -F; or
- p is 2 and R¹² is -F and methyl; or
- ring A has the structure:

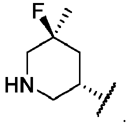

4. The compound of any one of claims 1 to 3, wherein:
- R⁶ is hydrogen; or
- R⁸ is hydrogen; or
- R⁶ is hydrogen and R⁸ is hydrogen; or
- R³ is hydrogen; or
- R⁶ is hydrogen and R³ is hydrogen; or
- R⁸ is hydrogen and R³ is hydrogen; or
- R⁶ is hydrogen and R⁸ is hydrogen and R³ is hydrogen.

5. The compound of any one of claims 1 to 4, wherein: R² is unsubstituted C₁₋₃ alkyl; or R² is methyl.

6. The compound of any one of claims 1 to 5, wherein R⁴ is -L²-R⁹-,
and optionally wherein:
(i) R⁹ is R¹⁰-substituted or unsubstituted phenyl, optionally further wherein L² is -NHSO₂- or -NHC(O)-; or
(ii) R⁹ is R¹⁰-substituted phenyl and R¹⁰ is halogen, optionally further wherein L² is -NHSO₂- or -NHC(O)-; or
(iii) R⁹ is R¹⁰-substituted or unsubstituted C₃₋₆ cycloalkyl, optionally further wherein L² is -NHSO₂- or -NHC(O)-; or
(iv) R⁹ is unsubstituted cyclopropanyl, optionally further wherein L² is -NHSO₂- or - NHC(O)-; or
(v) R⁹ is 2 to 6 membered unsubstituted haloalkyl, optionally further wherein L² is -NHSO₂- or -NHC(O)-; or
(vi) R⁹ is 3,3 difluorobutyl, optionally further wherein L² is -NHSO₂- or -NHC(O)-; or
(vii) R⁹ is R¹⁰-substituted or unsubstituted C₁₋₃ alkyl, optionally further wherein L² is - NHSO₂- or -NHC(O)-; or
(viii) R⁹ is unsubstituted C₁₋₃ alkyl, optionally further wherein L² is -NHSO₂- or -NHC(O)-.

7. The compound of any one of claims 1 to 6, wherein: R⁵ is halogen; or R⁵ is hydrogen; or R⁵ is unsubstituted C₁₋₃ alkyl.

8. The compound of claim 1 having:
(i) formula (I) or a pharmaceutically acceptable salt thereof; or
(ii) formula: 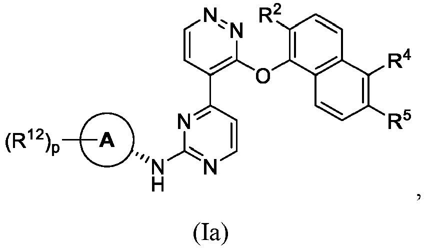 or a pharmaceutically acceptable salt thereof; or
(iii) formula: 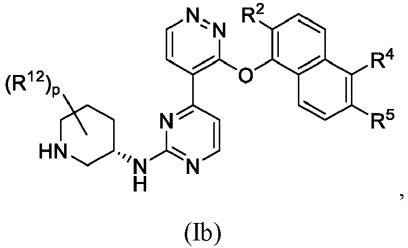 or a pharmaceutically acceptable salt thereof; or
(iv) formula: 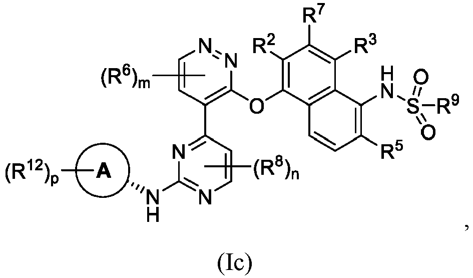 or a pharmaceutically acceptable salt thereof; or
(v) formula: 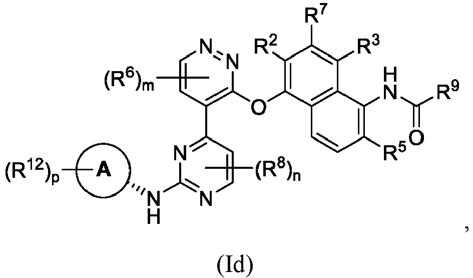 or a pharmaceutically acceptable salt thereof; or
(vi) formula: 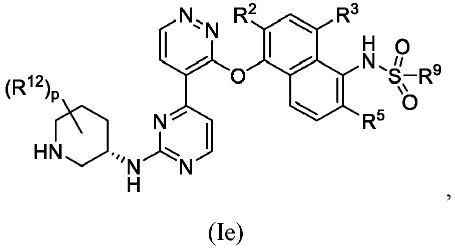 or a pharmaceutically acceptable salt thereof; or
(vii) formula: 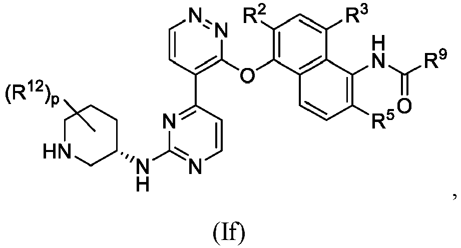 or a pharmaceutically acceptable salt thereof.

9. The compound of claim 1 having:
(i) formula (II) or a pharmaceutically acceptable salt thereof; or
(ii) formula: 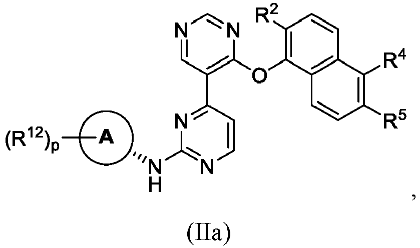 or a pharmaceutically acceptable salt thereof; or
(iii) formula: 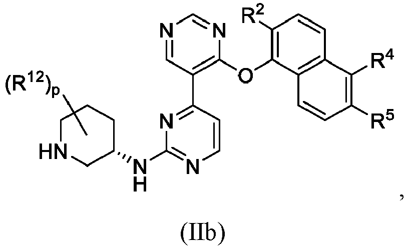 or a pharmaceutically acceptable salt thereof; or
(iv) formula: 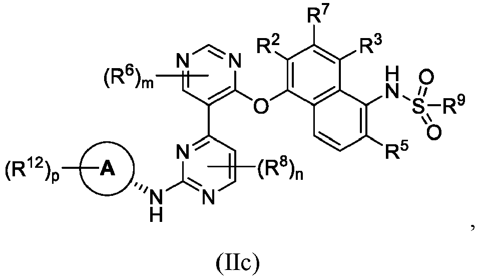 or a pharmaceutically acceptable salt thereof; or
(v) formula: 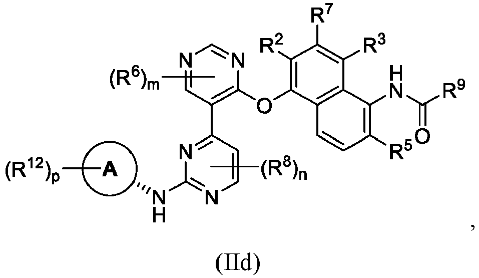 or a pharmaceutically acceptable salt thereof; or
(vi) formula: 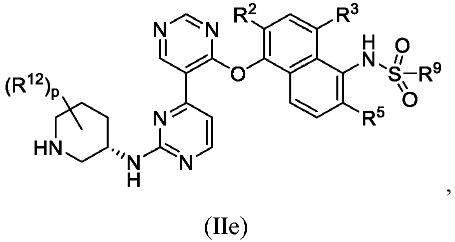 or a pharmaceutically acceptable salt thereof; or
(vii) formula: 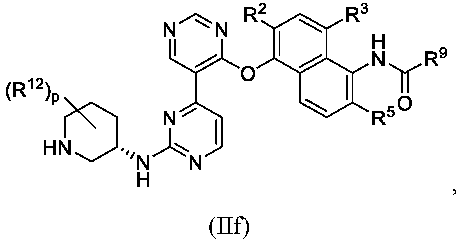 or a pharmaceutically acceptable salt thereof.

10. The compound or pharmaceutically acceptable salt thereof of claim 1, wherein the compound comprises the following compound No 1, No 2, No 3, No 4, No 5, No 6, No 7, No 8, or No 9:
| No | Structure | Name |
|---|---|---|
| 1 | 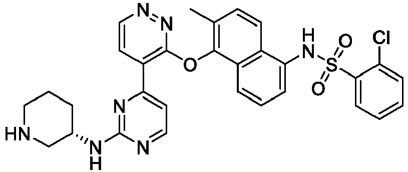 | (*S*)-2-chloro-*N*-(6-methyl-5-((4-(2-(piperidin-3-ylamino)pyrimidin-4-yl)pyridazin-3-yl)oxy)naphthalen-1-yl)benzenesulfonamide |
| 2 | 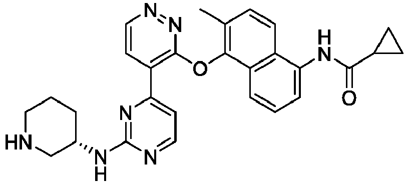 | (*S*)-*N*-(6-methyl-5-((4-(2-(piperidin-3-ylamino)pyrimidin-4-yl)pyridazin-3-yl)oxy)naphthalen-1-yl)cyclopropanecarboxamide |
| 3 | 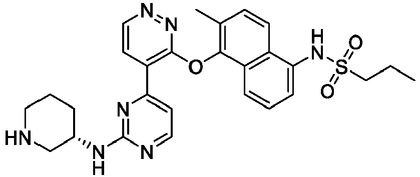 | (*S*)-*N*-(6-methyl-5-((4-(2-(piperidin-3-ylamino)pyrimidin-4-yl)pyridazin-3-yl)oxy)naphthalen-1-yl)propane-1-sulfonamide |
| 4 | 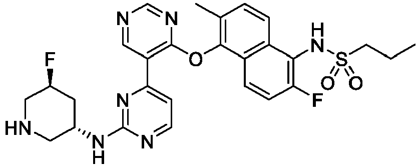 | *N*-(2-fluoro-5-((2-(((3*S*,5*S*)-5-fluoropiperidin-3-yl)amino)-[4,5'-bipyrimidin]-4'-yl)oxy)-6-methylnaphthalen-1-yl)propane-1-sulfonamide |
| 5 | 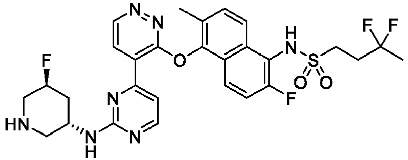 | 3,3-difluoro-*N*-[2-fluoro-6-methyl-5-[4-[2-[[-(3*S*,5*S*)-5-fluoro-3-piperidyl]amino] pyrimidin-4-yl]pyridazin-3-yl]oxy-1-naphthyl]butane-1-sulfonamide |
| 6 | 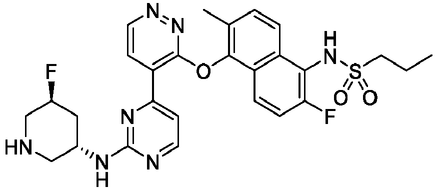 | *N*-(2-fluoro-5-((4-(2-(((3*S*,5*S*)-5-fluoropiperidin-3-yl)amino)pyrimidin-4-yl)pyridazin-3-yl)oxy)-6-methylnaphthalen-1-yl)propane-1-sulfonamide |
| 7 | 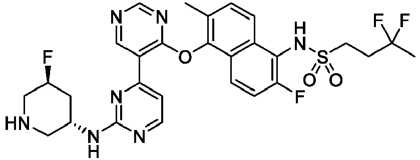 | 3,3-difluoro-*N*-(2-fluoro-5-((2-(((3*S*,5*S*)-5-fluoropiperidin-3-yl)amino)-[4,5'-bipyrimidin]-4'-yl)oxy)-6-methylnaphthalen-1-yl)butane-1-sulfonamide |
| 8 | 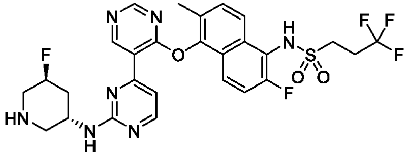 | 3,3,3-trifluoro-*N*-(2-fluoro-5-((2-(((3*S*,5*S*)-5-fluoropiperidin-3-yl)amino)-[4,5'-bipyrimidin]-4'-yl)oxy)-6-methylnaphthalen-1-yl)propane-1-sulfonamide |
| 9 | 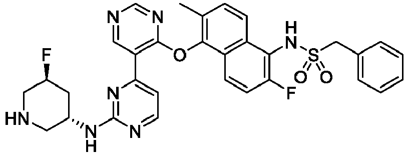 | *N*-(2-fluoro-5-((2-(((3*S*,5*S*)-5-fluoropiperidin-3-yl)amino)-[4,5'-bipyrimidin]-4'-yl)oxy)-6-methylnaphthalen-1-yl)-1-phenylmethanesulfonamide |

11. A pharmaceutical composition comprising a compound or pharmaceutically acceptable salt thereof of any one of claims 1 to 10 and one or more pharmaceutically acceptable excipients.

12. The compound or pharmaceutically acceptable salt thereof of any one of claims 1 to 10, or the pharmaceutical composition of claim 11, for use as a medicament.

13. The compound or pharmaceutically acceptable salt thereof of any one of claims 1 to 10, or the pharmaceutical composition of claim 11, for use in a method of treating cancer,
optionally wherein the cancer is:
- squamous cell carcinoma, small-cell lung cancer, non-small cell lung cancer (NSCLC), lung adenocarcinoma, squamous cell lung cancer, peritoneum cancer, hepatocellular cancer, stomach cancer, gastrointestinal cancer, esophageal cancer, pancreatic cancer, glioblastoma, cervical cancer, ovarian cancer, liver cancer, bladder cancer, breast cancer, colon cancer, rectal cancer, colorectal cancer, endometrial cancer, uterine cancer, salivary gland carcinoma, renal cancer, prostate cancer, vulval cancer, thyroid cancer, hepatocellular carcinoma (HCC), anal carcinoma, penile carcinoma, or head and neck cancer; or
- lymphoma, lymphocytic leukemia, multiple myeloma (MM), acute myelogenous leukemia (AML), chronic myelogenous leukemia (CML), myelodysplastic syndrome (MDS), or myeloproliferative disease (MPD); or
- multiple myeloma; or
- breast cancer; or
- breast cancer that is triple-negative breast cancer (TNBC); or
- an Ire1-mediated cancer, optionally further wherein the cancer is **characterized by** an increased expression of Ire1.

14. The compound, pharmaceutically acceptable salt, or pharmaceutical composition for use of claim 13, the method further comprising administering radiotherapy and/or one or more additional anti-cancer therapies in combination with the compound or pharmaceutically acceptable salt thereof or pharmaceutical composition,
optionally wherein:
- the anti-cancer therapy is administered before said compound or pharmaceutical composition; or
- the anti-cancer therapy is administered after said compound or pharmaceutical composition; or
- the anti-cancer therapy is administered concurrently with said compound or pharmaceutical composition.

15. The compound, pharmaceutically acceptable salt, or pharmaceutical composition for use of claim 14, wherein the anti-cancer therapy comprises a corticosteroid, a proteasome inhibitor, an immunomodulatory agent, an anti-CD38 antibody, an anti-VEGF-A antibody, an anti-PD-1 antibody, an anti-PD-L1 antibody, or an anti-interleukin-6 antibody, or a combination thereof,
optionally wherein:
- the corticosteroid comprises dexamethasone; or
- the proteasome inhibitor comprises carfilzomib, ixazomib or bortezomib; or
- the immunomodulatory agent comprises lenalidomide or pomalidomide; or
- the anti-PD-L1 antibody comprises, avelumab, durvalumab, or atezolizumab; or
- the anti-PD-1 antibody comprises pembrolizumab or nivolumab.

## Patentansprüche

1. Verbindung mit Formel (I) oder Formel (II); 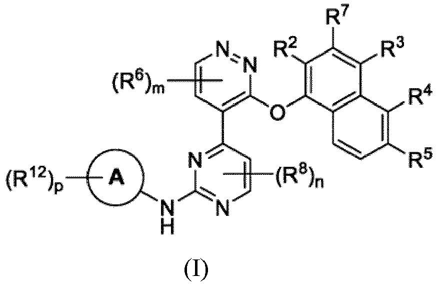 oder 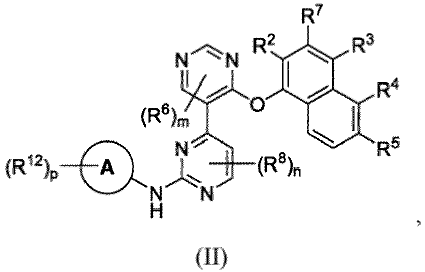
oder ein pharmazeutisch annehmbares Salz davon,
wobei;
R¹ Wasserstoff, Halogen, -CN, -NO₂, -NR^{a}R^{b}, -NR^{a}C(O)R^{b}, -NR^{a}C(O)R^{b}, - NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a}, - OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b}, - P(O)R^{a}R^{b}, R¹⁰-substituiertes oder -unsubstituiertes C₁₋₆-Alkyl, R¹⁰-substituiertes oder - unsubstituiertes 2- bis 6-gliedriges Heteroalkyl, R¹⁰-substituiertes oder -unsubstituiertes C₁₋₆-Haloalkyl, R¹⁰-substituiertes oder -unsubstituiertes C₃-C₇-Cycloalkyl, R¹⁰-substituiertes oder - unsubstituiertes 3- bis 7-gliedriges Heterocycloalkyl, R¹⁰-substituiertes oder -unsubstituiertes C₅₋₆-Aryl oder R¹⁰-substituiertes oder -unsubstituiertes 5- oder 6-gliedriges Heterocycloalkyl ist;
R² Wasserstoff, Halogen, -CN, R¹⁰-substituiertes oder -unsubstituiertes C₁₋₆-Alkoxy oder R¹⁰-substituiertes oder -unsubstituiertes C₁₋₆-Alkyl ist;
R³ -L¹-R¹, Wasserstoff, Halogen, -CN, -NO₂, -NR^{a}R^{b}, -NR^{a}C(O)R^{b}, -NR^{a}C(O)R^{b}, -NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a}, - OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b}, - P(O)R^{a}R^{b}, R¹⁰-substituiertes oder -unsubstituiertes C₁₋₆-Alkoxy, R¹⁰-substituiertes oder - unsubstituiertes C₁₋₆-Haloalkyl, R¹⁰-substituiertes oder -unsubstituiertes C₁₋₆-Alkyl, R¹⁰-substituiertes oder -unsubstituiertes C₃₋₇-Cycloalkyl, R¹⁰-substituiertes oder -unsubstituiertes 3- bis 7-gliedriges Heterocycloalkyl, R¹⁰-substituiertes oder -unsubstituiertes C₅₋₆-Aryl oder R¹⁰-substituiertes oder -unsubstituiertes 5- oder 6-gliedriges Heteroaryl ist;
R⁴ -L²-R⁹-, Halogen, -CN, -NO₂, -NR^{a}R^{b}, -NR^{a}C(O)R^{b}, -NR^{a}C(O)R^{b}, -NR^{a}C(O)OR^{b}, - NR^{a}C(O)NR^{b}, -S(O)₂NR^{a}, -C(O)R^{a}, -C(O)OR^{a}, -C(O)NR^{a}R^{b}, -OR^{a}, -OC(O)R^{a}, - OC(O)NR^{a}R^{b}, -SR^{a}, -S(O)R^{a}, -S(O)₂R^{a}, -S(O)₃R^{a}, -S(O)(=NH)R^{a}, -S(O)₂NR^{a}R^{b}, -P(O)R^{a}R^{b}, R¹⁰-substituiertes oder -unsubstituiertes C₁₋₆-Alkoxy, R¹⁰-substituiertes oder -unsubstituiertes C₁₋₆-Haloalkyl, R¹⁰-substituiertes oder -unsubstituiertes C₁₋₆-Alkyl, R¹⁰-substituiertes oder - unsubstituiertes 2- bis 6-gliedriges Heteroalkyl, R¹⁰-substituiertes oder -unsubstituiertes C₃₋₇-Cycloalkyl, R¹⁰-substituiertes oder -unsubstituiertes 3- bis 7-gliedriges Heterocycloalkyl, R¹⁰-substituiertes oder -unsubstituiertes C₅₋₆-Aryl oder R¹⁰-substituiertes oder -unsubstituiertes 5- oder 6-gliedriges Heteroaryl ist;
R⁵ Wasserstoff, Halogen oder R¹⁰-substituiertes oder -unsubstituiertes C₁₋₃-Alkyl ist;
jedes R⁶ unabhängig Wasserstoff, Halogen, -CN, -NO₂, -OR^{a}, -NR^{a}R^{b}, -C(O)R^{a}, - C(O)OR^{a}, -SR^{a}, -S(O)₂R^{a}, -P(O)R^{a}R^{b}, R¹⁰-substituiertes oder -unsubstituiertes C₁₋₆-Alkoxy, R¹⁰-substituiertes oder -unsubstituiertes C₁₋₆-Haloalkyl, R¹⁰-substituiertes oder - unsubstituiertes C₁₋₆-Alkyl, R¹⁰-substituiertes oder -unsubstituiertes C₃₋₆-Cycloalkyl, -SO₂(C₁₋₆-Alkyl) oder -SO₂(C₁₋₆-Haloalkyl) ist;
R⁷ Wasserstoff, Halogen, -CN, R¹⁰-substituiertes oder -unsubstituiertes C₁₋₆-Alkyl, R¹⁰-substituiertes oder -unsubstituiertes C₁₋₆-Alkoxy, R¹⁰-substituiertes oder -unsubstituiertes C₁₋₆-Haloalkyl oder R¹⁰-substituiertes oder -unsubstituiertes C₃₋₆-Cycloalkyl ist;
jedes R⁸ unabhängig Wasserstoff, Halogen oder R¹⁰-substituiertes oder - unsubstituiertes C₁₋₃-Alkyl ist;
R⁹ Wasserstoff, Halogen, R¹⁰-substituiertes oder -unsubstituiertes C₁₋₆-Alkyl, R¹⁰-substituiertes oder -unsubstituiertes 2-6-gliedriges Heteroalkyl, R¹⁰-substituiertes oder - unsubstituiertes C₁₋₆-Haloalkyl, R¹⁰-substituiertes oder -unsubstituiertes C₃-C₇-Cycloalkyl, R¹⁰-substituiertes oder -unsubstituiertes 3-7-gliedriges Heterocycloalkyl, R¹⁰-substituiertes oder -unsubstituiertes C₅₋₆-Aryl oder R¹⁰-substituiertes oder -unsubstituiertes 5- oder 6-gliedriges Heterocycloalkyl ist;
jedes R¹⁰ unabhängig Halogen, -N₃, -CF₃, -CN, -NO₂, -NR^{a}R^{b}, -NR^{a}C(O)R^{b}, - NR^{a}C(O)R^{b}, -NR^{a}C(O)OR^{b}, -NR^{a}C(O)NR^{b}, -S(O)₂NR^{a}, -C(O)R^{a}, -C(O)OR^{a}, -C(O)NR^{a}R^{b}, - OR^{a}, -OC(O)R^{a}, -OC(O)NR^{a}R^{b}, -SR^{a}, -S(O)R^{a}, -S(O)₂R^{a}, -S(O)₃R^{a}, -S(O)(=NH)R^{a}, - S(O)₂NR^{a}R^{b}, -P(O)R^{a}R^{b}, R¹¹-substituiertes oder -unsubstituiertes C₁₋₆-Alkyl, R¹¹-substituiertes oder -unsubstituiertes 2- bis 6-gliedriges Heteroalkyl, R¹¹-substituiertes oder - unsubstituiertes C₃₋₇-Cycloalkyl, R¹¹-substituiertes oder -unsubstituiertes 3- bis 7-gliedriges Heterocycloalkyl, R¹¹-substituiertes oder -unsubstituiertes C₅₋₆-Aryl oder R¹¹-substituiertes oder -unsubstituiertes 5- bis 6-gliedriges Heteroaryl ist;
jedes R¹¹ unabhängig Halogen, -N₃, -CF₃, -CCl₃, -CBr₃, -CI₃, -CN, -CHO, -OH, -NH₂, -COOH, -CONH₂, -NO₂, -SH, -SO₂Cl, -SO₃H, -SO₄H, -SO₂NH₂, unsubstituiertes C₁₋₆-Alkyl, unsubstituiertes 2- bis 6-gliedriges Heteroalkyl, unsubstituiertes C₃₋₇-Cycloalkyl, unsubstituiertes 3- bis 7-gliedriges Heterocycloalkyl, unsubstituiertes C₅₋₆-Aryl oder unsubstituiertes 5- bis 6-gliedriges Heteroaryl ist;
jedes R¹² unabhängig Wasserstoff, Halogen, ein NPG oder R¹⁰-substituiertes oder - unsubstituiertes C₁₋₃-Alkyl ist;
L¹ -NHSO₂-, -NHC(O)-, NHCO(O)-, -NHC(O)NH-, -NH-, -O-, -S- oder -SO₂- ist;
L² -NHSO₂-, -NHC(O)-, NHCO(O)-, -NHC(O)NH-, -NH-, -O-, -S- oder -SO₂- ist;
jedes R^{a} und R^{b} unabhängig Wasserstoff, unsubstituiertes C₁₋₆-Alkyl, unsubstituiertes C₁₋₆-Haloalkyl, unsubstituiertes C₁₋₆-Alkoxy, unsubstituiertes C₂₋₆-Alkenyl, unsubstituiertes C₂₋₆-Alkynyl, unsubstituiertes C₃₋₇-Cycloalkyl, unsubstituiertes 3- bis 7-gliedriges Heterocycloalkyl, unsubstituiertes C₅₋₆-Aryl oder unsubstituiertes 5- oder 6-gliedriges Heteroaryl ist;
m 1 oder 2 ist;
n 1 oder 2 ist;
p 1, 2, 3, 4, 5 oder 6 ist; und
Ring A C₃₋₆-Cycloalkyl, 3- bis 6-gliedriges Heterocycloalkyl, C₅₋₆-Aryl, 5- oder 6-gliedriges Heteroaryl ist.

2. Verbindung nach Anspruch 1, wobei:
(i) Ring A 6-gliedriges Heterocycloalkyl ist; oder
(ii) Ring A Piperidinyl ist.

3. Verbindung nach Anspruch 2, wobei R¹² Halogen oder C₁₋₃-Alkyl ist, und wobei optional:
- n p ist, 1 oder 2 ist; oder
- R¹² -F ist; oder
- n p ist, 1 oder 2 ist, und R¹² -F ist; oder
- p 2 ist und R¹² -F und Methyl ist; oder
- Ring A die folgende Struktur aufweist:

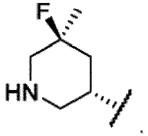

4. Verbindung nach einem der Ansprüche 1 bis 3, wobei:
- R⁶ Wasserstoff ist; oder
- R⁸ Wasserstoff ist; oder
- R⁶ Wasserstoff ist und R⁸ Wasserstoff ist; oder
- R³ Wasserstoff ist; oder
- R⁶ Wasserstoff ist und R³ Wasserstoff ist; oder
- R⁸ Wasserstoff ist und R³ Wasserstoff ist; oder
- R⁶ Wasserstoff ist und R⁸ Wasserstoff ist und R³ Wasserstoff ist.

5. Verbindung nach einem der Ansprüche 1 bis 4, wobei: R² unsubstituiertes C₁₋₃-Alkyl ist; oder R² Methyl ist.

6. Verbindung nach einem der Ansprüche 1 bis 5, wobei R⁴ -L²-R⁹- ist, und wobei optional:
(i) R⁹ R¹⁰-substituiertes oder -unsubstituiertes Phenyl ist, wobei optional ferner L² - NHSO₂- oder -NHC(O)- ist; oder
(ii) R⁹ R¹⁰-substituiertes Phenyl ist und R¹⁰ Halogen ist, wobei optional ferner L² - NHSO₂- oder -NHC(O)- ist; oder
(iii) R⁹ R¹⁰-substituiertes oder -unsubstituiertes C₃₋₆-Cycloalkyl ist, wobei optional ferner L² -NHSO₂- oder -NHC(O)- ist; oder
(iv) R⁹ unsubstituiertes Cyclopropanyl ist, wobei optional ferner L² -NHSO₂- oder - NHC(O)- ist; oder
(v) R⁹ 2- bis 6-gliedriges unsubstituiertes Haloalkyl ist, wobei optional ferner L² - NHSO₂- oder -NHC(O)- ist; oder
(vi) R⁹ 3,3-Difluorbutyl ist, wobei optional ferner L² -NHSO₂- oder -NHC(O)- ist; oder
(vii) R⁹ R¹⁰-substituiertes oder -unsubstituiertes C₁₋₃-Alkyl ist, wobei optional ferner L² -NHSO₂- oder -NHC(O)- ist; oder
(viii) R⁹ unsubstituiertes C₁₋₃-Alkyl ist, wobei optional ferner L² -NHSO₂- oder - NHC(O)- ist.

7. Verbindung nach einem der Ansprüche 1 bis 6, wobei: R⁵ Halogen ist; oder R⁵ Wasserstoff ist; oder R⁵ unsubstituiertes C₁₋₃-Alkyl ist.

8. Verbindung nach Anspruch 1 mit:
(i) Formel (I) oder einem pharmazeutisch annehmbaren Salz davon; oder
(ii) Formel: 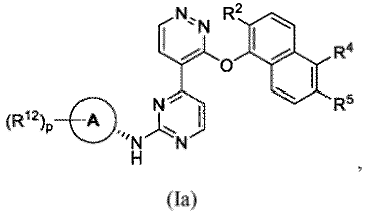 oder einem pharmazeutisch annehmbaren Salz davon; oder
(iii) Formel: 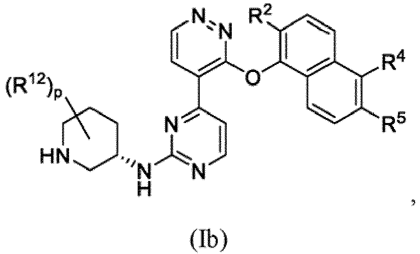 oder einem pharmazeutisch annehmbaren Salz davon; oder
(iv) Formel: 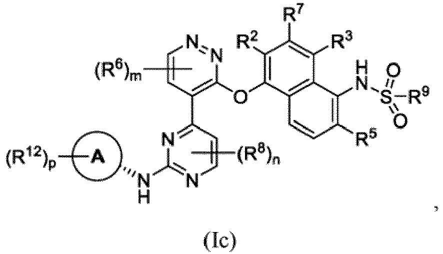 oder einem pharmazeutisch annehmbaren Salz davon; oder
(v) Formel: 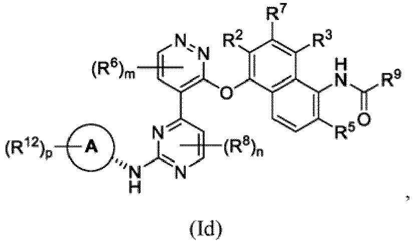 oder einem pharmazeutisch annehmbaren Salz davon; oder
(vi) Formel: 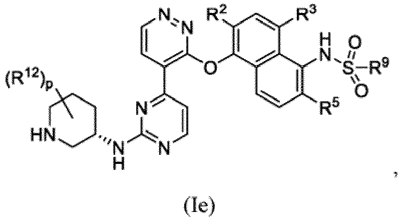 oder einem pharmazeutisch annehmbaren Salz davon; oder
(vii) Formel: 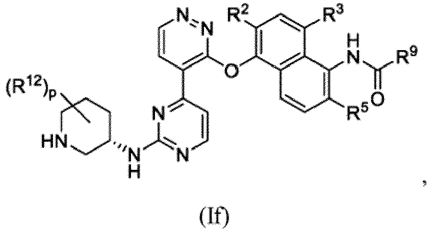 oder einem pharmazeutisch annehmbaren Salz davon.

9. Verbindung nach Anspruch 1 mit:
(i) Formel (II) oder einem pharmazeutisch annehmbaren Salz davon; oder
(ii) Formel: 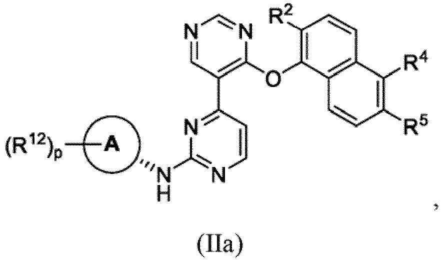 oder einem pharmazeutisch annehmbaren Salz davon; oder
(iii) Formel: 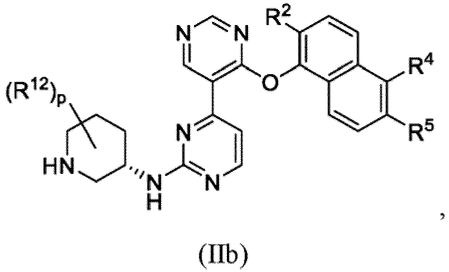 oder einem pharmazeutisch annehmbaren Salz davon; oder
(iv) Formel: 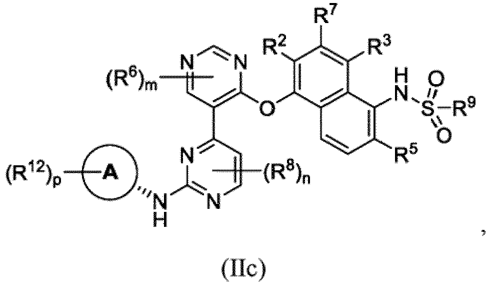 oder einem pharmazeutisch annehmbaren Salz davon; oder
(v) Formel: 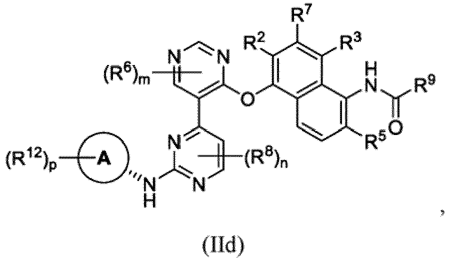 oder einem pharmazeutisch annehmbaren Salz davon; oder
(vi) Formel: 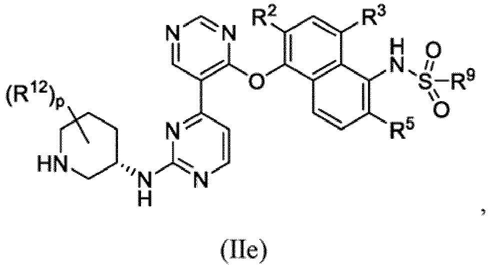 oder einem pharmazeutisch annehmbaren Salz davon; oder
(vii) Formel: 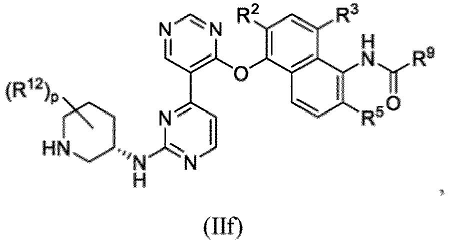 oder einem pharmazeutisch annehmbaren Salz davon.

10. Verbindung oder pharmazeutisch annehmbares Salz davon nach Anspruch 1, wobei die Verbindung die folgende Verbindung Nr. 1, Nr. 2, Nr. 3, Nr. 4, Nr. 5, Nr. 6, Nr. 7, Nr. 8 oder Nr. 9 umfasst:
| Nr. | Struktur | Name |
|---|---|---|
| 1 | 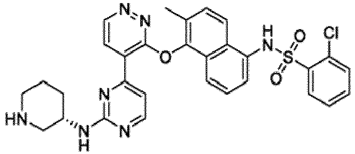 | (*S*)-2-Chlor-*N*-(6-methyl-5-((4-(2-(piperidin-3-ylamino)pyrimidin-4-yl)pyridazin-3-yl)oxy)naphthalen-1-yl)benzolsulfonamid |
| 2 | 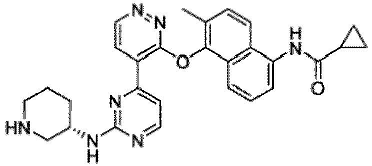 | (*S*)-*N*-(6-Methyl-5-((4-(2-(piperidin-3-ylamino)pyrimidin-4-yl)pyridazin-3-yl)oxy)naphthalen-1 - yl)cyclopropancarboxamid |
| 3 | 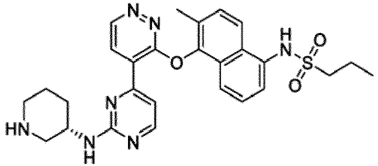 | (*S*)-*N*-(6-Methyl-5-((4-(2-(piperidin-3-ylamino)pyrimidin-4-yl)pyridazin-3-yl)oxy)naphthalen-1-yl)propan-1-sulfonamid |
| 4 | 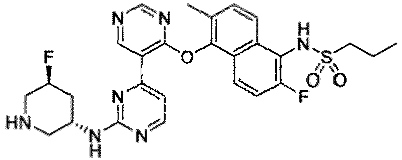 | *N*-(2-Fluor-5-((2-(((3*S*,5*S*)-5-fluorpiperidin-3-yl)amino)-[4,5'-bipyrimidin]-4'-yl)oxy)-6-methylnaphthalen-1-yl)propan-1-sulfonamid |
| 5 | 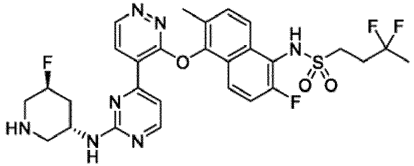 | 3,3-Difluor-*N*-[2-fluor-6-methyl-5-[4-[2-[[-(3*S*,5*S*-fluor-3-piperidyl]amino]pyrimidin-4-yl]pyridazin-3-yl]oxy-1-naphthyl]butan-1-sulfonamid |
| 6 | 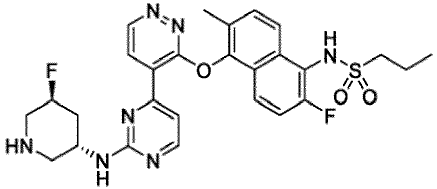 | *N*-(2-Fluor-5-((4-(2-(((3*S*,5*S*)-5-fluorpiperidin-3-yl)amino)pyrimidin-4-yl)pyridazin-3-yl)oxy)-6-methylnaphthalen-1-yl)propan-1-sulfonamid |
| 7 | 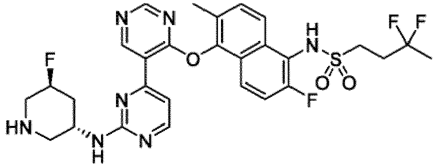 | 3,3-Difluoro-*N*-(2-fluor-5-((2-(((3*S*,5*S*)-5-fluorpiperidin-3-yl)amino)-[4,5'-bipyrimidin]-4'-yl)oxy)-6-methylnaphthalen-1-yl)butan-1-sulfonamid |
| 8 | 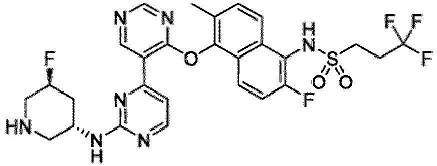 | 3,3,3-Trifluor-*N*-(2-fluor-5-((2-(((3*S*,5*S*)-5-fluorpiperidin-3-yl)amino)-[4,5'-bipyrimidn]-4'-yl)oxy)-6-methylnaphthalen-1-yl)propan-1-sulfonamid |
| 9 | 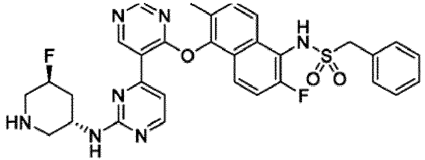 | *N*-(2-Fluor-5-((2-(((3*S*,5*S*)-5-fluorpiperidin-3-yl)amino)-[4,5'-bipyrimidin]-4'-yl)oxy)-6-methylnaphthalen-1-yl)-1-phenylmethansulfonamid |

11. Pharmazeutische Zusammensetzung, umfassend eine Verbindung oder ein pharmazeutisch annehmbares Salz davon nach einem der Ansprüche 1 bis 10 und einen oder mehrere pharmazeutisch annehmbare Hilfsstoffe.

12. Verbindung oder pharmazeutisch annehmbares Salz davon nach einem der Ansprüche 1 bis 10 oder pharmazeutische Zusammensetzung nach Anspruch 11 zur Verwendung als ein Medikament.

13. Verbindung oder pharmazeutisch annehmbares Salz davon nach einem der Ansprüche 1 bis 10 oder pharmazeutische Zusammensetzung nach Anspruch 11 zur Verwendung in einem Verfahren des Behandelns von Krebs,
wobei optional der Krebs Folgendes ist:
- Plattenepithelkarzinom, kleinzelliger Lungenkrebs, nicht-kleinzelligem Lungenkrebs (NSCLC), Lungenadenokarzinom, Plattenepithel-Lungenkrebs, Bauchfellkarzinom, Leberzellkrebs, Magenkrebs, gastrointestinaler Krebs, Speiseröhrenkrebs, Bauchspeicheldrüsenkrebs, Glioblastom, Gebärmutterhalskrebs, Eierstockkrebs, Leberkrebs, Blasenkrebs, Brustkrebs, Darmkrebs, Rektumkarzinom, Dickdarmkrebs, Endometriumkarzinom, Gebärmutterkrebs, Speicheldrüsenkarzinom, Nierenkrebs, Prostatakrebs, Vulvakrebs, Schilddrüsenkrebs, hepatozelluläres Karzinom (HCC), Analkarzinom, Peniskarzinom oder Hals- und Kopfkrebs; oder
- Lymphom, lymphozytische Leukämie, multiplem Myelom (MM), akute myeloische Leukämie (AML), chronische myeloische Leukämie (CML), myelodysplastisches Syndrom (MDS) oder myeloproliferative Erkrankung (MPD); oder
- multiples Myelom; oder
- Brustkrebs; oder
- Brustkrebs, der dreifach-negativer Brustkrebs (TNBC) ist; oder
- ein Ire1-vermittelter Krebs, wobei optional ferner der Krebs **gekennzeichnet ist durch** eine erhöhte Expression von Ire1.

14. Verbindung, pharmazeutisch annehmbares Salz oder pharmazeutische Zusammensetzung zur Verwendung nach Anspruch 13, wobei das Verfahren ferner das Verabreichen von Strahlentherapie und/oder einer oder mehrerer zusätzlicher Krebstherapien in Kombination mit der Verbindung oder dem pharmazeutisch annehmbaren Salz davon oder der pharmazeutischen Zusammensetzung umfasst,
wobei optional:
- die Krebstherapie vor der Verbindung oder pharmazeutischen Zusammensetzung verabreicht wird; oder
- die Krebstherapie nach der Verbindung oder pharmazeutischen Zusammensetzung verabreicht wird; oder
- die Krebstherapie gleichzeitig mit der Verbindung oder pharmazeutischen Zusammensetzung verabreicht wird.

15. Verbindung, pharmazeutisch annehmbares Salz oder pharmazeutische Zusammensetzung zur Verwendung nach Anspruch 14, wobei die Krebstherapie ein Corticosteroid, ein Proteasom-Inhibitor, ein Immunmodulator, ein anti-CD38-Antikörper, ein anti-VEGF-A-Antikörper, ein anti-PD-1-Antikörper, ein anti-PD-L1-Antikörper oder ein anti-Interleukin-6-Antikörper oder eine Kombination davon umfasst,
wobei optional:
- das Corticosteroid Dexamethason umfasst; oder
- der Proteasom-Inhibitor Carfilzomib, Ixazomib oder Bortezomib umfasst; oder
- der Immunmodulator Lenalidomid oder Pomalidomid umfasst; oder
- der anti-PD-L1-Antikörper Avelumab, Durvalumab oder Atezolizumab umfasst; oder
- der anti-PD-1-Antikörper Pembrolizumab oder Nivolumab umfasst.

## Revendications

1. Composé ayant la formule (I) ou la formule (II) : 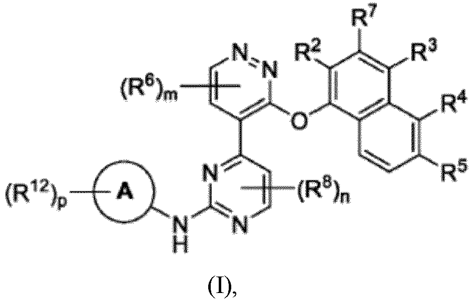 ou 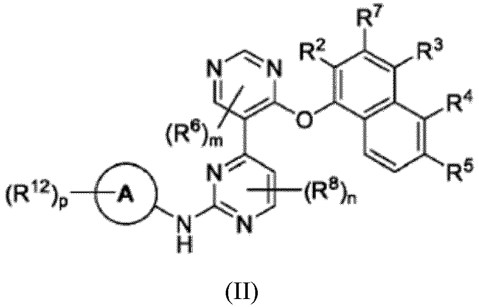
ou sel pharmaceutiquement acceptable de celui-ci, dans lequel ;
R¹ est hydrogène, halogène, -CN, -NO₂, -NR^{a}R^{b}, -NR^{a}C(O)R^{b}, -NR^{a}C(O)R^{b}, -NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a}, -OC(O)R^{a}, -OC(O)NR^{a}R^{b}, -SR^{a}, -S(O)R^{a}, -S(O)2R^{a}, -S(O)3R^{a}, -S(O)(=NH)R^{a}, -S(O)₂NR^{a}R^{b}, -P(O)R^{a}R^{b}, alkyle C₁₋₆ substitué ou non substitué en R¹⁰, hétéroalkyle, à 2 à 6 membres, substitué ou non substitué en R¹⁰, haloalkyle C₁₋₆ substitué ou non substitué en R¹⁰, cycloalkyle C₃-C₇ substitué ou non substitué en R¹⁰, hétérocycloalkyle, à 3 à 7 membres, substitué ou non substitué en R¹⁰, aryle C₅₋₆ substitué ou non substitué en R¹⁰, ou hétérocycloalkyle, à 5 ou 6 membres, substitué ou non substitué en R¹⁰ ;
R² est hydrogène, halogène, -CN, alkoxy C₁₋₆ substitué ou non substitué en R¹⁰, ou alkyle C₁₋₆ substitué ou non substitué en R¹⁰ ;
R³ est -L¹-R¹, hydrogène, halogène -CN, -NO₂, -NR^{a}R^{b}, -NR^{a}C(O)R^{b}, -NR^{a}C(O)R^{b}, -NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a}, -OC(O)R^{a}, -OC(O)NR^{a}R^{b}, -SR^{a}, -S(O)R^{a}, -S(O)2R^{a}, -S(O)3R^{a}, -S(O)(=NH)R^{a}, -S(O)₂NR^{a}R^{b}, -P(O)R^{a}R^{b}, alkoxy C₁₋₆ substitué ou non substitué en R¹⁰, haloalkyle C₁₋₆ substitué ou non substitué en R¹⁰, alkyle C₁₋₆ substitué ou non substitué en R¹⁰, cycloalkyle C₃₋₇ substitué ou non substitué en R¹⁰, hétérocycloalkyle, à 3 à 7 membres, substitué ou non substitué en R¹⁰, aryle C₅₋₆ substitué ou non substitué R¹⁰, ou hétéroaryle, à 5 ou 6 membres, substitué ou non substitué en R¹⁰ ;
R⁴ est -L²-R⁹-, halogène, -CN -NO₂, -NR^{a}R^{b}, -NR^{a}C(O)R^{b}, -NR^{a}C(O)R^{b}, -NR^{a}C(O)OR^{b}, -NR^{a}C(O)NR^{b}, -S(O)₂NR^{a}, -C(O)R^{a}, -C(O)OR^{a}, -C(O)NR^{a}R^{b}, -OR^{a}, -OC(O)R^{a}, -OC(O)NR^{a}R^{b}, -SR^{a}, -S(O)R^{a}, -S(O)2R^{a}, -S(O)3R^{a}, -S(O)=NH)R^{a}, -S(O)₂NR^{a}R^{b}, -P(O)R^{a}R^{b}, alkoxy C₁₋₆ substitué ou non substitué en R¹⁰, haloalkyle C₁₋₆ substitué ou non substitué en R¹⁰, alkyle C₁₋₆ substitué ou non substitué en R¹⁰, hétéroalkyle, à 2 à 6 membres, substitué ou non substitué en R¹⁰, cycloalkyle C₃₋₇ substitué ou non substitué en R¹⁰, hétérocycloalkyle, à 3 à 7 membres, substitué ou non substitué en R¹⁰, aryle C₅₋₆ substitué ou non substitué en R¹⁰, ou substitué ou non substitué en R¹⁰ hétéroaryle à 5 ou 6 membres ;
R⁵ est hydrogène, halogène, ou alkyle C₁₋₃ substitué ou non substitué en R¹⁰ ;
chaque R⁶ est indépendamment hydrogène, halogène, -CN, -NO₂, -OR^{a}, -NR^{a}R^{b}, -C(O)R^{a}, -C(O)OR^{a}, -SR^{a}, -S(O)2R^{a}, -P(O)R^{a}R^{b}, alkoxy C₁₋₆ substitué ou non substitué en R¹⁰, haloalkyle C₁₋₆ substitué ou non substitué en R¹⁰, alkyle C₁₋₆ substitué ou non substitué en R¹⁰, cycloalkyle C₃₋₆ substitué ou non substitué en R¹⁰, -SO₂(alkyle C₁₋₆), ou -SO₂(haloalkyle C₁₋₆) ;
R⁷ est hydrogène, halogène, -CN, alkyle C₁₋₆ substitué ou non substitué en R¹⁰, C₁₋₆ alkoxy substitué ou non substitué en R¹⁰, haloalkyle C₁₋₆ substitué ou non substitué en R¹⁰, cycloalkyle C₃₋₆ ou substitué ou non substitué en R¹⁰ ;
chaque R⁸ est indépendamment hydrogène, halogène, ou alkyle C₁₋₃ substitué ou non substitué en R¹⁰ ;
R⁹ est hydrogène, halogène, alkyle C₁₋₆ substitué ou non substitué en R¹⁰, substitué ou non substitué en R¹⁰ hétéroalkyle à 2 à 6 membres, haloalkyle C₁₋₆ substitué ou non substitué en R¹⁰, cycloalkyle C₃-C₇ substitué ou non substitué en R¹⁰, substitué ou non substitué en R¹⁰ hétérocycloalkyle à 3 à 7 membres, aryle C₅₋₆ substitué ou non substitué en R¹⁰, ou hétérocycloalkyle, à 5 ou 6 membres, substitué ou non substitué en R¹⁰ ;
chaque R¹⁰ est indépendamment halogène, -N₃, -CF₃, -CN, -NO₂, -NR^{a}R^{b}, -NR^{a}C(O)R^{b}, -NR^{a}C(O)R^{b}, -NR^{a}C(O)OR^{b}, -NR^{a}C(O)NR^{b}, -S(O)₂NR^{a}, -C(O)R^{a},
-C(O)OR^{a}, -C(O)NR^{a}R^{b}, -OR^{a}, -OC(O)R^{a}, -OC(O)NR^{a}R^{b}, -SR^{a}, -S(O)R^{a}, -S(O)2R^{a}, -S(O)3R^{a}, -S(O)(=NH)R^{a}, -S(O)₂NR^{a}R^{b}, -P(O)R^{a}R^{b}, alkyle C₁₋₆ substitué ou non substitué en R¹¹, hétéroalkyle à 2 à 6 membres substitué ou non substitué en R¹¹, cycloalkyle C₃₋₇ substitué ou non substitué en R¹¹, hétérocycloalkyle, à 3 à 7 membres, substitué ou non substitué en R¹¹, aryle C₅₋₆ substitué ou non substitué en R¹¹, ou hétéroaryle, à 5 à 6 membres, substitué ou non substitué en R¹¹ ;
chaque R¹¹ est indépendamment halogène, -N₃, -CF₃, -CCl₃, -CBr₃, -CI₃, -CN, -CHO, -OH, -NH₂, -COOH, -CONH₂, -NO₂, -SH, -SO₂Cl, -SO₃H, -SO₄H, -SO₂NH₂, C₁₋₆ alkyle non substitué, hétéroalkyle, à 2 à 6 membres, non substitué, cycloalkyle C₃₋₇ non substitué, hétérocycloalkyle, à 3 à 7 membres, non substitué, aryle C₅₋₆ non substitué, ou hétéroaryle, à 5 à 6 membres, non substitué ;
chaque R¹² est indépendamment hydrogène, halogène, un NPG, ou alkyle C₁₋₃ substitué ou non substitué en R¹⁰ ;
L¹ est -NHSO₂-, -NHC(O)-, NHCO(O)-, -NHC(O)NH-, -NH-, -O-, -S-, ou -SO₂- ;
L² est -NHSO₂-, -NHC(O)-, NHCO(O)-, -NHC(O)NH-, -NH-, -O-, -S-, ou -SO₂- ;
chaque R^{a} et R^{b} est indépendamment hydrogène, alkyle C₁₋₆ non substitué, haloalkyle C₁₋₆ non substitué, alkoxy C₁₋₆ non substitué, alcényle C₂₋₆ non substitué, alkynyle C₂₋₆ non substitué, cycloalkyle C₃₋₇ non substitué, hétérocycloalkyle, à 3 à 7 membres, non substitué, aryle C₅₋₆ non substitué, ou hétéroaryle, à 5 ou 6 membres, non substitué ;
m est 1 ou 2 ;
n est 1 ou 2 ;
p est 1, 2, 3, 4, 5 ou 6 ; et
anneau A est cycloalkyle C₃₋₆, hétérocycloalkyle à 3 à 6 membres, aryle C₅₋₆, ou hétéroaryle à 5 ou 6 membres.

2. Composé selon la revendication 1, dans lequel :
(i) anneau A est hétérocycloalkyle à 6 membres ; ou
(ii) anneau A est pipéridinyle.

3. Composé selon la revendication 2, dans lequel R¹² est halogène ou alkyle C₁₋₃, et optionnellement en outre dans lequel :
- n est p est 1 ou 2 ; ou
- R¹² est -F ; ou
- n est p est 1 ou 2, et R¹² est -F ; ou
- p est 2 et R¹² est -F et méthyle ; ou
- anneau A a la structure :

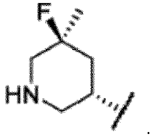

4. Composé selon l'une quelconque des revendications 1 à 3, dans lequel :
- R⁶ est hydrogène ; ou
- R⁸ est hydrogène ; ou
- R⁶ est hydrogène et R⁸ est hydrogène ; ou
- R³ est hydrogène ; ou
- R⁶ est hydrogène et R³ est hydrogène ; ou
- R⁸ est hydrogène et R³ est hydrogène ; ou
- R⁶ est hydrogène et R⁸ est hydrogène et R³ est hydrogène.

5. Composé selon l'une quelconque des revendications 1 à 4, dans lequel : R² est alkyle C₁₋₃ non substitué; ou R² est méthyle.

6. Composé selon l'une quelconque des revendications 1 à 5, dans lequel R⁴ est -L²-R⁹-, et optionnellement dans lequel :
(i) R⁹ est phényle substitué ou non substitué en R¹⁰, optionnellement en outre dans lequel L² est -NHSO₂- ou -NHC(O)- ; ou
(ii) R⁹ est phényle substitué en R¹⁰ et R¹⁰ est halogène, optionnellement en outre dans lequel L² est -NHSO₂- or -NHC(O)- ; ou
(iii) R⁹ est cycloalkyle C₃₋₆ substitué ou non substitué en R¹⁰, optionnellement en outre dans lequel L² est -NHSO₂- ou -NHC(O)- ; ou
(iv) R⁹ est cyclopropanyle non substitué, optionnellement en outre dans lequel L² est -NHSO₂- ou -NHC(O)- ; ou
(v) R⁹ est haloalkyle, à 2 à 6 membres, non substitué, optionnellement en outre dans lequel L² est -NHSO₂- ou -NHC(O)- ; ou
(vi) R⁹ est 3,3-difluorobutyle, optionnellement en outre dans lequel L² est -NHSO₂- ou -NHC(O)- ; ou
(vii) R⁹ est alkyle C₁₋₃ substitué ou non substitué en R¹⁰, optionnellement en outre dans lequel L² est -NHSO₂- ou -NHC(O)- ; ou
(viii) R⁹ est alkyle C₁₋₃ non substitué, optionnellement en outre dans lequel L² est -NHSO₂- ou -NHC(O)-.

7. Composé selon l'une quelconque des revendications 1 à 6, dans lequel : R⁵ est halogène ; ou R⁵ est hydrogène ; ou R⁵ est alkyle C₁₋₃ non substitué.

8. Composé selon la revendication 1, ayant :
(i) la formule (I) ou sel pharmaceutiquement acceptable de celui-ci ; ou
(ii) la formule : 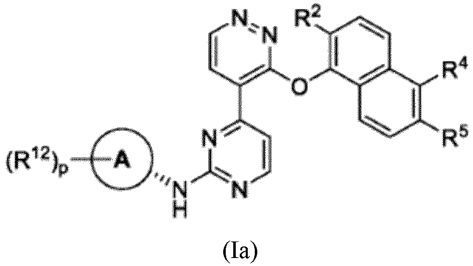 ou sel pharmaceutiquement acceptable de celui-ci ; ou
(iii) la formule : 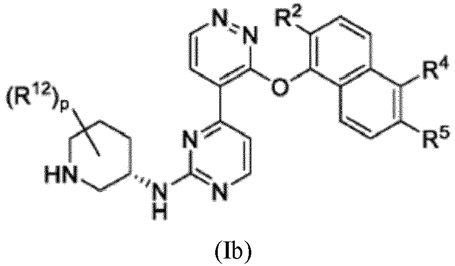 ou sel pharmaceutiquement acceptable de celui-ci ; ou
(iv) la formule : 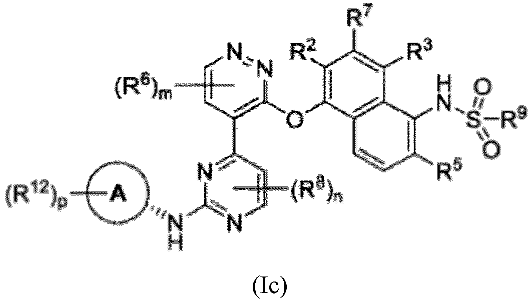 ou sel pharmaceutiquement acceptable de celui-ci ; ou
(v) la formule : 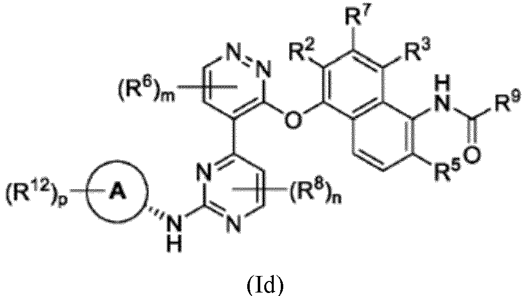 ou sel pharmaceutiquement acceptable de celui-ci ; ou
(vi) la formule : 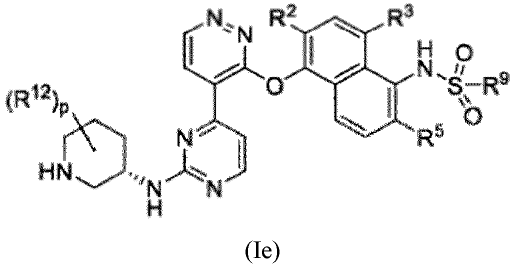 ou sel pharmaceutiquement acceptable de celui-ci ; ou
(vii) la formule : 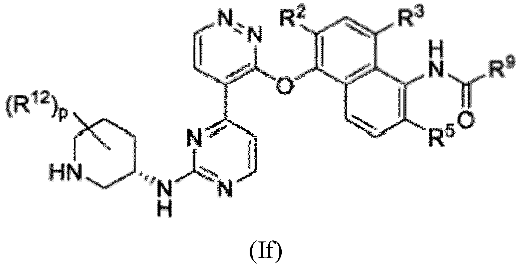 ou sel pharmaceutiquement acceptable de celui-ci.

9. Composé selon la revendication 1, ayant :
(i) la formule (II) ou sel pharmaceutiquement acceptable de celui-ci ; ou
(ii) la formule : 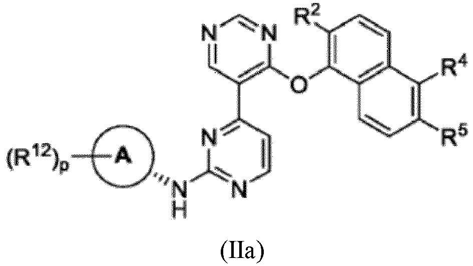 ou sel pharmaceutiquement acceptable de celui-ci ; ou
(iii) la formule : 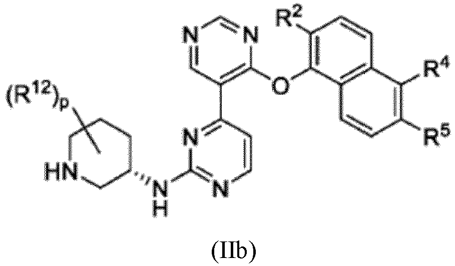 ou sel pharmaceutiquement acceptable de celui-ci ; ou
(iv) la formule : 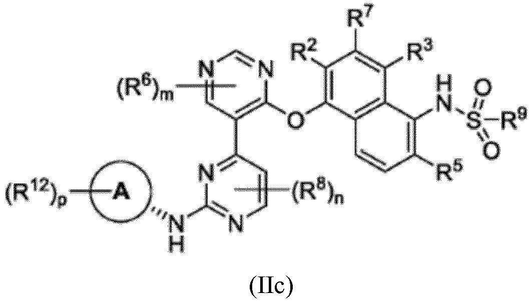 ou sel pharmaceutiquement acceptable de celui-ci ; ou
(v) la formule : 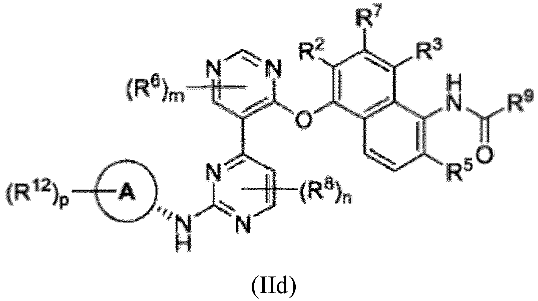 ou sel pharmaceutiquement acceptable de celui-ci ; ou
(vi) la formule : 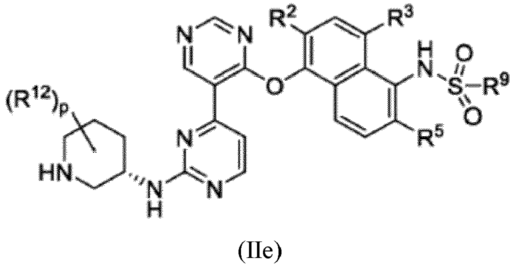 ou sel pharmaceutiquement acceptable de celui-ci ; ou
(vii) la formule : 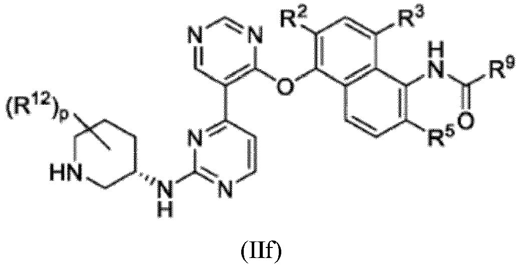 ou sel pharmaceutiquement acceptable de celui-ci.

10. Composé ou sel pharmaceutiquement acceptable de celui-ci selon la revendication 1, dans lequel
le composé comprend le composé suivant n° 1, n° 2, n° 3, n° 4, n° 5, n° 6, n° 7, n° 8, ou n° 9 :
| n° | Structure | Nom |
|---|---|---|
| 1 | 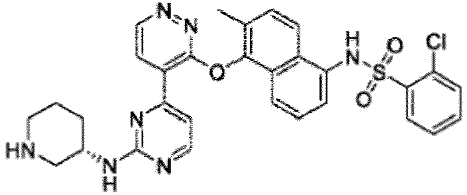 | (*S*)-2-chloro-*N*-(6-méthyl-5-((4-(2-(pipéridin-3-ylamino)pyrimidin-4-yl)pyridazin-3-yl)oxy)naphtalen-1-yl)benzènesulfonamide |
| 2 | 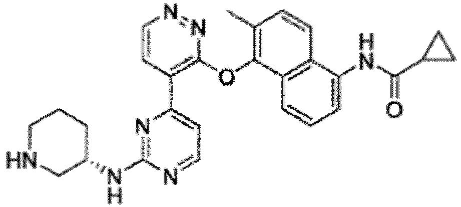 | (*S*)-*N*-(6-méthyl-5-((4-(2-(pipéridin-3-ylamino)pyrimidin-4-yl)pyridazin-3-yl)oxy)naphtalen-1-yl)cyclopropanecarboxamid e |
| 3 | 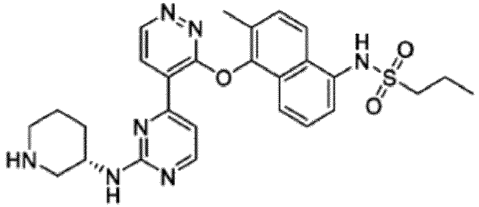 | (*S*)-*N*-(6-méthyl-5-((4-(2-(pipéridin-3-ylamino)pyrimidin-4-yl)pyridazin-3-yl)oxy)naphtalen-1-yl)propane-1-sulfonamide |
| 4 | 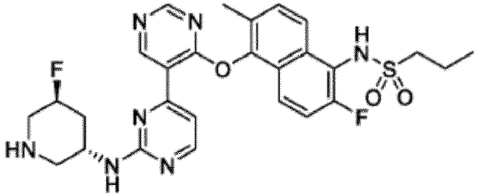 | #-(2-fluoro-5-((2-(((3S,5S)-5-fluoropipéridin-3-yl)amino)-[4,5'-bipyrimidin]-4'-yl)oxy)-6-méthylnaphthalen-1-yl)propane-1-sulfonamide |
| 5 | 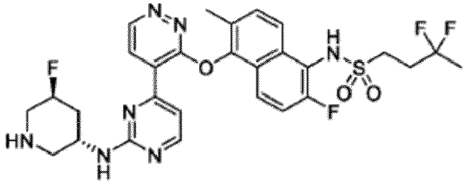 | 3,3-difluoro-*N*-[2-fluoro-6-méthyl-5-[4-[2-[[-(3S,5S)-5-fluoro-3-pipéridyl]amino]pyrimidin-4-yl]pyridazin-3-yl]oxy-1-naphtyl]butane-1-sulfonamide |
| 6 | 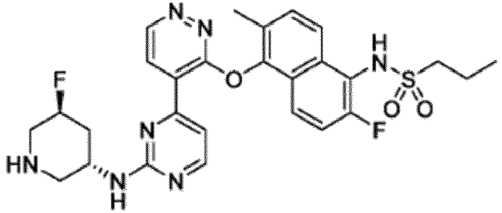 | *N*-(2-fluoro-5-((4-(2-(((3*S*,5*S*)-5-fluoropipéridin-3-yl)amino)pyrimidin-4-yl)pyridazin-3-yl)oxy)-6-méthylnaphtalen-1-yl)propane-1-sulfonamide |
| 7 | 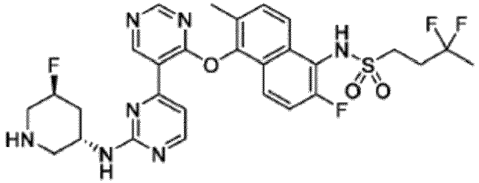 | 3,3-difluoro-N-(2-fluoro-5-((2-(((3*S*,5*S*)-5-fluoropipéridin-3-yl)amino)-[4,5'-bipyrimidin]-4'-yl)oxy)-6-méthylnaphtalen-1-yl)butane-1-sulfonamide |
| 8 | 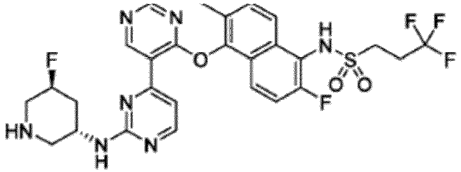 | 3,3,3-trifluoro-N-(2-fluoro-5-((2-(((3*S*,5*S*)-5-fluoropipéridin-3-yl)amino)-[4,5'-bipyrimidin]-4'-yl)oxy)-6-méthylnaphtalen-l-yl)propane-1-sulfonamide |
| 9 | 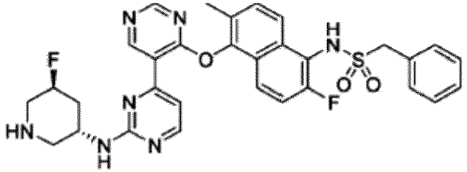 | N-(2-fluoro-5-((2-{((3*S*,5*S*)-5-fluoropipéridin-3-yl)amino)-[4,5'-bipyrimidin]-4'-yl)oxy)-6-méthylnaphtalen-1-yl)-1-phénylméthanesulfonamide |

11. Composition pharmaceutique, comprenant un composé ou un sel pharmaceutiquement acceptable de celui-ci selon l'une quelconque des revendications 1 à 10 et un ou plusieurs excipients pharmaceutiquement acceptables.

12. Composé ou sel pharmaceutiquement acceptable de celui-ci selon l'une quelconque des revendications 1 à 10, ou composition pharmaceutique selon la revendication 11, pour l'utilisation en tant que médicament.

13. Composé ou sel pharmaceutiquement acceptable de celui-ci selon l'une quelconque des revendications 1 à 10, ou composition pharmaceutique selon la revendication 11, pour l'utilisation dans un procédé de traitement du cancer,
optionnellement dans lequel le cancer est :
- carcinome à cellules squameuses, cancer du poumon à petites cellules, cancer du poumon non à petites cellules (CPNPC), adénocarcinome du poumon, cancer du poumon à cellules squameuses, cancer du péritoine, cancer hépatocellulaire, cancer de l'estomac, cancer gastrointestinal, cancer oesophagien, cancer du pancréas, glioblastome, cancer cervical, cancer ovarien, cancer du foie, cancer de la vessie, cancer du sein, cancer du côlon, cancer rectal, cancer colorectal, cancer endométrial, cancer utérin, carcinome des glandes salivaires, cancer rénal, cancer de la prostate, cancer vulvaire, cancer de la thyroïde, carcinome hépatocellulaire (CHC), carcinome anal, carcinome pénien, ou cancer de la tête et du cou ; ou
- lymphome, leucémie lymphocytique, myélome multiple (MM), leucémie myélogène aiguë (LMA), leucémie myélogène chronique (LMC), syndrome myélodysplasique (SMD), ou maladie myéloproliférative (MMP) ; ou
- myélome multiple ; ou
- cancer du sein ; ou
- le cancer du sein qui est cancer du sein triple négatif (TNBC) ; ou
- un cancer à médiation par Ire1, optionnellement en outre dans lequel le cancer est **caractérisé par** une expression accrue de Ire1.

14. Composé, sel pharmaceutiquement acceptable, ou composition pharmaceutique pour l'utilisation selon la revendication 13, le procédé comprenant en outre l'administration de radiothérapie et/ou d'une ou de plusieurs thérapies anti-cancer supplémentaires en association avec le composé ou le sel pharmaceutiquement acceptable de celui-ci ou la composition pharmaceutique,
optionnellement dans lequel :
- la thérapie anti-cancer est administrée avant ledit composé ou ladite composition pharmaceutique ; ou
- la thérapie anti-cancer est administrée après ledit composé ou ladite composition pharmaceutique ; ou
- la thérapie anti-cancer est administrée simultanément audit composé ou à ladite composition pharmaceutique.

15. Composé, sel pharmaceutiquement acceptable, ou composition pharmaceutique pour l'utilisation selon la revendication 14, dans lequel/laquelle la thérapie anti-cancer comprend un corticostéroïde, un inhibiteur de protéasome, un agent immunomodulateur, un anticorps anti-CD38, un anticorps anti-VEGF-A, un anticorps anti-PD-1, un anticorps anti-PD-L1, ou an anticorps anti-interleukine-6,
ou une association de ceux-ci,
optionnellement dans lequel/laquelle :
- le corticostéroïde comprend dexaméthasone ; ou
- l'inhibiteur de protéasome comprend carfilzomib, ixazomib ou bortézomib ; ou
- l'agent immunomodulateur comprend lénalidomide ou pomalidomide ; ou
- l'anticorps anti-PD-L1 comprend, avélumab, durvalumab, ou atézolizumab ; ou
- l'anticorps anti-PD-1 comprend pembrolizumab ou nivolumab.
